(19)

Europäisches
Patentamt

European
Patent Office

Office européen
des brevets

(11) **EP 3 426 285 B1**

(12) **EUROPEAN PATENT SPECIFICATION**

(45) Date of publication and mention
of the grant of the patent:
**29.05.2024 Bulletin 2024/22**

(21) Application number: **17715298.0**

(22) Date of filing: **11.03.2017**

(51) International Patent Classification (IPC):
*A61K 38/44* (2006.01)   *A61K 31/436* (2006.01)
*B82Y 5/00* (2011.01)   *A61K 47/60* (2017.01)
*A61P 19/06* (2006.01)   *A61P 29/00* (2006.01)

(52) Cooperative Patent Classification (CPC):
(C-Sets available)
**A61K 9/0019; A61K 9/5146; A61K 31/436;
A61K 38/44; A61K 45/06; A61K 47/60;
A61P 19/06; A61P 29/00; A61P 37/06;
C12Y 107/03003; B82Y 5/00**   (Cont.)

(86) International application number:
**PCT/US2017/021991**

(87) International publication number:
**WO 2017/156513 (14.09.2017 Gazette 2017/37)**

(54) **FORMULATIONS AND DOSES OF PEGYLATED URICASE**

FORMULIERUNGEN UND DOSEN VON PEGYLIERTER URICASE

FORMULATIONS ET DOSES D'URICASE PÉGYLÉE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **11.03.2016   US 201662307412 P
22.05.2016   US 201662339944 P
06.06.2016   US 201662346348 P
21.09.2016   US 201662397832 P
22.09.2016   US 201662398422 P
03.10.2016   US 201662403664 P
06.12.2016   US 201662430547 P
05.01.2017   US 201762442948 P**

(43) Date of publication of application:
**16.01.2019   Bulletin 2019/03**

(73) Proprietor: **Cartesian Therapeutics, Inc.
Gaithersburg, MD 20878 (US)**

(72) Inventors:
• **JOHNSTON, Lloyd
Belmont
MA 02478 (US)**

• **KISHIMOTO, Takashi K.
Lexington,
MA 02420 (US)**
• **CAUTREELS, Werner
Boston,
MA 02115 (US)**
• **SANDS, Earl
Alpharetta,
GA 30005 (US)**

(74) Representative: **Kehoe, Laura Ellen et al
Keltie LLP
No.1 London Bridge
London SE1 9BA (GB)**

(56) References cited:
**WO-A1-2014/179771**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 426 285 B1

- Anonymous: "Selecta Biosciences Announces Dosing of First Patient in Phase 1b Clinical Trial of SEL-212, Designed to be the First Non-Immunogenic Biologic Treatment for Gout | Selecta Biosciences, Inc.", , 23 December 2015 (2015-12-23), pages 1-3, XP055371277, Retrieved from the Internet: URL:http://selectabio.com/2015/12/23/selecta-biosciences-announces-dosing-of-first-patient-in-phase-1b-clinical-trial-of-sel-212-designed-to-be-the-first-non-immunogenic-biologic-treatment-for-gout/ [retrieved on 2017-05-10]
- MICHAEL S HERSHFIELD ET AL: "Induced and pre-existing anti-polyethylene glycol antibody in a trial of every 3-week dosing of pegloticase for refractory gout, including in organ transplant recipients", ARTHRITIS RESEARCH AND THERAPY, BIOMED CENTRAL, LONDON, GB, vol. 16, no. 2, 7 March 2014 (2014-03-07), page R63, XP021181314, ISSN: 1478-6354, DOI: 10.1186/AR4500
- MALDONADO R A ET AL: "Polymeric synthetic nanoparticles for the induction of antigen-specific immunological tolerance", PROCEEDINGS NATIONAL ACADEMY OF SCIENCES PNAS, NATIONAL ACADEMY OF SCIENCES, US, vol. 112, no. 2, 13 January 2015 (2015-01-13), pages E156-E165, XP002764343, ISSN: 0027-8424
- PETER E LIPSKY ET AL: "Pegloticase immunogenicity: the relationship between efficacy and antibody development in patients treated for refractory chronic gout", ARTHRITIS RESEARCH AND THERAPY, BIOMED CENTRAL, LONDON, GB, vol. 16, no. 2, 4 March 2014 (2014-03-04), page R60, XP021184150, ISSN: 1478-6354, DOI: 10.1186/AR4497
- TAKASHI K. KISHIMOTO ET AL: "Improving the efficacy and safety of biologic drugs with tolerogenic nanoparticles", NATURE NANOTECHNOLOGY, vol. 11, no. 10, 1 August 2016 (2016-08-01), pages 890-899, XP055371105, GB ISSN: 1748-3387, DOI: 10.1038/nnano.2016.135
- ADEY A. BERHANU ET AL: "Pegloticase failure and a possible solution: Immunosuppression to prevent intolerance and inefficacy in patients with gout", SEMINARS IN ARTHRITIS AND RHEUMATISM., 1 September 2016 (2016-09-01), XP055371236, AMSTERDAM, NL ISSN: 0049-0172, DOI: 10.1016/j.semarthrit.2016.09.007

(52) Cooperative Patent Classification (CPC): (Cont.)

C-Sets
A61K 31/436, A61K 2300/00;
A61K 38/44, A61K 2300/00

2

**Description**

**FIELD OF THE INVENTION**

Provided herein are methods and compositions and kits related to uricase compositions and/or compositions comprising synthetic nanocarriers comprising an immunosuppressant. Also provided herein are methods and compositions and kits for the treatment of subjects, including subjects with hyperuricemia, gout or a condition associated with gout, and for preventing gout flare.

**SUMMARY OF THE INVENTION**

The invention is defined by the claims.

The development of anti-drug antibodies (ADAs) is a common cause for biotherapeutic treatment failure and adverse hypersensitivity reactions. It has been demonstrated that synthetic nanocarriers comprising an immunosuppressant are capable of inducing immunological tolerance to a composition comprising uricase, resulting in improved efficacy of the uricase-comprising composition. The improved efficacy has been demonstrated at least with a significantly higher rate of reduction in serum uric acid levels over time as compared to other treatments. It has also been demonstrated that synthetic nanocarriers comprising an immunosuppressant, when administered concomitantly with a composition comprising uricase, are capable of significantly reducing the incidence of gout flare as compared to other treatments. The compositions comprising synthetic nanocarriers comprising an immunosuppressant and compositions comprising a uricase as provided herein can be used to efficaciously and durably (e.g., for at least 30 days) reduce serum uric acid levels and/or reduce the incidence of gout flare.

Disclosed herein are methods comprising administering to a subject any one of the compositions comprising uricase provided herein alone or in combination with any one of the compositions comprising synthetic nanocarriers comprising an immunosuppressant. Also disclosed herein are methods of preventing gout flare, comprising concomitantly administering to a subject a composition comprising synthetic nanocarriers comprising an immunosuppressant and a composition comprising uricase, such as one that is not administered an additional therapeutic to prevent gout flare concomitantly with the concomitant administration. The subject may be identified as having had or as being expected to have gout flare from treatment with a gout therapy without concomitant administration of an additional therapeutic to prevent gout flare. The subject may be in need thereof. The subject may be any one of the subjects described herein.

Also disclosed herein are methods of treating a subject with gout or a condition associated with gout comprising administering any one of the compositions comprising uricase provided herein alone or in combination with any one of the compositions comprising synthetic nanocarriers comprising an immunosuppressant provided herein. The compositions comprising uricase disclosed herein alone or in combination with any one of the compositions comprising synthetic nanocarriers comprising an immunosuppressant may be repeatedly administered to the subject. The subject may be one in need thereof. The subject may be any one of the subjects described herein.

Disclosed herein is a method of treating a human subject with gout or a condition associated with gout, comprising administering to the subject a composition comprising uricase and a pharmaceutically acceptable carrier. The administration may be via a non-intramuscular mode of administration. The composition comprising uricase and a pharmaceutically acceptable carrier may be administered more than once to the subject. The composition comprising uricase and a pharmaceutically acceptable carrier may be administered more than twice, more than thrice, or more than four times to the subject. The composition comprising uricase and a pharmaceutically acceptable carrier may be administered every two to four weeks. The composition comprising uricase and a pharmaceutically acceptable carrier may be administered monthly. The composition comprising uricase and a pharmaceutically acceptable carrier may be administered concomitantly with a composition comprising an immunosuppressant.

Disclosed herein is a method of treating a subject with gout or a condition associated with gout, comprising concomitantly administering to the subject a composition comprising synthetic nanocarriers comprising an immunosuppressant and a composition comprising uricase.

Also disclosed herein are methods of treating a subject that may experience gout flare comprising administering any one of the compositions comprising uricase provided herein in combination with any one of the compositions comprising synthetic nanocarriers comprising an immunosuppressant provided herein. Disclosed herein is a method of preventing gout flare in a subject, comprising concomitantly administering to the subject a composition comprising synthetic nanocarriers comprising an immunosuppressant and a composition comprising uricase. In a disclosure, the subject is not administered an additional therapeutic to prevent the gout flare, such as an anti-gout flare therapeutic, concomitantly with the concomitant administration. In a disclosure, the subject is not administered colchicine or an NSAID concomitantly with the concomitant administration. The subject may be identified as having had or as being expected to have gout flare from treatment with a gout therapeutic, such as a uric acid lowering therapeutic. The subject may be identified as having had or as being expected to have gout flare without concomitant administration of an additional therapeutic to prevent the gout flare.

In the methods disclosed herein, the concomitant administration may occur more than once in the subject. The concomitant administration may occur at least twice (e.g., at least three, four, five, six, seven, eight, nine, or ten times) in the subject. The composition comprising synthetic nanocarriers comprising an immunosuppressant and the composition comprising uricase may be administered concomitantly every two to four weeks. The composition comprising synthetic nanocarriers comprising an immunosuppressant and the composition comprising uricase may be administered monthly concomitantly. The composition comprising synthetic nanocarriers comprising an immunosuppressant and the composition comprising uricase may be administered monthly for at least three months (e.g., 4, 5, 6, 7, 7, 8, 9, 10 or more months) concomitantly.

In methods disclosed herein, the composition comprising uricase may be administered at a label dose of 0.1 mg/kg - 1.2 mg/kg uricase with each administration, such as each concomitant administration. The composition comprising uricase may be administered at a label dose of 0.1 mg/kg, 0.2 mg/kg, 0.3 mg/kg, 0.4 mg/kg, 0.5 mg/kg, 0.6 mg/kg, 0.7 mg/kg, 0.8 mg/kg, 0.9 mg/kg, 1.0 mg/kg, 1.1 mg/kg, or 1.2 mg/kg uricase with each administration, such as each concomitant administration. The composition comprising uricase may be administered at a label dose of 0.2 - 0.4 mg/kg uricase with each administration, such as each concomitant administration.

In the methods disclosed herein, the composition comprising synthetic nanocarriers comprising an immunosuppressant may be administered at a label dose of 0.05 mg/kg - 0.5 mg/kg immunosuppressant with each concomitant administration. The composition comprising synthetic nanocarriers comprising an immunosuppressant may be administered at a label dose of 0.05 mg/kg, 0.07 mg/kg, 0.075 mg/kg, 0.08 mg/kg, 0.1 mg/kg, 0.125 mg/kg, 0.15 mg/kg, 0.2 mg/kg, 0.25 mg/kg, 0.3 mg/kg, 0.35 mg/kg, 0.4 mg/kg, 0.45 mg/kg, or 0.5 mg/kg immunosuppressant with each concomitant administration. The composition comprising synthetic nanocarriers comprising an immunosuppressant may be administered at a label dose of 0.075 - 0.2 mg/kg or 0.08 - 0.125 mg/kg immunosuppressant with each concomitant administration.

In the methods disclosed herein, the composition comprising synthetic nanocarriers comprising an immunosuppressant may be administered at a label dose of 0.5 mg/kg - 6.5 mg/kg with each concomitant administration, wherein the dose is given as the mg of the synthetic nanocarriers comprising the immunosuppressant. The composition comprising synthetic nanocarriers comprising an immunosuppressant may be administered at a label dose of 0.55 mg/kg, 0.6 mg/kg, 0.65 mg/kg, 0.7 mg/kg, 0.75 mg/kg, 0.8 mg/kg, 0.85 mg/kg, 0.9 mg/kg, 0.95 mg/kg, 1.0 mg/kg, 1.10 mg/kg 1.125 mg/kg, 1.5 mg/kg, 1.75 mg/kg, 2.0 mg/kg, 2.5 mg/kg, 3.0 mg/kg, 3.5 mg/kg, 4.0 mg/kg, 4.5 mg/kg, 5 mg/kg, 5.5 mg/kg, 6.0 mg/kg, or 6.5 mg/kg with each concomitant administration, wherein the dose is given as the mg of the synthetic nanocarriers comprising the immunosuppressant. The composition comprising synthetic nanocarriers comprising an immunosuppressant may be administered at a label dose of 0.6 - 2.5 mg/kg, 0.7 - 2.5 mg/kg, 0.8 - 2.5 mg/kg, 0.9 - 2.5 mg/kg, 1.0 - 2.5 mg/kg, 1.5 - 2.5 mg/kg, or 2.0 - 2.5 mg/kg with each concomitant administration, wherein the dose is given as the mg of the synthetic nanocarriers comprising the immunosuppressant. The composition comprising synthetic nanocarriers comprising an immunosuppressant may be administered at a label dose of 0.65 - 2.5 mg/kg, 0.65 - 2.0 mg/kg, 0.65 - 1.5 mg/kg, or 0.65 - 1.0 mg/kg with each concomitant administration, wherein the dose is given as the mg of the synthetic nanocarriers comprising the immunosuppressant. The composition comprising synthetic nanocarriers comprising an immunosuppressant may be administered at a label dose of 0.75 - 2.0 mg/kg, 0.8 - 1.5 mg/kg, 0.9 - 1.5 mg/kg or 1 - 2 mg/kg with each concomitant administration, wherein the dose is given as the mg of the synthetic nanocarriers comprising the immunosuppressant. The composition comprising synthetic nanocarriers comprising an immunosuppressant may be administered at a label dose of 0.9 - 2 mg/kg or 1 - 1.5 mg/kg with each concomitant administration, wherein the dose is given as the mg of the synthetic nanocarriers comprising the immunosuppressant.

In the methods disclosed herein, the method may further comprises administering a composition comprising uricase to the subject at least once (e.g., at least 2, 3, 4, 5, 6, 7, 8, 9, or 10 or more times) after the concomitant administration(s) without concomitant administration of an additional therapeutic, such as a composition comprising an immunosuppressant, such as a composition comprising synthetic nanocarriers comprising an immunosuppressant. The method may further comprise administering the composition comprising uricase at least twice after the concomitant administration(s). The method may further comprise administering the composition comprising uricase monthly for two months after the concomitant administration(s) each administration without concomitant administration of an additional therapeutic, such as a composition comprising an immunosuppressant, such as a composition comprising synthetic nanocarriers comprising an immunosuppressant. The composition comprising uricase may be administered at a label dose of 0.1 - 1.2 mg/kg uricase with each administration after the one or more concomitant administrations without an immunosuppressant. The composition comprising uricase may be administered at a label dose of 0.1 mg/kg, 0.2 mg/kg, 0.3 mg/kg, 0.4 mg/kg, 0.5 mg/kg, 0.6 mg/kg, 0.7 mg/kg, 0.8 mg/kg, 0.9 mg/kg, 1.0 mg/kg, 1.1 mg/kg, 1.2 mg/kg uricase with each administration after the one or more concomitant administrations without an immunosuppressant.

In the methods disclosed herein, the composition comprising synthetic nanocarriers comprising an immunosuppressant may be administered prior to the composition comprising uricase, such as with each concomitant administration. The composition comprising synthetic nanocarriers comprising an immunosuppressant and the composition comprising uricase may be administered within an hour of each other.

In a method disclosed herein, the subject is not administered an additional therapeutic, such as an additional gout therapeutic, such as one to prevent gout flare. The additional therapeutic, such as the additional gout therapeutic, such as one to prevent gout flare, is not administered concomitantly with each concomitant administration.

Any one of the methods, compositions or kits disclosed herein may be used to treat any one of the subjects disclosed herein.

In any one of the methods, compositions or kits disclosed herein, the subject may have an elevated serum uric acid level. The subject may have a serum uric acid level of $\geq 5$ mg/dL. The subject may have a serum uric acid level of $\geq 6$ mg/dL. The subject may have a serum uric acid level of $\geq 7$ mg/dL. The subject may have or may be at risk of having hyperuricemia; acute gout; chronic gout with or without tophi; idiopathic gout; refractory gout; secondary gout; unspecified gout; gout associated with a cardiovascular condition, renal condition, pulmonary condition, neurological condition, ocular condition, dermatological condition or hepatic condition; or has had a gout attack or gout flare. The subject may be expected to have gout flare from treatment with a gout therapeutic, such as a uric acid lowering therapeutic, such as a composition comprising uricase. The subject may have gout having at least one of a) tophi, b) gout flare within the last 6 months and c) chronic gouty arthropathy.

In the methods or compositions or kits disclosed herein, the uricase may be a pegylated uricase. In the methods or compositions or kits disclosed herein, the pegylated uricase may be pegsiticase or pegloticase. In the methods disclosed herein, the pegylated uricase may be pegsiticase. In a composition for use according to the present invention, the pegylated uricase is pegsiticase.

In the methods or compositions or kits disclosed herein, the immunosuppressant may be encapsulated in the synthetic nanocarriers. In the methods or compositions or kits disclosed herein, the immunosuppressant may be an mTOR inhibitor. In the methods or compositions or kits disclosed herein, the mTOR inhibitor may be a rapalog. In the methods or compositions or kits provided herein, the rapalog may be rapamycin. In a composition for use according to the present invention, the rapalog is rapamycin.

In the methods or compositions or kits disclosed herein, the synthetic nanocarriers may be polymeric synthetic nanocarriers. The polymeric synthetic nanocarriers may comprise a hydrophobic polyester. The hydrophobic polyester may comprise PLA, PLG, PLGA or polycaprolactone. The polymeric synthetic nanocarriers may further comprise PEG. The PEG may be conjugated to the PLA, PLG, PLGA or polycaprolactone. The polymeric synthetic nanocarriers may comprise PLA, PLG, PLGA or polycaprolactone and PEG conjugated to PLA, PLG, PLGA or polycaprolactone. In the methods or compositions or kits provided herein, the polymeric synthetic nanocarriers may comprise PLA and PLA-PEG. In a composition for use according to the present invention, the polymeric synthetic nanocarriers comprise PLA and PLA-PEG. IThe synthetic nanocarriers may be those as described according to or obtainable by any one of the exemplified methods disclosed herein. In the methods of composition or kits disclosed herein the synthetic nanocarriers may be those as described according to or obtainable by any one of the exemplified methods disclosed herein.

In the methods or compositions or kits disclosed herein, the mean of a particle size distribution obtained using dynamic light scattering of the synthetic nanocarriers may be a diameter greater than 120nm. The diameter may be greater than 150nm. The diameter may be greater than 200nm. The diameter may be greater than 250nm. The diameter may be less than 300nm. The diameter may be less than 250nm. The diameter may be less than 200nm.

In the methods or compositions or kits provided herein, the load of the immunosuppressant of the synthetic nanocarriers may be 7-12% or 8-12% by weight. The load of the immunosuppressant of the synthetic nanocarriers may be 7-10% or 8-10% by weight. The load of the immunosuppressant of the synthetic nanocarriers may be 9-11% by weight. The load of the immunosuppressant of the synthetic nanocarriers may be 7%, 8%, 9%, 10%, 11% or 12% by weight.

In the methods disclosed herein, each administration may be an intravenous administration. The intravenous administration may be an intravenous infusion.

The method disclosed herein may further comprise administering an additional therapeutic to the subject. The additional therapeutic may be an anti-inflammatory therapeutic, such as a corticosteroid. The additional therapeutic may be a gout therapeutic, such as an oral gout therapeutic. The additional therapeutic may be administered subsequently. The additional therapeutic may be administered subsequent to the completion of treatment with the concomitant administration of the uricase composition(s) and synthetic nanocarrier composition(s), such as according to any one of the regimens disclosed herein.

In the methods disclosed herein, the additional therapeutic may be an anti-gout flare treatment. The anti-gout flare treatment may be a prophylactic treatment administered concomitantly but prior to the administration of each uricase composition that is administered, such as according to any one of the regimens provided herein. The anti-gout flare treatment may be colchicine or an NSAID.

In the methods disclosed herein, the additional therapeutic may be a corticosteroid, and the corticosteroid may be administered concomitantly, such as concomitantly prior to the administration of each uricase composition that is administered, such as according to any one of the regimens disclosed herein.

Disclosed herein is a method comprising administering to any of the subjects described herein a composition comprising uricase at any one of the doses, including label doses, disclosed herein and a pharmaceutically acceptable carrier one or more times (e.g., 2, 3, 4, 5, 6, 7, 8, 9, or 10 or more times). The at least one administration or each administration may be via a non-intramuscular mode of administration. At least one administration or each administration may be an intravenous administration, such as intravenous infusion. In the methods disclosed herein, the composition comprising uricase and a pharmaceutically acceptable carrier may be administered every two or four weeks. In the methods disclosed herein, the composition comprising uricase and a pharmaceutically acceptable carrier may be administered monthly. A composition for use according to the present invention is administered monthly. In the methods disclosed herein, the composition comprising uricase and a pharmaceutically acceptable carrier may be administered concomitantly with any one of the compositions comprising an immunosuppressant described herein.

Disclosed herein is a composition or kit comprising one or more compositions comprising uricase alone or in combination with one or more compositions comprising synthetic nanocarriers comprising an immunosuppressant. Each composition comprising uricase may be any one of the compositions comprising uricase as provided herein in any one of the compositions or kits. Each composition comprising uricase may be in an amount such that it provides any one or more doses, including label doses, of the uricase as provided herein in any one of the compositions or kits. Each composition comprising uricase may be in lyophilized form in any one of the compositions or kits. Each composition comprising synthetic nanocarriers comprising an immunosuppressant may be any one of the compositions comprising synthetic nanocarriers comprising an immunosuppressant as provided herein in any one of the compositions or kits. Each composition comprising synthetic nanocarriers comprising an immunosuppressant may be in an amount such that it provides any one or more doses, including label doses, of the synthetic nanocarriers comprising an immunosuppressant or immunosuppressant as provided herein in any one of the compositions or kits. Each composition comprising synthetic nanocarriers comprising an immunosuppressant may be in lyophilized form in any one of the compositions or kits. Each composition comprising synthetic nanocarriers comprising an immunosuppressant may be in a frozen suspension in any one of the compositions or kits. The frozen suspension may further comprise phosphate-buffered saline (PBS). The lyophilized form may further comprise PBS and/or mannitol. The composition or kit may further comprise 0.9% sodium chloride, USP.

## BRIEF DESCRIPTION OF THE DRAWINGS

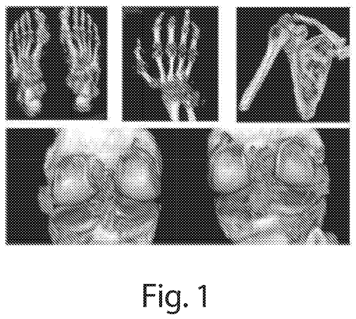

Fig. 1 is an image showing tophi/uric acid deposits visualized using DECT.

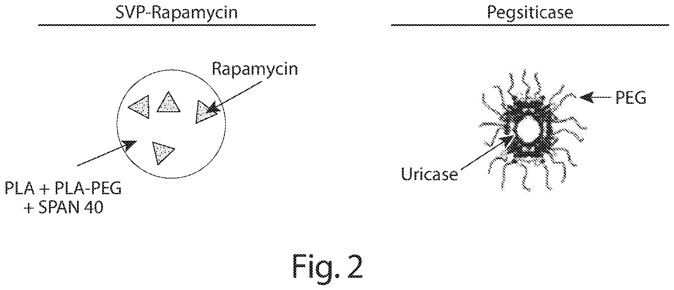

Fig. 2 is a cartoon representation of the components of SEL-212.

Fig. 3 is a graph of ADA levels in non-human primates after treatment with empty nanocarriers + pegsiticase or pegsiticase + 0.1X or 1X synthetic nanocarriers comprising rapamycin (SVP-Rapamycin).

Fig. 4 is a graph of mean serum uric acid (sUA) levels in the 5 cohorts of the phase 1a clinical trial following a single intravenous infusion of pegsiticase.

Fig. 5 is a graphical illustration showing the serum uric acid levels and uricase-specific ADA levels for each subject in Cohort #3 of the Phase 1a clinical trial and Cohort #9, Cohort # 4, and Cohort #6 in the Phase 1b clinical trial.

Fig. 6 is a graph showing the serum uric acid levels of Cohort #3 from the Phase 1a clinical and Cohort #9, Cohort #1, Cohort #2, Cohort #3, Cohort #4, Cohort #5 and Cohort #6 from the Phase 1b clinical trial trial.

Fig. 7 from left to right shows data from two replicate Kystexxa® trials, in the middle is the data of SVP-Rapamycin alone vs. pegsiticase alone (Cohort #9) and then Rapamycin alone vs. Cohort #6 (a SEL-212 cohort).

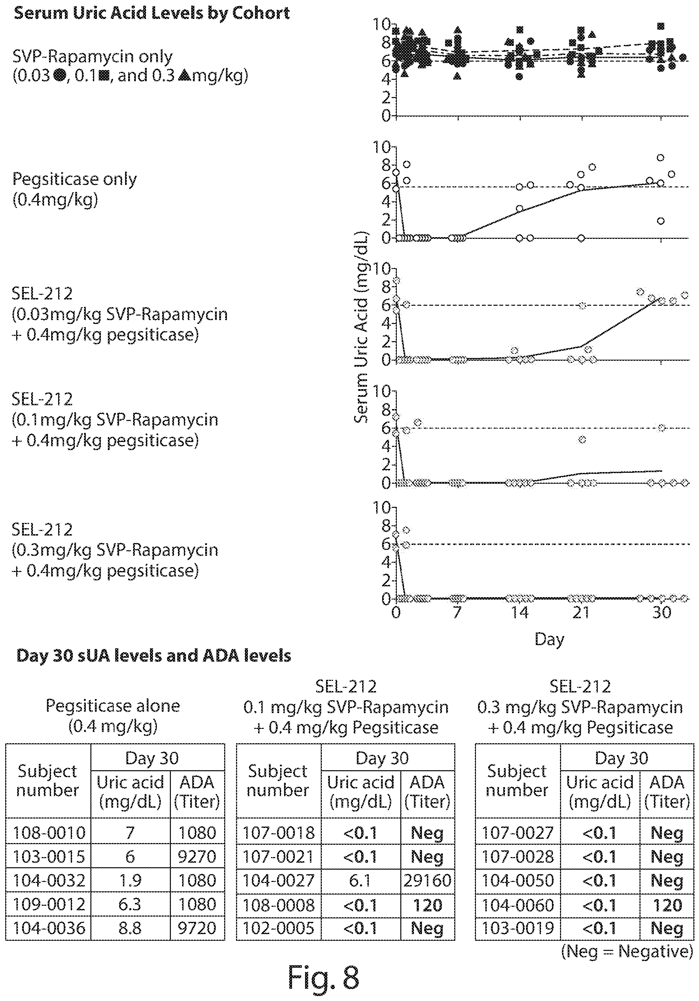

Fig. 8 is a graphical illustration showing the serum uric acid levels of subjects treated with pegstiticase alone, or in combination with synthetic nanocarriers comprising rapamycin (SVP-Rapamycin) (0.1 or 0.3 mg/kg).

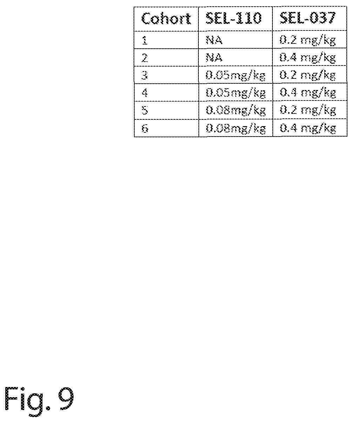

Fig. 9 shows doses for the phase 2 clinical trial.

## DETAILED DESCRIPTION OF THE INVENTION

A. OVERVIEW

Gout can be painful and disabling and is thought to result from excess uric acid. Additionally, high concentrations of uric acid, such as serum uric acid, can increase the risk of co-morbidities, including cardiovascular, cardiometabolic, joint and kidney disease. There are approximately 8.3 million and 10 million gout sufferers in the United States and the European Union, respectively.

As provided herein, it has been found that pegsiticase safely reduces uric acid serum concentration in subjects with elevated uric acid levels. As exemplified herein, the effect of a single intravenous infusion of pegsiticase resulted in serum uric acid levels that dropped significantly in all 22 subjects within approximately 10 hours. However, the serum uric acid levels did rebound by 14 to 21 days after dosing in a majority of patients. Without being bound by any particular theory, this is believed to be due to the formation of ADAs.

It was found that a PLA-PEG nanoparticle comprising rapamycin induced pegsiticase-specific immune tolerance when concomitantly administered with the pegylated uricase pegsiticase in a number of species including wild-type mice, uricase deficient (knock-out) mice, rats, and cynomolgus monkeys and resulting in efficacious and durable serum uric acid level reduction.

In addition to this surprising durable efficacy, another surprising result was noted. The complication of gout flares, which can occur following initiation of uric acid lowering therapy (Mikuls T.R.: Urate-Lowering Therapy. In Firestein G.S., Budd R.C., Harris E.D., McInnes I.B., Ruddy S., and Sergent J.S. (eds): Kelley's Textbook of Rheumatology, 8th ed. Philadelphia, PA: Elsevier Saunders, 2009), was significantly reduced in the subjects studied as described in **Examples 2** and **3.**

The pegloticase trials (phases 1, 2 and 3), on the other hand, resulted in increased gout flares in the first few months of therapy. Acute gout flares were extremely common with pegloticase. In the phase 2 trial of pegloticase, 88% of study subjects reported one or more flares during the three-month study (Sundy JS, Becker MA, Baraf HS, et al. Reduction of plasma urate levels following treatment with multiple doses of pegloticase (polyethylene glycol-conjugated uricase) in patients with treatment-failure gout: results of a phase II randomized study. Arthritis Rheum. 2008;58:2882-2891). In two phase 3 trials conducted over a 6-month period, gout flares in the first 3 months were reported in about 80% of patients, despite the administration of gout flare prophylaxis (colchicine or NSAIDs) (John S. Sundy, MD, PhD; Herbert S. B. Baraf, MD; Robert A. Yood, MD; et al. Efficacy and Tolerability of Pegloticase for the Treatment of Chronic Gout in Patients Refractory to Conventional Treatment Two Randomized Controlled Trials. JAMA. 2011;306(7):711-720).

Similarly, when pegsiticase was administered alone in the Phase 1 described in **Example 2**, 57% (4 out of 7 patients) of those with a history of gout had signs of gout flare in the first month after receiving the study drug (**Table 1, Example 3**). In contrast, however, when PLA/PLA-PEG synthetic nanocarriers comprising rapamycin were concomitantly administered with pegsiticase in a Phase 2 trial described in **Example 3**, only one gout flare was reported in the subjects who had a history of gout (16 out of 63 enrolled patients) (**Table 2, Example 3**). This subject was in the cohort that received only the rapamycin-comprising nanocarrier (without uricase). One additional subject, who did not have a prior diagnosis of gout, reported a post-treatment flare. This patient's serum uric acid level dropped from 8.8 mg/dL to 0.1 mg/dL within 90 minutes following drug administration. So, although this subject had only been diagnosed with asymptomatic hyperuricemia before the study, a flare did seem to coincide with a drop in serum uric acid.

A phase 2 study has also been undertaken (**Example 3**). This study involved the administration of multiple IV infusions of PLA/PLA-PEG synthetic nanocarriers comprising rapamycin together with pegsiticase in order to assess its safety and tolerability. Thirty-eight subjects were randomized and dosed, with 8 subjects reported as suffering from a gout flare (**Table 3, Example 3**).

Flare rates subjects were compared to the flare rates in the pegloticase trials. For subjects who received gout flare prophylaxis, 2 flares in total have occurred over 48 treatment cycles. This can be equated to 0.04 flares per treatment cycle; in other words, a flare frequency of 0.04 flares per patient month. In contrast, the Phase 3 pegloticase trials (John S. Sundy, MD, PhD; Herbert S. B. Baraf, MD; Robert A. Yood, MD; et al. Efficacy and Tolerability of Pegloticase for the Treatment of Chronic Gout in Patients Refractory to Conventional Treatment Two Randomized Controlled Trials. JAMA. 2011;306(7):711-720) reported the following: 2.3 flares per patient over the first 3 months for 85 patients who received biweekly pegloticase, and 2.7 flares per patient over the first 3 months for 84 patients who received monthly pegloticase. These numbers equate to a flare frequency of 0.77 and 0.9 flares per patient month, respectively.

Further comparisons were made with the two primary branded oral uric acid lowering medication, febuxostat and lesinurad. Based on data from a phase 3, randomized, double-blind, multi-center trial (Michael A. Becker, M.D., H. Ralph Schumacher, Jr., M.D., Robert L. Wortmann, M.D., Patricia A. MacDonald, B.S.N., N.P., Denise Eustace, B.A., William A. Palo, M.S., Janet Streit, M.S., and Nancy Joseph-Ridge, M.D. Febuxostat Compared with Allopurinol in Patients with Hyperuricemia and Gout. N Engl J Med 2005; 353:2450-2461December 8, 2005), a dose of 80 mg/day resulted in 55 out of 255 subjects requiring treatment for at least one gout flare. This would be the equivalent to a flare frequency of at least 0.22 flares per patient month, and possibly more. At a dose of 120 mg/day, 90 out of 250 subjects required treatment for at least one gout flare, equating to at least a flare frequency of 0.36 flares per patient month, and possibly more.

Still further comparisons were made. During a phase 2, randomized, double-blind study to assess the efficacy and tolerability of lesinurad (Perez -Ruiz F, Sundy JS, Miner JN for the RDEA594-203 Study Group, et al. Lesinurad in combination with allopurinol: results of a phase 2, randomised, double-blind study in patients with gout with an inadequate response to allopurinol, Annals of the Rheumatic Diseases 2016;75:1074-1080), gout flares requiring treatment were reported in 10 out of 46 patients in a month in those dosed at 200 mg daily, 13 out of 42 patients in a month in those dosed at 400 mg daily, and 15 out of 48 patients in a month in those dosed at 600 mg daily. This equates to a flare frequency of 0.22, 0.31, and 0.31 flares per patient month, respectively. The aforementioned comparisons are described in **Table 4, Example 3.**

The flare frequency is clearly reduced for the subjects who received the rapamycin-containing nanocarrier concomitantly administered with pegsiticase as compared to all of the other medications. This unexpected outcome is significantly better than with other therapies. This also has the benefit for patient adherence to uric acid lowering therapies, such as uricase, as adherence is greatly reduced when rebound flares occur following initiation of therapy (Treatment of chronic gouty arthritis: it is not just about urate-lowering therapy. Schlesinger N - Semin. Arthritis Rheum. - October 1, 2012; 42 (2); 155-65).

Based on studies and data, examples of which are provided above and elsewhere herein, it has been demonstrated that the compositions and methods provided are substantially more efficacious than currently available treatments, can reduce undesired immune responses associated with the delivery of uricase, such as pegylated uricase, can provide strong and durable control of serum uric acid levels in patients, can provide for the removal of painful and damaging uric acid deposits for patients, such as with chronic tophaceous gout, and/or can substantially reduce or eliminate the risk of gout flare that may occur with uric acid lowering therapies, such as uricase.

B. DEFINITIONS

"Additional therapeutic", as used herein, refers to any therapeutic that is used in addition to another treatment. For example, when the method is one directed to treatment with synthetic nanocarriers comprising an immunosuppressant, and the method comprises the use of an additional therapeutic, the additional therapeutic is in addition to synthetic nanocarriers comprising an immunosuppressant. As another example, when the method is one directed to treatment with a combination of a composition comprising a uricase and a composition comprising synthetic nanocarriers comprising an immunosuppressant, and the method comprises the use of an additional therapeutic, the additional therapeutic is in addition to the uricase and synthetic nanocarrier composition combination. Generally, the additional therapeutic will be a different therapeutic. The additional therapeutic may be administered at the same time or at a different time and/or via the same mode of administration or via a different mode of administration, as that of the other therapeutic. In preferred embodiments, the additional therapeutic will be given at a time and in a way that will provide a benefit to the subject during the effective treatment window of the other therapeutic. When two compositions are administered with a specific time period, generally the time period is measured from the start of the first composition to the start of the second composition. As used herein, when two compositions are given within an hour, for example, the time before the start of the administration of the first composition is about an hour before the start of the administration of the second composition.

In some embodiments, the additional therapeutic is another therapeutic for the treatment of gout or a condition associated with gout. As used herein, a "gout therapeutic" is any therapeutic that can be administered and from which a subject with gout may derive a benefit because of its administration. In some embodiments, the gout therapeutic is an oral gout therapeutic (i.e., a gout therapeutic that can be taken or given orally).

The additional therapeutic may be any one of the previously approved therapeutics described herein or otherwise known in the art. In some embodiments, the additional therapeutic is an uric acid lowering therapeutic. Such a therapeutic is any that results in a lower serum uric acid level in a subject as compared to a serum uric acid level in the subject without the administration of the therapeutic. Such uric acid lowering therapeutics include, uricases.

In some embodiments, the additional therapeutic is a therapeutic for preventing gout flare or also referred to herein as an anti-gout flare therapeutic. Any therapeutic that can be used to prevent a gout flare is included in this class of therapeutics. In some of these embodiments, the therapeutic for preventing gout flare is given prior to the administration of the other therapeutic. In some embodiments, the therapeutic for preventing gout flare is colchicine. In other embodiments, the therapeutic for preventing gout flare is an NSAID.

Any one of the methods for treating any one of the subjects or any one of the compositions or kits as provided herein can include the administration of an additional therapeutic or an additional therapeutic, respectively. Any one of the methods for treating any one of the subjects or any one of the compositions or kits as provided herein does not include the administration of an additional therapeutic, such as within the effective treatment window of the other therapeutic, or an additional therapeutic, respectively.

"Administering" or "administration" or "administer" means giving a material to a subject in a manner such that there is a pharmacological result in the subject. This may be direct or indirect administration, such as by inducing or directing another subject, including another clinician or the subject itself, to perform the administration.

"Amount effective" in the context of a composition or dose for administration to a subject refers to an amount of the composition or dose that produces one or more desired responses in the subject. In some embodiments, the amount effective is a pharmacodynamically effective amount. Therefore, in some embodiments, an amount effective is any amount of a composition or dose provided herein that produces one or more of the desired therapeutic effects and/or immune responses as provided herein. This amount can be for in vitro or in vivo purposes. For in vivo purposes, the amount can be one that a clinician would believe may have a clinical benefit for a subject in need thereof. Any one of the compositions or doses, including label doses, as provided herein can be in an amount effective.

Amounts effective can involve reducing the level of an undesired response, although in some embodiments, it involves preventing an undesired response altogether. Amounts effective can also involve delaying the occurrence of an undesired response. An amount that is effective can also be an amount that produces a desired therapeutic endpoint or a desired therapeutic result. In other embodiments, the amounts effective can involve enhancing the level of a desired response, such as a therapeutic endpoint or result. Amounts effective, preferably, result in a therapeutic result or endpoint and/or reduced or eliminated ADAs against the treatment and/or result in prevention of gout flare in any one of the subjects provided herein. The achievement of any of the foregoing can be monitored by routine methods.

Amounts effective will depend, of course, on the particular subject being treated; the severity of a condition, disease or disorder; the individual patient parameters including age, physical condition, size and weight; the duration of the treatment; the nature of concurrent therapy (if any); the specific route of administration and like factors within the knowledge and expertise of the health practitioner. These factors are well known to those of ordinary skill in the art and can be addressed with no more than routine experimentation. It is generally preferred that a maximum dose be used, that is, the highest safe dose according to sound medical judgment. It will be understood by those of ordinary skill in the art, however, that a patient may insist upon a lower dose or tolerable dose for medical reasons, psychological reasons or for virtually any other reason.

Doses of the components in any one of the compositions of the invention or used in any one of the methods of the invention may refer to the amount of the components in the composition, the actual amounts of the respective components received by an administered subject, or the amount that appears on a label (also referred to herein as label dose). The dose can be administered based on the number of synthetic nanocarriers that provide the desired amount of the component(s).

"Attach" or "Attached" or "Couple" or "Coupled" (and the like) means to chemically associate one entity (for example a moiety) with another. In some embodiments, the attaching is covalent, meaning that the attachment occurs in the context of the presence of a covalent bond between the two entities. In non-covalent embodiments, the non-covalent attaching is mediated by non-covalent interactions including but not limited to charge interactions, affinity interactions, metal coordination, physical adsorption, host-guest interactions, hydrophobic interactions, TT stacking interactions, hydrogen bonding interactions, van der Waals interactions, magnetic interactions, electrostatic interactions, dipole-dipole interactions, and/or combinations thereof. In embodiments, encapsulation is a form of attaching.

"Average", as used herein, refers to the arithmetic mean unless otherwise noted.

"Concomitantly" means administering two or more materials/agents to a subject in a manner that is correlated in time, preferably sufficiently correlated in time so as to provide a modulation in a physiologic or immunologic response, and even more preferably the two or more materials/agents are administered in combination. In embodiments, concomitant administration may encompass administration of two or more materials/agents within a specified period of time, preferably within 1 month, more preferably within 1 week, still more preferably within 1 day, and even more preferably within 1 hour. In embodiments, the two or more materials/agents are sequentially administered. In embodiments, the materials/agents may be repeatedly administered concomitantly; that is concomitant administration on more than one occasion.

"Dose" refers to a specific quantity of a pharmacologically active material for administration to a subject for a given time. Unless otherwise specified, the doses recited for compositions comprising pegylated uricase refer to the weight of the uricase (i.e., the protein without the weight of the PEG or any other components of the composition comprising the pegylated uricase). Also, unless otherwise specified, the doses recited for compositions comprising synthetic nanocarriers comprising an immunosuppressant refer to the weight of the immunosuppressant (i.e, without the weight of the synthetic nanocarrier material or any of the other components of the synthetic nanocarrier composition). When referring to a dose for administration, in an embodiment of any one of the methods, compositions or kits disclosed herein, any one of the doses provided herein is the dose as it appears on a label/label dose.

"Encapsulate" means to enclose at least a portion of a substance within a synthetic nanocarrier. In some embodiments, a substance is enclosed completely within a synthetic nanocarrier. In other embodiments, most or all of a substance that is encapsulated is not exposed to the local environment external to the synthetic nanocarrier. In other embodiments, no more than 50%, 40%, 30%, 20%, 10% or 5% (weight/weight) is exposed to the local environment. Encapsulation is distinct from absorption, which places most or all of a substance on a surface of a synthetic nanocarrier, and leaves the substance exposed to the local environment external to the synthetic nanocarrier. In embodiments of any one of the methods or compositions disclosed herein, the immunosuppressants are encapsulated within the synthetic nanocarriers.

"Elevated serum uric acid level" refers to any level of uric acid in a subject's serum that may lead to an undesirable result or would be deemed by a clinician to be elevated. In an embodiment, the subject of any one of the methods provided herein can have a serum uric acid level of $\geq 5$ mg/dL, $\geq 6$ mg/dL, or $\geq 7$ mg/dL. Such a subject may be a hyperuremic subject. Whether or not a subject has elevated blood uric acid levels can be determined by a clinician, and in some embodiments, the subject is one in which a clinician has identified or would identify as having elevated serum uric acid levels.

"Gout" generally refers to a disorder or condition associated with the buildup of uric acid, such as deposition of uric crystals in tissues and joints, and/or a clinically relevant elevated serum uric acid level. Accumulation of uric acid may be due to overproduction of uric acid or reduced excretion of uric acid. Gout may range from asymptomatic to severe and painful inflammatory conditions. A "condition associated with gout" refers to any condition in a subject where the subject experiences local and/or systemic effects of gout, including inflammation and immune responses, and in which the condition is caused or exacerbated by, or the condition can result in or exacerbate, gout. A gout flare is an "attack" or exacerbation of gout symptoms, which can happen at any time. Gout flares can include gout flares that occur after the administration of a uric acid lowering therapy.

"Hydrophobic polyester" refers to any polymer that comprises one or more polyester polymers or units thereof and that has hydrophobic characteristics. Polyester polymers include, but are not limited to, PLA, PLGA, PLG and polycaprolactone. "Hydrophobic" refers to a material that does not substantially participate in hydrogen bonding to water. Such materials are generally non-polar, primarily non-polar, or neutral in charge. Synthetic nanocarriers may be completely comprised of hydrophobic polyesters or units thereof. In some embodiments, however, the synthetic nanocarriers comprise hydrophobic polyesters or units thereof in combination with other polymers or units thereof. These other polymers or units thereof may by hydrophobic but are not necessarily so. In some preferred embodiments, when synthetic nanocarriers include one or more other polymers or units thereof in addition to a hydrophobic polyester, the matrix of other polymers or units thereof with the hydrophobic polyester is hydrophobic overall. Examples of synthetic nanocarriers that can be used in the invention and that comprise hydrophobic polyesters can be found in U.S. Publication Nos. US 2016/0128986 and US 2016/0128987.

"Immunosuppressant", as used herein, means a compound that can cause a tolerogenic immune response specific to an antigen, also referred to herein as an "immunosuppressive effect". An immunosuppressive effect generally refers to the production or expression of cytokines or other factors by an antigen-presenting cell (APC) that reduces, inhibits or prevents an undesired immune response or that promotes a desired immune response, such as a regulatory immune response, against a specific antigen. When the APC acquires an immunosuppressive function (under the immunosuppressive effect) on immune cells that recognize an antigen presented by this APC, the immunosuppressive effect is said to be specific to the presented antigen. Examples of immunosuppressants include "mTOR inhibitors", a class of drugs that inhibit mTOR, a serine/threonine-specific protein kinase that belongs to the family of phosphatidylinositol-3 kinase (PI3K) related kinases (PIKKs). mTOR inhibitors include, but are not limited to, rapalogs, such as rapamycin, as well as ATP-competitive mTOR kinase inhibitors, such as mTORC1/mTORC2 dual inhibitors.

In embodiments of the compositions disclosed herein, the immunosuppressants provided herein are attached to synthetic nanocarriers. In preferable embodiments, the immunosuppressant is an element that is in addition to the material that makes up the structure of the synthetic nanocarrier. For example, in one embodiment, where the synthetic nanocarrier is made up of one or more polymers, the immunosuppressant is a compound that is in addition and attached to the one or more polymers. In embodiments, such as where the material of the synthetic nanocarrier also results in an immunosuppressive effect, the immunosuppressant is an element present in addition to the material of the synthetic nanocarrier that results in an immunosuppressive effect.

"Load", when comprise in a composition comprising a synthetic nanocarrier, such as coupled thereto, is the amount of the immunosuppressant in the composition based on the total dry recipe weight of materials in an entire synthetic nanocarrier (weight/weight). Generally, such a load is calculated as an average across a population of synthetic nanocarriers. In one embodiment, the load on average across the synthetic nanocarriers is between 0.1% and 15%. In another embodiment, the load is between 0.1% and 10%. In a further embodiment, the load is between 1% and 15%. In yet a further embodiment, the load is between 5% and 15%. In still a further embodiment, the load is between 7% and 12%. In still a further embodiment, the load is between 8% and 12%. In still another embodiment, the load is between 7% and 10%. In still another embodiment, the load is between 8% and 10%. In yet a further embodiment, the load is 5%, 6%, 7%, 8%, 9%, 10%, 11%, 12%, 13%, 14%, or 15% on average across the population of synthetic nanocarriers. In any one of the methods, compositions or kits provided herein, the load of the immunosuppressant, such as rapamycin, may be any one of the loads provided herein.

The rapamycin load of the nanocarrier in suspension is calculated by dividing the rapamycin content of the nanocarrier as determined by HPLC analysis of the test article by the nanocarrier mass. The total polymer content is measured either by gravimetric yield of the dry nanocarrier mass or by the determination of the nanocarrier solution total organic content following pharmacopeia methods and corrected for PVA content.

"Maximum dimension of a synthetic nanocarrier" means the largest dimension of a nanocarrier measured along any axis of the synthetic nanocarrier. "Minimum dimension of a synthetic nanocarrier" means the smallest dimension of a synthetic nanocarrier measured along any axis of the synthetic nanocarrier. For example, for a spheroidal synthetic nanocarrier, the maximum and minimum dimension of a synthetic nanocarrier would be substantially identical, and would be the size of its diameter. Similarly, for a cuboidal synthetic nanocarrier, the minimum dimension of a synthetic nanocarrier would be the smallest of its height, width or length, while the maximum dimension of a synthetic nanocarrier would be the largest of its height, width or length. In an embodiment, a minimum dimension of at least 75%, preferably at least 80%, more preferably at least 90%, of the synthetic nanocarriers in a sample, based on the total number of synthetic nanocarriers in the sample, is equal to or greater than 100 nm. In an embodiment, a maximum dimension of at least 75%, preferably at least 80%, more preferably at least 90%, of the synthetic nanocarriers in a sample, based on the total number of synthetic nanocarriers in the sample, is equal to or less than 5 $\mu$m. Preferably, a minimum dimension of at least 75%, preferably at least 80%, more preferably at least 90%, of the synthetic nanocarriers in a sample, based on the total number of synthetic nanocarriers in the sample, is greater than 110 nm, more preferably greater than 120 nm, more preferably greater than 130 nm, and more preferably still greater than 150 nm. Aspects ratios of the maximum and minimum dimensions of synthetic nanocarriers may vary depending on the embodiment. For instance, aspect ratios of the maximum to minimum dimensions of the synthetic nanocarriers may vary from 1:1 to 1,000,000:1, preferably from 1:1 to 100,000:1, more preferably from 1:1 to 10,000:1, more preferably from 1:1 to 1000:1, still more preferably from 1:1 to 100:1, and yet more preferably from 1:1 to 10:1.

Preferably, a maximum dimension of at least 75%, preferably at least 80%, more preferably at least 90%, of the synthetic nanocarriers in a sample, based on the total number of synthetic nanocarriers in the sample is equal to or less than 3 $\mu$m, more preferably equal to or less than 2 $\mu$m, more preferably equal to or less than 1 $\mu$m, more preferably equal to or less than 800 nm, more preferably equal to or less than 600 nm, and more preferably still equal to or less than 500 nm. In preferred embodiments, a minimum dimension of at least 75%, preferably at least 80%, more preferably at least 90%, of the synthetic nanocarriers in a sample, based on the total number of synthetic nanocarriers in the sample, is equal to or greater than 100 nm, more preferably equal to or greater than 120 nm, more preferably equal to or greater than 130 nm, more preferably equal to or greater than 140 nm, and more preferably still equal to or greater than 150 nm. Measurement of synthetic nanocarrier dimensions (e.g., effective diameter) may be obtained, in some embodiments, by suspending the synthetic nanocarriers in a liquid (usually aqueous) media and using dynamic light scattering (DLS) (e.g., using a Brookhaven ZetaPALS instrument). For example, a suspension of synthetic nanocarriers can be diluted from an aqueous buffer into purified water to achieve a final synthetic nanocarrier suspension concentration of approximately 0.01 to 0.5 mg/mL. The diluted suspension may be prepared directly inside, or transferred to, a suitable cuvette for DLS analysis. The cuvette may then be placed in the DLS, allowed to equilibrate to the controlled temperature, and then scanned for sufficient time to acquire a stable and reproducible distribution based on appropriate inputs for viscosity of the medium and refractive indicies of the sample. The effective diameter, or mean of the distribution, is then reported. Determining the effective sizes of high aspect ratio, or non-spheroidal, synthetic nanocarriers may require augmentative techniques, such as electron microscopy, to obtain more accurate measurements. "Dimension" or "size" or "diameter" of synthetic nanocarriers means the mean of a particle size distribution, for example, obtained using dynamic light scattering.

"Pegylated uricase" refers to any uricase that is attached to one or more PEG (poly(ethylene glycol), poly (ethylene oxide) or poly (oxyethylene)) molecules (i.e, a poly(ethylene glycol), poly (ethylene oxide) or poly (oxyethylene) polymer or unit thereof). Preferably in some embodiments, the one or more PEG molecules are poly(ethylene glycol) molecules. The terms "pegylated" or "pegylation" refer to the conjugated form or the act of conjugating to the uricase, respectively. Such a modified uricase is referred to as pegylated uricase. Pegylated uricases include, but are not limited to pegsiticase and pegloticase (KRYSTEXXA®).

"Pharmaceutically acceptable excipient" or "pharmaceutically acceptable carrier" means a pharmacologically inactive material used together with a pharmacologically active material to formulate the compositions. Pharmaceutically acceptable excipients comprise a variety of materials known in the art, including but not limited to saccharides (such as glucose, lactose, and the like), preservatives such as antimicrobial agents, reconstitution aids, colorants, saline (such as phosphate buffered saline), and buffers. Any one of the compositions provided herein may include a pharmaceutically acceptable excipient or carrier.

"Rapalog" refers to rapamycin and molecules that are structurally related to (an analog) of rapamycin (sirolimus), and are preferably hydrophobic. Examples of rapalogs include, without limitation, temsirolimus (CCI-779), deforolimus, everolimus (RAD001), ridaforolimus (AP-23573), zotarolimus (ABT-578). Additional examples of rapalogs may be found, for example, in WO Publication WO 1998/002441 and U.S. Patent No. 8,455,510. In any one of the methods or compositions or kits provided herein, the immunosuppressant may be a rapalog.

"Subject" means animals, including warm blooded mammals such as humans and primates; avians; domestic household or farm animals such as cats, dogs, sheep, goats, cattle, horses and pigs; laboratory animals such as mice, rats and guinea pigs; fish; reptiles; zoo and wild animals; and the like. In any one of the methods, compositions and kits provided herein, the subject is human. In any one of the methods, compositions and kits provided herein, the subject is any one of the subjects provided herein, such as one that has any one of the conditions provided herein, such as gout or other condition associated with gout.

"Synthetic nanocarrier(s)" means a discrete object that is not found in nature, and that possesses at least one dimension that is less than or equal to 5 microns in size. Synthetic nanocarriers may be a variety of different shapes, including but not limited to spheroidal, cuboidal, pyramidal, oblong, cylindrical, toroidal, and the like. Synthetic nanocarriers comprise one or more surfaces.

A synthetic nanocarrier can be, but is not limited to, one or a plurality of lipid-based nanoparticles (also referred to herein as lipid nanoparticles, i.e., nanoparticles where the majority of the material that makes up their structure are lipids), polymeric nanoparticles, metallic nanoparticles, surfactant-based emulsions, dendrimers, buckyballs, nanowires, virus-like particles (i.e., particles that are primarily made up of viral structural proteins but that are not infectious or have low infectivity), peptide or protein-based particles (also referred to herein as protein particles, i.e., particles where the majority of the material that makes up their structure are peptides or proteins) (such as albumin nanoparticles) and/or nanoparticles that are developed using a combination of nanomaterials such as lipid-polymer nanoparticles. Synthetic nanocarriers may be a variety of different shapes, including but not limited to spheroidal, cuboidal, pyramidal, oblong, cylindrical, toroidal, and the like. Examples of synthetic nanocarriers include (1) the biodegradable nanoparticles disclosed in US Patent 5,543,158 to Gref et al., (2) the polymeric nanoparticles of Published US Patent Application 20060002852 to Saltzman et al., (3) the lithographically constructed nanoparticles of Published US Patent Application 20090028910 to DeSimone et al., (4) the disclosure of WO 2009/051837 to von Andrian et al., (5) the nanoparticles disclosed in Published US Patent Application 2008/0145441 to Penades et al., (6) the nanoprecipitated nanoparticles disclosed in P. Paolicelli et al., "Surface-modified PLGA-based Nanoparticles that can Efficiently Associate and Deliver Virus-like Particles" Nanomedicine. 5(6):843-853 (2010), and (7) those of Look et al., Nanogel-based delivery of mycophenolic acid ameliorates systemic lupus erythematosus in mice" J. Clinical Investigation 123(4): 1741-1749(2013).

Synthetic nanocarriers may have a minimum dimension of equal to or less than about 100 nm, preferably equal to or less than 100 nm, do not comprise a surface with hydroxyl groups that activate complement or alternatively comprise a surface that consists essentially of moieties that are not hydroxyl groups that activate complement. In an embodiment, synthetic nanocarriers that have a minimum dimension of equal to or less than about 100 nm, preferably equal to or less than 100 nm, do not comprise a surface that substantially activates complement or alternatively comprise a surface that consists essentially of moieties that do not substantially activate complement. In a more preferred embodiment, synthetic nanocarriers according to the invention that have a minimum dimension of equal to or less than about 100 nm, preferably equal to or less than 100 nm, do not comprise a surface that activates complement or alternatively comprise a surface that consists essentially of moieties that do not activate complement. In embodiments, synthetic nanocarriers exclude virus-like particles. In embodiments, synthetic nanocarriers may possess an aspect ratio greater than 1:1, 1:1.2, 1:1.5, 1:2, 1:3, 1:5, 1:7, or greater than 1:10.

"Treating" refers to the administration of one or more therapeutics with the expectation that the subject may have a resulting benefit due to the administration. The treating may also result in the prevention of a condition as provided herein and, therefore, treating includes prophylactic treatment. When used prophylactically, the subject is one in which a clinician expects that there is a likelihood for the development of a condition or other undesired response as provided herein. In some embodiments, a subject that is expected to have a gout flare is one in which a clinician believes there is a likelihood that a gout flare will occur. Treating may be direct or indirect, such as by inducing or directing another subject, including another clinician or the subject itself, to treat the subject.

"Weight%" or "% by weight" refers to the ratio of one weight to another weight times 100. For example, the weight% can be the ratio of the weight of one component to another times 100 or the ratio of the weight of one component to a total weight of more than one component times 100. Generally, the weight% is measured as an average across a population of synthetic nanocarriers or an average across the synthetic nanocarriers in a composition or suspension.

## C. METHODS AND RELATED COMPOSITIONS

As mentioned elsewhere herein, it has been demonstrated that the compositions and methods provided are substantially more efficacious than currently available treatments, can reduce undesired immune responses associated with the delivery of a therapeutic, such as pegylated uricase, can provide strong and durable control of serum uric acid levels in patients, can provide for the removal of painful and damaging uric acid deposits for patients, such as with chronic tophaceous gout, and/or can substantially reduce the incidence of gout flare.

Specifically, it has been found that synthetic nanocarriers comprising an immunosuppressant, such as rapamycin, can induce durable immune tolerance to a therapeutic, such as pegylated uricase, for example, pegsiticase.

The methods and compositions disclosed herein can overcome undesired immune responses and optimize the effectiveness of a uricase-based treatment in controlling uric acid levels and, as a result, enable the effective dissolution and removal of uric acid crystals. It has also been found that the methods and compositions provided here can lead to significantly reduced gout flare occurrences with or without gout flare prophylactic treatment.

*Uricase and Pegylated Uricase*

The methods and compositions and kits described herein involve compositions comprising uricase. Uricase is generally thought to catalyze the conversion of uric acid to allantoin, which is soluble and may be excreted. Uricase is an enzyme endogenous to all mammals, except for humans and certain primates. The gene encoding the uricase enzyme

may be obtained from any source known in the art, including mammalian and microbial sources as well as by recombinant and synthetic technologies. As will be evident to one of ordinary skill in the art, a gene may be obtained from a source and recombinantly (or transgenically) expressed and produced in another organism using standard methods. See Erlich, H A, (Ed.) (1989) PCR Technology. Principles and Applications for DNA Amplification. New York: Stockton Press; Sambrook, J, et al., (1989) Molecular Cloning. A Laboratory Manual, Second Edition. Cold Spring Harbor, N.Y.: Cold Spring Harbor Laboratory Press. For example, U.S. Patent No. 5,700,674 describes recombinant production of uricase in E. coli cells. In the methods and compositions and kits described herein, the enzyme may be produced by fermentation in E. coli.

In the methods and compositions and kits described herein, the gene encoding the uricase, or a portion thereof, may be obtained from a mammal, for example a pig, bovine, sheep, goat, baboon, monkey mouse, rabbit, or domestic animal. In the methods and compositions and kits described herein he gene encoding the uricase, or a portion thereof, may be obtained from a microorganism, such as a bacteria or fungi (including yeast). In the methods and compositions and kits described herein, the gene encoding the uricase may be obtained from a bacterial source, such as bacterium belonging to Streptomyces spp., Bacillus spp., or E. coli. In the methods and compositions and kits described herein, the gene encoding the uricase may be obtained from a fungal (including yeast) source, such as Candida (e.g., Candida utilis), Anthrobacter (e.g., Anthrobacter globiformis), Saccharomyces, Schizosaccaromyces, Emericella, Aspergillus (e.g., Aspergillus flavus), and Neurospora spp. In the methods and compositions and kits described herein, the uricase may be derived from Candida utilis. In the methods and compositions and kits described herein, the uricase may be that of pegsiticase (3SBio as described in U.S. Patent No. 6,913,915). In a composition for use according to the present invention, the uricase is that of pegsiticase. In the methods and compositions and kits described herein, the uricase may be derived from Aspergillus flavus. The uricase may be rasburicase (ELITEK®; FASTURTEC®, from Sanofi Genzyme).

In the methods and compositions and kits described herein, the uricase may be chimeric uricase, in which portions of the gene encoding the uricase are obtained from different sources. For example, a portion of the gene encoding the chimeric uricase may be obtained from one organism and one or more other portions of the gene encoding the chimeric uricase may be obtained from another organism. A portion of the gene encoding the chimeric uricase may be obtained from a pig and another portion of the gene encoding the chimeric uricase is obtained from a baboon. The chimeric uricase may be that of pegloticase/KRYSTEXXA®.

Also disclosed herein are variant uricases, which may include one or more mutations (substitutions, insertions, deletions). Mutations may be made in the nucleotide sequence encoding the uricase protein, which may or may not result in an amino acid mutation. In general, mutations may be made, for example, to enhance production of the protein, turnover/half-life of the protein or mRNA encoding the protein, modulate (enhance or reduce) the enzymatic activity of the uricase.

In the methods and compositions and kits described herein, the gene encoding the uricase may be obtained from a plant or invertebrate source, such as Drosophila or C. elegans.

Any of the uricase proteins described herein may be pegylated. Uricase may be covalently bonded to PEG via a biocompatible linking group, using methods known in the art, as described, for example, by Park et al, Anticancer Res., 1:373-376 (1981); and Zaplipsky and Lee, Polyethylene Glycol Chemistry: Biotechnical and Biomedical Applications, J. M. Harris, ed., Plenum Press, New York, Chapter 21 (1992). The linking group used to covalently attach PEG to uricase may be any biocompatible linking group, meaning the linking group non-toxic and may be utilized in vitro or in vivo without causing adverse effects. Alternatively, the PEG may be directly conjugated to the uricase, such as directly to a lysine residue of uricase.

Uricase may be pegylated at many different amino acid resides of the uricase protein. The number of PEG molecules and/or residue to which the PEG is conjugated may affect the activity of the uricase. In the methods and compositions and kits described herein, the pegylated uricase may comprise at least one PEG molecule. In the methods and compositions and kits described herein, the pegylated uricase may comprise at least 2, 3, 4, 5, 6, 7,8, 9, 10, 11, 12, 13, 14, 15, 16, 17, 18, 19, 20, 21, 22, 23, 24, 25, 26, 27, 28, 29, 30, 31, 32, 33,34, 35, 36, 37, 38, 39, 40, 45, 50, or more PEG molecules on average per uricase protein. In the methods and compositions and kits described herein, the pegylated uricase may comprise about 20-25 PEG molecules per uricase protein.

On average, PEG has a molecular weight between 5 kDa to 100 kDa. In the methods and compositions and kits described herein, both the molecular weight (size) of the PEG used as well as the number of PEG molecules used to pegylate the uricase may be varied. The average molecular weight of the PEG may be between 5 kDa to 100 kDa, 5 kDa to 75 kDa, 5 kDa to 50 kDa, 5 kDa to 30 kDa, 5 kDa to 20 kDa, 5 kDa to 10 kDa, 10 kDa to 75 kDa, 10 kDa to 50 kDa, 10 kDa to 30 kDa, 5 kDa to 30 kDa, 15 kDa to 50 kDa, 15 kDa to 30 kDa, 15 kDa to 25 kDa, 20 kDa to 75 kDa, 30 kDa to 80 kDa, 30 kDa to 70 kDa, or 30 kDa to 50 kDa. The molecular weight of the PEG may be about 5 kDa, 6 kDa, 7 kDa, 8 kDa, 9 kDa, 10 kDa, 11 kDa, 12 kDa, 13 kDa, 14 kDa, 15 kDa, 16 kDa, 17 kDa, 18 kDa, 19 kDa, 20 kDa, 21 kDa, 22 kDa, 23 kDa, 24 kDa, 25 kDa, 30 kDa, 35 kDa, 40 kDa, 45 kDa, 50 kDa, 55 kDa, 60 kDa, 65 kDa, 70 kDa, 75 kDa, 80 kDa, 85 kDa, 90 kDa, 95 kDa, or 100 kDa. In general, the PEG is referred to based on the molecular weight of the PEG. For example, PEG-20 refers to PEG molecules with a molecular weight of 20 kDa, and PEG-5 refers to PEG molecules with a molecular weight of 5 kDa. The uricase may be pegylated with PEG molecules having a molecule weight of 20 kDa (PEG-20).

Pegylated uricases include, without limitation, pegsiticase (available from 3Sbio, and as described in U.S. Patent No. 6,913,915) and pegloticase/KRYSTEXXA® (Horizon Pharmaceuticals).

In a composition for use according to the present invention, the pegylated uricase is pegsiticase, a recombinant uricase conjugated to multiple 20 kDa molecular weight poly (ethylene glycol) molecules. The uricase component of pegsiticase can be cloned from the yeast Candida utilis and expressed in E. coli for production.

The uric acid catalysis activity of uricase, including pegylated uricase, can be assessed using methods known in the art or as otherwise provided herein.

*Synthetic Nanocarriers*

A variety of synthetic nanocarriers can be used. In some embodiments, synthetic nanocarriers are spheres or spheroids. In some embodiments, synthetic nanocarriers are flat or plate-shaped. In some embodiments, synthetic nanocarriers are cubes or cubic. In some embodiments, synthetic nanocarriers are ovals or ellipses. In some embodiments, synthetic nanocarriers are cylinders, cones, or pyramids.

In some embodiments, it is desirable to use a population of synthetic nanocarriers that is relatively uniform in terms of size or shape so that each synthetic nanocarrier has similar properties. For example, at least 80%, at least 90%, or at least 95% of the synthetic nanocarriers, based on the total number of synthetic nanocarriers, may have a minimum dimension or maximum dimension that falls within 5%, 10%, or 20% of the average diameter or average dimension of the synthetic nanocarriers.

Synthetic nanocarriers can be solid or hollow and can comprise one or more layers. In some embodiments, each layer has a unique composition and unique properties relative to the other layer(s). To give but one example, synthetic nanocarriers may have a core/shell structure, wherein the core is one layer (e.g. a polymeric core) and the shell is a second layer (e.g. a lipid bilayer or monolayer). Synthetic nanocarriers may comprise a plurality of different layers.

In preferred embodiments, the synthetic nanocarriers comprise a polymer as provided herein. Polymers may be natural or unnatural (synthetic) polymers. Polymers may be homopolymers or copolymers comprising two or more monomers. In terms of sequence, copolymers may be random, block, or comprise a combination of random and block sequences. Typically, polymers in accordance with the present invention are organic polymers.

The synthetic nanocarriers as provided herein may comprise hydrophobic polyesters. Such polyesters can include copolymers comprising lactic acid and glycolic acid units, such as poly(lactic acid-co-glycolic acid) and poly(lactide-co-glycolide), collectively referred to herein as "PLGA"; and homopolymers comprising glycolic acid units, referred to herein as "PGA," and lactic acid units, such as poly-L-lactic acid, poly-D-lactic acid, poly-D,L-lactic acid, poly-L-lactide, poly-D-lactide, and poly-D,L-lactide, collectively referred to herein as "PLA." In some embodiments, exemplary polyesters include, for example, polyhydroxyacids; PEG copolymers and copolymers of lactide and glycolide (e.g., PLA-PEG copolymers, PGA-PEG copolymers, PLGA-PEG copolymers, and derivatives thereof. In some embodiments, polyesters include, for example, poly(caprolactone), poly(caprolactone)-PEG copolymers, poly(L-lactide-co-L-lysine), poly(serine ester), poly(4-hydroxy-L-proline ester), poly[$\alpha$-(4-aminobutyl)-L-glycolic acid], and derivatives thereof.

In some embodiments, the polyester may be PLGA. PLGA is a biocompatible and biodegradable co-polymer of lactic acid and glycolic acid, and various forms of PLGA are characterized by the ratio of lactic acid:glycolic acid. Lactic acid can be L-lactic acid, D-lactic acid, or D,L-lactic acid. T he degradation rate of PLGA can be adjusted by altering the lactic acid:glycolic acid ratio. In some embodiments, PLGA to be used in accordance with the present invention is characterized by a lactic acid:glycolic acid ratio of approximately 85:15, approximately 75:25, approximately 60:40, approximately 50:50, approximately 40:60, approximately 25:75, or approximately 15:85.

The synthetic nanocarriers may comprise one or more non-polyester polymers or units thereof that are also hydrophobic and/or polymers or units thereof that are not hydrophobic. In some embodiments, it is preferred that overall the synthetic nanocarrier comprises a hydrophobic polyester and, in some embodiments, is itself hydrophobic.

The synthetic nanocarriers may comprise one or more polymers that are a non-methoxy-terminated, pluronic polymer, or a unit thereof. "Non-methoxy-terminated polymer" means a polymer that has at least one terminus that ends with a moiety other than methoxy. In some embodiments, the polymer has at least two termini that ends with a moiety other than methoxy. In other embodiments, the polymer has no termini that ends with methoxy. "Non-methoxy-terminated, pluronic polymer" means a polymer other than a linear pluronic polymer with methoxy at both termini.

The synthetic nanocarriers may comprise, in some embodiments, polyhydroxyalkanoates, polyamides, polyethers, polyolefins, polyacrylates, polycarbonates, polystyrene, silicones, fluoropolymers, or a unit thereof. Further examples of polymers that may be comprised in the synthetic nanocarriers provided herein include polycarbonate, polyamide, or polyether, or unit thereof. In other embodiments, the polymers of the synthetic nanocarriers may comprise poly(ethylene glycol) (PEG), polypropylene glycol, or unit thereof.

In some embodiments, it is preferred that the synthetic nanocarriers comprise polymer that is biodegradable.

Therefore, in such embodiments, the polymers of the synthetic nanocarriers may include a polyether, such as poly(ethylene glycol) or polypropylene glycol or unit thereof. Additionally, the polymer may comprise a block-co-polymer of a polyether and a biodegradable polymer such that the polymer is biodegradable. In other embodiments, the polymer does not solely comprise a polyether or unit thereof, such as poly(ethylene glycol) or polypropylene glycol or unit thereof.

In some embodiments, polymers in accordance with the present invention include polymers which have been approved for use in humans by the U.S. Food and Drug Administration (FDA) under 21 C.F.R. § 177.2600.

Other examples of polymers suitable for use in synthetic nanocarriers include, but are not limited to polyethylenes, polycarbonates (e.g. poly(1,3-dioxan-2one)), polyanhydrides (e.g. poly(sebacic anhydride)), polypropylfumerates, polyamides (e.g. polycaprolactam), polyacetals, polyethers, polyesters (e.g., polylactide, polyglycolide, polylactide-co-glycolide, polycaprolactone, polyhydroxyacid (e.g. poly($\beta$-hydroxyalkanoate))), poly(orthoesters), polycyanoacrylates, polyvinyl alcohols, polyurethanes, polyphosphazenes, polyacrylates, polymethacrylates, polyureas, polystyrenes, and polyamines, polylysine, polylysine-PEG copolymers, and poly(ethyleneimine), polyethylene imine)-PEG copolymers.

Still other examples of polymers that may be included in the synthetic nanocarriers include acrylic polymers, for example, acrylic acid and methacrylic acid copolymers, methyl methacrylate copolymers, ethoxyethyl methacrylates, cyanoethyl methacrylate, aminoalkyl methacrylate copolymer, poly(acrylic acid), poly(methacrylic acid), methacrylic acid alkylamide copolymer, poly(methyl methacrylate), poly(methacrylic acid anhydride), methyl methacrylate, polymethacrylate, poly(methyl methacrylate) copolymer, polyacrylamide, aminoalkyl methacrylate copolymer, glycidyl methacrylate copolymers, polycyanoacrylates, and combinations comprising one or more of the foregoing polymers.

In some embodiments, the polymers of a synthetic nanocarrier associate to form a polymeric matrix. A wide variety of polymers and methods for forming polymeric matrices therefrom are known conventionally. In some embodiments, a synthetic nanocarrier comprising a hydrophobic polyester has a hydrophobic environment within the synthetic nanocarrier.

In some embodiments, polymers may be modified with one or more moieties and/or functional groups. A variety of moieties or functional groups can be used in accordance with the present invention. In some embodiments, polymers may be modified with polyethylene glycol (PEG), with a carbohydrate, and/or with acyclic polyacetals derived from polysaccharides (Papisov, 2001, ACS Symposium Series, 786:301). Certain embodiments may be made using the general teachings of US Patent No. 5543158 to Gref et al., or WO publication WO2009/051837 by Von Andrian et al.

In some embodiments, polymers may be modified with a lipid or fatty acid group. In some embodiments, a fatty acid group may be one or more of butyric, caproic, caprylic, capric, lauric, myristic, palmitic, stearic, arachidic, behenic, or lignoceric acid. In some embodiments, a fatty acid group may be one or more of palmitoleic, oleic, vaccenic, linoleic, alpha-linoleic, gamma-linoleic, arachidonic, gadoleic, arachidonic, eicosapentaenoic, docosahexaenoic, or erucic acid.

In some embodiments, polymers can be linear or branched polymers. In some embodiments, polymers can be dendrimers. In some embodiments, polymers can be substantially cross-linked to one another. In some embodiments, polymers can be substantially free of cross-links. In some embodiments, polymers can be used in accordance with the present invention without undergoing a cross-linking step. It is further to be understood that the synthetic nanocarriers may comprise block copolymers, graft copolymers, blends, mixtures, and/or adducts of any of the foregoing and other polymers. Those skilled in the art will recognize that the polymers listed herein represent an exemplary, not comprehensive, list of polymers that can be of use in accordance with the present invention provided they meet the desired criteria.

The properties of these and other polymers and methods for preparing them are well known in the art (see, for example, U.S. Patents 6,123,727; 5,804,178; 5,770,417; 5,736,372; 5,716,404; 6,095,148; 5,837,752; 5,902,599; 5,696,175; 5,514,378; 5,512,600; 5,399,665; 5,019,379; 5,010,167; 4,806,621; 4,638,045; and 4,946,929; Wang et al., 2001, J. Am. Chem. Soc., 123:9480; Lim et al., 2001, J. Am. Chem. Soc., 123:2460; Langer, 2000, Acc. Chem. Res., 33:94; Langer, 1999, J. Control. Release, 62:7; and Uhrich et al., 1999, Chem. Rev., 99:3181). More generally, a variety of methods for synthesizing certain suitable polymers are described in Concise Encyclopedia of Polymer Science and Polymeric Amines and Ammonium Salts, Ed. by Goethals, Pergamon Press, 1980; Principles of Polymerization by Odian, John Wiley & Sons, Fourth Edition, 2004; Contemporary Polymer Chemistry by Allcock et al., Prentice-Hall, 1981; Deming et al., 1997, Nature, 390:386; and in U.S. Patents 6,506,577, 6,632,922, 6,686,446, and 6,818,732.

Synthetic nanocarriers may be prepared using a wide variety of methods known in the art. For example, synthetic nanocarriers can be formed by methods such as nanoprecipitation, flow focusing using fluidic channels, spray drying, single and double emulsion solvent evaporation, solvent extraction, phase separation, milling (including cryomilling), supercritical fluid (such as supercritical carbon dioxide) processing, microemulsion procedures, microfabrication, nanofabrication, sacrificial layers, simple and complex coacervation, and other methods well known to those of ordinary skill in the art. Alternatively or additionally, aqueous and organic solvent syntheses for monodisperse semiconductor, conductive, magnetic, organic, and other nanomaterials have been described (Pellegrino et al., 2005, Small, 1:48; Murray et al., 2000, Ann. Rev. Mat. Sci., 30:545; and Trindade et al., 2001, Chem. Mat., 13:3843). Additional methods have been described in the literature (see, e.g., Doubrow, Ed., "Microcapsules and Nanoparticles in Medicine and Pharmacy," CRC Press, Boca Raton, 1992; Mathiowitz et al., 1987, J. Control. Release, 5:13; Mathiowitz et al., 1987, Reactive Polymers, 6:275; and Mathiowitz et al., 1988, J. Appl. Polymer Sci., 35:755; US Patents 5578325 and 6007845; P.

Paolicelli et al., "Surface-modified PLGA-based Nanoparticles that can Efficiently Associate and Deliver Virus-like Particles" Nanomedicine. 5(6):843-853 (2010)).

Immunosuppressants may be encapsulated into synthetic nanocarriers as desirable using a variety of methods including but not limited to C. Astete et al., "Synthesis and characterization of PLGA nanoparticles" J. Biomater. Sci. Polymer Edn, Vol. 17, No. 3, pp. 247-289 (2006); K. Avgoustakis "Pegylated Poly(Lactide) and Poly(Lactide-Co-Glycolide) Nanoparticles: Preparation, Properties and Possible Applications in Drug Delivery" Current Drug Delivery 1:321-333 (2004); C. Reis et al., "Nanoencapsulation I. Methods for preparation of drug-loaded polymeric nanoparticles" Nanomedicine 2:8- 21 (2006); P. Paolicelli et al., "Surface-modified PLGA-based Nanoparticles that can Efficiently Associate and Deliver Virus-like Particles" Nanomedicine. 5(6):843-853 (2010). Other methods suitable for encapsulating materials into synthetic nanocarriers may be used, including without limitation methods disclosed in United States Patent 6,632,671 to Unger issued October 14, 2003.

In certain embodiments, synthetic nanocarriers are prepared by a nanoprecipitation process or spray drying. Conditions used in preparing synthetic nanocarriers may be altered to yield particles of a desired size or property (e.g., hydrophobicity, hydrophilicity, external morphology, "stickiness," shape, etc.). The method of preparing the synthetic nanocarriers and the conditions (e.g., solvent, temperature, concentration, air flow rate, etc.) used may depend on the materials to be included in the synthetic nanocarriers and/or the composition of the carrier matrix.

If synthetic nanocarriers prepared by any of the above methods have a size range outside of the desired range, such synthetic nanocarriers can be sized, for example, using a sieve.

Preferably, in the methods or compositions or kits provided herein, the synthetic nanocarriers are those that comprise synthetic nanocarriers composed of PLA and PLA-PEG. In the compositions for use according to the present invention, the synthetic nanocarriers preferably are those that comprise synthetic nanocarriers composed of PLA and PLA-PEG. PLA is part of the broader poly(lactic co glycolic acid), or PLGA, family of biodegradable polymers that have more than 30 years of commercial use and are formulation components in a number of approved products. Polyethylene glycol, or PEG, has been widely studied in clinical trials and is also a formulation component in many approved biologic products.

As examples, the synthetic nanocarriers comprising rapamycin are those produced or obtainable by one of the following methods:

1) PLA with an inherent viscosity of 0.41 dL/g is purchased from Evonik Industries (Rellinghauser Straße 1-11 45128 Essen, Germany), product code Resomer Select 100 DL 4A. PLA-PEG-OMe block co-polymer with a methyl ether terminated PEG block of approximately 5,000 Da and an overall inherent viscosity of 0.50 DL/g is purchased from Evonik Industries (Rellinghauser Straße 1-11 45128 Essen, Germany), product code Resomer Select 100 DL mPEG 5000 (15 wt% PEG). Rapamycin is purchased from Concord Biotech Limited (1482-1486 Trasad Road, Dholka 382225, Ahmedabad India), product code SIROLIMUS. EMPROVE® Polyvinyl Alcohol 4-88, USP (85-89% hydrolyzed, viscosity of 3.4-4.6 mPa·s) is purchased from MilliporeSigma (EMD Millipore, 290 Concord Road Billerica, Massachusetts 01821), product code 1.41350. Dulbecco's phosphate buffered saline 1X (DPBS) is purchased from Lonza (Muenchensteinerstrasse 38, CH-4002 Basel, Switzerland), product code 17-512Q. Sorbitan monopalmitate is purchased from Croda International (300-A Columbus Circle, Edison, NJ 08837), product code SPAN 40. Solutions are prepared as follows. Solution 1 is prepared by dissolving PLA at 150 mg/mL and PLA-PEG-Ome at 50 mg/mL in dichloromethane. Solution 2 is prepared by dissolving rapamycin at 100 mg/mL in dichloromethane. Solution 3 is prepared by dissolving SPAN 40 at 50 mg/mL in dichloromethane. Solution 4 is prepared by dissolving PVA at 75 mg/mL in 100 mM phosphate buffer pH 8. O/W emulsions are prepared by adding Solution 1 (0.50 mL), Solution 2 (0.12 mL), Solution 3 (0.10 mL), and dichloromethane (0.28 mL), in a thick walled glass pressure tube. The combined organic phase solutions are then mixed by repeat pipetting. To this mixture, Solution 4 (3 mL), is added. The pressure tube is then vortex mixed for 10 seconds. Next, the crude emulsion is homogenized by sonication at 30% amplitude for 1 minute using a Branson Digital Sonifier 250 with a 1/8" tapered tip, and the pressure tube immersed in an ice water bath. The emulsion is then added to a 50 mL beaker containing DPBS (30 mL). This is stirred at room temperature for 2 hours to allow the dichloromethane to evaporate and for the nanocarriers to form. A portion of the nanocarriers is washed by transferring the nanocarrier suspension to a centrifuge tube and centrifuging at 75,600×g at 4 °C for 50 minutes, removing the supernatant, and re-suspended the pellet in DPBS containing 0.25% w/v PVA. The wash procedure is repeated and the pellet is re-suspended in DPBS containing 0.25% w/v PVA to achieve a nanocarrier suspension having a nominal concentration of 10 mg/mL on a polymer basis. The nanocarrier suspension is then filtered using a 0.22 μm PES membrane syringe filter from MilliporeSigma (EMD Millipore, 290 Concord Rd. Billerica MA, product code SLGP033RB). The filtered nanocarrier suspension is stored at -20°C.

2) PLA with an inherent viscosity of 0.41 dL/g is purchased from Evonik Industries (Rellinghauser Straße 1-11 45128 Essen, Germany), product code Resomer Select 100 DL 4A. PLA-PEG-OMe block co-polymer with a methyl ether terminated PEG block of approximately 5,000 Da and an overall inherent viscosity of 0.50 DL/g is purchased from Evonik Industries (Rellinghauser Straße 1-11 45128 Essen, Germany), product code Resomer Select 100 DL mPEG 5000 (15 wt% PEG). Rapamycin is purchased from Concord Biotech Limited (1482-1486 Trasad Road, Dholka 382225, Ahmedabad India), product code SIROLIMUS. Sorbitan monopalmitate is purchased from Sigma-Aldrich (3050 Spruce St., St. Louis, MO 63103), product code 388920. EMPROVE® Polyvinyl Alcohol (PVA) 4-88, USP (85-89% hydrolyzed, viscosity of 3.4-4.6 mPa·s) is purchased from MilliporeSigma (EMD Millipore, 290 Concord Road Billerica, Massachusetts 01821), product code 1.41350. Dulbecco's phosphate buffered saline 1X (DPBS) is purchased from Lonza (Muenchensteinerstrasse 38, CH-4002 Basel, Switzerland), product code 17-512Q. Solutions are prepared as follows: Solution 1: A polymer, rapamycin, and sorbitan monopalmitate mixture is prepared by dissolving PLA at 37.5 mg/mL, PLA-PEG-Ome at 12.5 mg/mL, rapamycin at 8 mg/mL, and sorbitan monopalmitate at 2.5 in dichloromethane. Solution 2: Polyvinyl alcohol is prepared at 50 mg/mL in 100 mM pH 8 phosphate buffer. An O/W emulsion is prepared by combining Solution 1 (1.0 mL) and Solution 2 (3 mL) in a small glass pressure tube, and vortex mixed for 10 seconds. The formulation is then homogenized by sonication at 30% amplitude for 1 minute using a Branson Digital Sonifier 250 with a 1/8" tapered tip, with the pressure tube immersed in an ice water bath. The emulsion is then added to a 50 mL beaker containing DPBS (15 mL), and covered with aluminum foil. A second O/W emulsion is prepared using the same materials and method as above and then added to the same beaker using a fresh aliquot of DPBS (15 mL). The combined emulsion is then left uncovered and stirred at room temperature for 2 hours to allow the dichloromethane to evaporate and for the nanocarriers to form. A portion of the nanocarriers is washed by transferring the nanocarrier suspension to a centrifuge tube and centrifuging at $75,600 \times g$ and 4 °C for 50 minutes, removing the supernatant, and re-suspending the pellet in DPBS containing 0.25% w/v PVA. The wash procedure is repeated and then the pellet re-suspended in DPBS containing 0.25% w/v PVA to achieve a nanocarrier suspension having a nominal concentration of 10 mg/mL on a polymer basis. The nanocarrier suspension is then filtered using a 0.22 $\mu$m PES membrane syringe filter from MilliporeSigma (EMD Millipore, 290 Concord Rd. Billerica MA, product code SLGP033RB). The filtered nanocarrier suspension is then stored at -20°C.

*Immunosuppressants*

Any immunosuppressant as disclosed herein can be used in any one of the methods or compositions disclosed herein and can be, in some embodiments, attached to synthetic nanocarriers. Immunosuppressants include, but are not limited to, mTOR inhibitors. Examples of mTOR inhibitors include rapamycin and rapalogs (e.g., CCL-779, RAD001, AP23573, C20-methallylrapamycin (C20-Marap), C16-(S)-butylsulfonamidorapamycin (C16-BSrap), C16-(S)-3-methylindolerapamycin (C16-iRap) (Bayle et al. Chemistry & Biology 2006, 13:99-107)), AZD8055, BEZ235 (NVP-BEZ235), chrysophanic acid (chrysophanol), deforolimus (MK-8669), everolimus (RAD0001), KU-0063794, PI-103, PP242, temsirolimus, and WYE-354 (available from Selleck, Houston, TX, USA).

Preferably, in the methods or compositions or kits disclosed herein, the immunosuppressant is rapamycin. In a composition for use according to the present invention, the immunosuppressant is rapamycin. The rapamycin may be encapsulated in the synthetic nanocarriers. Rapamycin is the active ingredient of Rapamune, an immunosuppressant which has extensive prior use in humans and is currently FDA approved for prophylaxis of organ rejection in kidney transplant patients aged 13 or older.

When coupled to a synthetic nanocarrier, the amount of the immunosuppressant coupled to the synthetic nanocarrier based on the total dry recipe weight of materials in an entire synthetic nanocarrier (weight/weight), is as described elsewhere herein. Preferably, in some embodiments of any one of the methods or compositions or kits provided herein, the load of the immunosuppressant, such as rapamycin or rapalog, is between 7% and 12% or 8% and 12% by weight.

*Dosing*

Unless otherwise specified herein, the amount (by weight) of a dose of a composition comprising pegylated uricase as well as the concentrations per vial provided herein refers to the amount or concentration of the uricase protein, respectively, not including the PEG molecules conjugated thereto or any added excipients in the composition. The actual amount of the pegylated uricase, in such instances, will be higher than the dose described due to the higher weight of the pegylated protein form. In one example, a dose of 0.4 mg/kg of a composition comprising pegylated uricase refers to a dose of 0.4 mg/kg uricase protein.

Thus, a dose of a composition comprising pegylated uricase for administration to a subject may be calculated based on the dose provided herein and the weight of the subject, according to the following equation:

(dose in mg/kg (this is of the uricase protein))×(subject weight (kg))/(concentration per mL in vial (again this is of the uricase protein))=volume to be administered

As an example, the pegylated uricase may be reconstituted in sterile water to a concentration of 6 mg/mL. Thus, for this example, for a dose of 0.4 mg/kg to be administered to a subject weighing 90.7 kg (200 lbs), 6.048 mL of the reconstituted pegylated uricase composition should be administered to the subject:

$$(0.4 \text{ mg/kg}) \times (90.7 \text{ kg}) / (6 \text{ mg/mL}) = 6.048 \text{ mL}$$

In some embodiments, the appropriate volume of the composition comprising pegylated uricase is diluted in a pharmaceutically acceptable excipient (e.g., sterile saline solution) for, for example, intravenous infusion to a subject over a desired period of time (e.g., 60 minutes).

Similarly, unless otherwise specified herein, the amount (by weight) of a dose of a composition comprising synthetic nanocarriers comprising an immunosuppressant as well as the concentrations per vial as provided herein refers to the amount or concentration of the immunosuppressant, respectively, and not including the synthetic nanocarrier material or any added excipients or other components in the composition. The actual amount of the synthetic nanocarrier composition comprising the immunosuppressant will be higher than the dose described due to the added weight of the synthetic nanocarrier material and any added excipients or other components in the composition. In one example, a dose of 0.08 mg/kg of a composition comprising synthetic nanocarriers comprising an immunosuppressant refers to a dose of 0.08 mg/kg immunosuppressant.

Thus, a dose of a composition comprising synthetic nanocarriers comprising an immunosuppressant for administration to a subject may be calculated based on the weight of the subject, according to the following equation:

(dose in mg/kg (this is of the immunosuppressant))×(subject weight (kg))/(concentration per mL in vial (again this is of the immunosuppressant))=volume to be administered

As an example, the composition comprising synthetic nanocarriers comprising an immunosuppressant is at a concentration of 2 mg/mL (again this is the concentration of the immunosuppressant). Thus, for this example, for a dose of 0.08 mg/kg to be administered to a subject weighing 90.7 kg (200 lbs), 3.6 mL of the composition should be administered to the subject:

$$(0.08 \text{ mg/kg}) \times (90.7 \text{ kg}) / (2 \text{ mg/mL}) = 3.6 \text{ mL}$$

The load of the immunosuppressant (e.g., rapamycin) of the synthetic nanocarriers comprising an immunosuppresant may be determined by extracting the immunosuppressant from the synthetic nanocarriers using liquid liquid extraction compatible with both the immunosuppressant and the synthetic nanocarriers (e.g., polymers comprising the synthetic nanocarriers) and analyzing the extract by reverse phase liquid chromatography with UV detection specific for the analyte. The immunosuppressant load (content of the synthetic nanocarriers) may be accurately and precisely calculated from a calibration standard curve of a qualified reference standard prepared in conditions compatible with the chromatography and the nanoparticle extraction procedure and analyzed concomitantly.

The amount (by weight) of a dose of a composition comprising synthetic nanocarriers comprising an immunosuppressant may be calculated based on the amount (by weight) of the immunosuppressant dose, according to the following equation:

(1/load of immunosuppressant) × (dose given based on the amount of immunosuppressant) =dose of immunosuppressant given as the amount of the synthetic nanocarriers comprising the immunosuppressant

As an example, the load of immunosuppressant in the synthetic nanocarriers can be about 10% and if a dose of 0.08 mg/kg of the immunosuppressant is desired, the dose given as the amount of the synthetic nanocarriers comprising the immunosuppressant is 8 mg/kg.

The amount of uricase protein present in a pegylated uricase may be determined using methods known in the art, for example colorimetry, UV absorbance or amino acid analysis. The colorimetric approach relies on a standardized kit commercially available leveraging typical dye based reactions such as those described for Bradford or bicinchoninic acid (BCA) assays. The uricase protein quantity is accurately and precisely calculated from a calibration standard curve of a qualified protein reference standard, preferably purchased from compendial sources, and analyzed concomitantly using the same spectrophotometer. Single or multiple point calibration of a known protein of similar or different chemical properties may be run within the same assay to ensure consistency of the read out at the chosen UV absorbance. The amino acid mixture obtained from acid hydrolysis of the drug product may also be analyzed and generally provides a precise and accurate quantification. The amino acid mixture is analyzed by HPLC with either UV or fluorescence detection and using pre-chromatography or post-chromatography derivatization of the primary and secondary amines. Commercially available mixtures of common amino acids are analyzed within the same assay to build the individual amino acid calibration curves against which each amino acid is quantified. In some embodiments, the determination of the uricase protein quantity is supplemented by measuring the enzyme activity, which may be performed by measuring the decrease of an excess of uric acid monitored by UV absorbance at 595 nm. Alternatively or in addition, the uricase activity can be determined using a commercially available kit, which may involve, for example, labeling the enzymatic reaction product and measuring the response of the uricase against a calibration curve established by analyzing a known quantity of the enzyme.

Similar to the immediately above formula, the amount (by weight) of a dose of a composition comprising pegylated uricase can be calculated based on the amount (by weight) of the uricase dose, according to the following equation:

(1-(weight or uricase of a pegylated uricase/weight of the pegylated uricase))×(dose given based on the amount of uricase)=dose of pegylated uricase given as the amount of the pegylated uricase

It should be understood that the amount provided herein can be an average amount based on a population of the respective molecules in a composition.

Exemplary doses of uricase for the compositions disclosed herein comprising uricase, such as pegsiticase, as disclosed herein can be 0.10 mg/kg, 0.11 mg/kg, 0.12 mg/kg, 0.13 mg/kg, 0.14 mg/kg, 0.15 mg/kg, 0.16 mg/kg, 0.17 mg/kg, 0.18 mg/kg, 0.19 mg/kg, 0.20 mg/kg, 0.21 mg/kg, 0.22 mg/kg, 0.23 mg/kg, 0.24 mg/kg, 0.25 mg/kg, 0.26 mg/kg, 0.27 mg/kg, 0.28 mg/kg, 0.29 mg/kg, 0.30 mg/kg, 0.31 mg/kg, 0.32 mg/kg, 0.34 mg/kg, 0.35 mg/kg, 0.36 mg/kg, 0.37 mg/kg, 0.38 mg/kg, 0.39 mg/kg, 0.40 mg/kg, 0.41 mg/kg, 0.42 mg/kg, 0.43 mg/kg, 0.44 mg/kg, 0.45 mg/kg, 0.46 mg/kg, 0.47 mg/kg, 0.48 mg/kg, 0.49 mg/kg, 0.50 mg/kg, 0.51 mg/kg, 0.52 mg/kg, 0.53 mg/kg, 0.54 mg/kg, 0.55 mg/kg, 0.56 mg/kg, 0.57 mg/kg, 0.58 mg/kg, 0.59 mg/kg, 0.60 mg/kg, 0.61 mg/kg, 0.62 mg/kg, 0.63 mg/kg,0.64 mg/kg, 0.65 mg/kg, 0.66 mg/kg, 0.67 mg/kg, 0.68 mg/kg, 0.69 mg/kg, 0.70 mg/kg, 0.71 mg/kg, 0.72 mg/kg, 0.73 mg/kg, 0.74 mg/kg, 0.75 mg/kg, 0.76 mg/kg, 0.77 mg/kg, 0.78 mg/kg, 0.79 mg/kg, 0.80 mg/kg, 0.81 mg/kg, 0.82 mg/kg, 0.83 mg/kg, 0.84 mg/kg, 0.85 mg/kg, 0.86 mg/kg, 0.87 mg/kg, 0.88 mg/kg, 0.89 mg/kg, 0.90 mg/kg, 0.91 mg/kg, 0.92 mg/kg, 0.93 mg/kg, 0.94 mg/kg, 0.95 mg/kg, 0.96 mg/kg, 0.97 mg/kg, 0.98 mg/kg, 0.90 mg/kg, 1.0 mg/kg, 1.01 mg/kg, 1.02 mg/kg, 1.03 mg/kg, 1.04 mg/kg, 1.05 mg/kg, 1.06 mg/kg, 1.07 mg/kg, 1.08 mg/kg, 1.09 mg/kg, 1.10 mg/kg, 1.11 mg/kg, 1.12 mg/kg, 1.13 mg/kg, 1.14 mg/kg, 1.15 mg/kg, 1.16 mg/kg, 1.17 mg/kg, 1.18 mg/kg, 1.19 mg/kg, or 1.20 mg/kg uricase.

Exemplary doses of rapamycin for the compositions provided herein comprising synthetic nanocarriers comprising rapamycin as provided herein can be 0.050 mg/kg, 0.055 mg/kg, 0.060 mg/kg, 0.065 mg/kg, 0.070 mg/kg, 0.075 mg/kg, 0.080 mg/kg, 0.085 mg/kg, 0.090 mg/kg, 0.095 mg/kg, 0.100 mg/kg, 0.105 mg/kg, 0.110 mg/kg, 0.115 mg/kg, 0.120 mg/kg, 0.125 mg/kg, 0.130 mg/kg, 0.135 mg/kg, 0.140 mg/kg, 0.145 mg/kg, 0.150 mg/kg, 0.155 mg/kg, 0.160 mg/kg, 0.165 mg/kg, 0.170 mg/kg, 0.175 mg/kg, 0.180 mg/kg, 0.185 mg/kg, 0.190 mg/kg, 0.195 mg/kg, 0.200 mg/kg, 0.205 mg/kg, 0.210 mg/kg, 0.215 mg/kg, 0.220 mg/kg, 0.225 mg/kg, 0.230 mg/kg, 0.235 mg/kg, 0.240 mg/kg, 0.245 mg/kg, 0.250 mg/kg, 0.255 mg/kg, 0.260 mg/kg, 0.265 mg/kg, 0.270 mg/kg, 0.275 mg/kg, 0.280 mg/kg, 0.285 mg/kg, 0.290 mg/kg, 0.295 mg/kg, 0.300 mg/kg, 0.305 mg/kg, 0.310 mg/kg, 0.315 mg/kg, 0.320 mg/kg, 0.325 mg/kg, 0.330 mg/kg, 0.335 mg/kg, 0.340 mg/kg, 0.345 mg/kg, 0.350 mg/kg, 0.355 mg/kg, 0.360 mg/kg, 0.365 mg/kg, 0.370 mg/kg, 0.375 mg/kg, 0.380 mg/kg, 0.385 mg/kg, 0.390 mg/kg, 0.395 mg/kg, 0.400 mg/kg, 0.405 mg/kg, 0.410 mg/kg, 0.415 mg/kg, 0.420 mg/kg, 0.425 mg/kg, 0.430 mg/kg, 0.435 mg/kg, 0.440 mg/kg, 0.445 mg/kg, 0.450 mg/kg, 0.455 mg/kg, 0.460 mg/kg, 0.465 mg/kg, 0.470 mg/kg, 0.475 mg/kg, 0.480 mg/kg, 0.485 mg/kg, 0.490 mg/kg, 0.495 mg/kg, 0.500 mg/kg rapamycin.

Exemplary doses of compositions provided herein comprising synthetic nanocarriers comprising rapamycin as provided herein can be 0.55 mg/kg, 0.56 mg/kg, 0.57 mg/kg, 0.58 mg/kg, 0.59 mg/kg, 0.60 mg/kg, 0.61 mg/kg, 0.62 mg/kg, 0.63 mg/kg,0.64 mg/kg, 0.65 mg/kg, 0.66 mg/kg, 0.67 mg/kg, 0.68 mg/kg, 0.69 mg/kg, 0.70 mg/kg, 0.71 mg/kg, 0.72 mg/kg, 0.73 mg/kg, 0.74 mg/kg, 0.75 mg/kg, 0.76 mg/kg, 0.77 mg/kg, 0.78 mg/kg, 0.79 mg/kg, 0.80 mg/kg, 0.81 mg/kg, 0.82 mg/kg, 0.83 mg/kg, 0.84 mg/kg, 0.85 mg/kg, 0.86 mg/kg, 0.87 mg/kg, 0.88 mg/kg, 0.89 mg/kg, 0.90 mg/kg, 0.91 mg/kg, 0.92 mg/kg, 0.93 mg/kg, 0.94 mg/kg, 0.95 mg/kg, 0.96 mg/kg, 0.97 mg/kg, 0.98 mg/kg, 0.90 mg/kg, 1.0 mg/kg, 1.01 mg/kg, 1.02 mg/kg, 1.03 mg/kg, 1.04 mg/kg, 1.05 mg/kg, 1.06 mg/kg, 1.07 mg/kg, 1.08 mg/kg, 1.09 mg/kg, 1.10 mg/kg, 1.11 mg/kg, 1.12 mg/kg, 1.13 mg/kg, 1.14 mg/kg, 1.15 mg/kg, 1.16 mg/kg, 1.17 mg/kg, 1.18 mg/kg, 1.19 mg/kg, 1.20 mg/kg, 1.21 mg/kg, 1.22 mg/kg, 1.23 mg/kg, 1.24 mg/kg, 1.25 mg/kg, 1.26 mg/kg, 1.27 mg/kg, 1.28 mg/kg, 1.29 mg/kg, 1.30 mg/kg, 1.31 mg/kg, 1.32 mg/kg, 1.33 mg/kg, 1.34 mg/kg, 1.35 mg/kg, 1.36 mg/kg, 1.37 mg/kg, 1.38 mg/kg, 1.39 mg/kg, 1.40 mg/kg, 1.41 mg/kg, 1.42 mg/kg, 1.43 mg/kg, 1.44 mg/kg, 1.45 mg/kg, 1.46 mg/kg, 1.47 mg/kg, 1.48 mg/kg, 1.49 mg/kg, 1.50 mg/kg, 1.51 mg/kg, 1.52 mg/kg, 1.53 mg/kg, 1.54 mg/kg, 1.55 mg/kg, 1.56 mg/kg, 1.57 mg/kg, 1.58 mg/kg, 1.59 mg/kg, 1.60 mg/kg, 1.61 mg/kg, 1.62 mg/kg, 1.63 mg/kg, 1.64 mg/kg, 1.65 mg/kg, 1.66 mg/kg, 1.67 mg/kg, 1.68 mg/kg, 1.69 mg/kg, 1.70 mg/kg, 1.71 mg/kg, 1.72 mg/kg, 1.73 mg/kg, 1.74 mg/kg, 1.75 mg/kg, 1.76 mg/kg, 1.77 mg/kg, 1.78 mg/kg, 1.79 mg/kg, 1.80 mg/kg, 1.81 mg/kg, 1.82 mg/kg, 1.83 mg/kg, 1.84 mg/kg, 1.85 mg/kg, 1.86 mg/kg, 1.87 mg/kg, 1.88 mg/kg, 1.89 mg/kg, 1.90 mg/kg, 1.91 mg/kg, 1.92 mg/kg, 1.93 mg/kg, 1.94 mg/kg, 1.95 mg/kg, 1.96 mg/kg, 1.97 mg/kg, 1.98 mg/kg, 1.99 mg/kg, 2.00 mg/kg, 2.01 mg/kg, 2.02 mg/kg, 2.03 mg/kg, 2.04 mg/kg, 2.05 mg/kg, 2.06 mg/kg, 2.07 mg/kg, 2.08 mg/kg, 2.09 mg/kg, 2.10 mg/kg, 2.11 mg/kg, 2.12 mg/kg, 2.13 mg/kg, 2.14 mg/kg, 2.15 mg/kg, 2.16 mg/kg, 2.17 mg/kg, 2.18 mg/kg, 2.19 mg/kg, 2.20 mg/kg, 2.21 mg/kg, 2.22 mg/kg, 2.23 mg/kg, 2.24 mg/kg, 2.25 mg/kg, 2.26 mg/kg, 2.27 mg/kg, 2.28 mg/kg, 2.29 mg/kg, 2.30 mg/kg, 2.31 mg/kg, 2.32 mg/kg, 2.33 mg/kg, 2.34 mg/kg, 2.35 mg/kg, 2.36 mg/kg, 2.37 mg/kg, 2.38 mg/kg, 2.39 mg/kg, 2.40 mg/kg, 2.41 mg/kg, 2.42 mg/kg, 2.43 mg/kg, 2.44 mg/kg, 2.45 mg/kg, 2.46 mg/kg, 2.47 mg/kg, 2.48 mg/kg, 2.49 mg/kg, 2.50 mg/kg, 2.51 mg/kg, 2.52 mg/kg, 2.53 mg/kg, 2.54 mg/kg, 2.55 mg/kg, 2.56 mg/kg, 2.57 mg/kg, 2.58 mg/kg, 2.59 mg/kg, 2.60 mg/kg, 2.61 mg/kg, 2.62 mg/kg, 2.63 mg/kg, 2.64 mg/kg, 2.65 mg/kg, 2.66 mg/kg, 2.67 mg/kg, 2.68 mg/kg, 2.69 mg/kg, 2.70 mg/kg, 2.71 mg/kg, 2.72 mg/kg, 2.73 mg/kg, 2.74 mg/kg, 2.75 mg/kg, 2.76 mg/kg, 2.77 mg/kg, 2.78 mg/kg, 2.79 mg/kg, 2.80 mg/kg, 2.81 mg/kg, 2.82 mg/kg, 2.83 mg/kg, 2.84 mg/kg, 2.85 mg/kg, 2.86 mg/kg, 2.87 mg/kg, 2.88 mg/kg, 2.89 mg/kg, 2.90 mg/kg, 2.91 mg/kg, 2.92 mg/kg, 2.93 mg/kg, 2.94 mg/kg, 2.95 mg/kg, 2.96 mg/kg, 2.97 mg/kg, 2.98 mg/kg, 2.99 mg/kg, 3.00 mg/kg, 3.01 mg/kg, 3.02 mg/kg, 3.03 mg/kg, 3.04 mg/kg, 3.05 mg/kg, 3.06 mg/kg, 3.07 mg/kg, 3.08 mg/kg, 3.09 mg/kg, 3.10 mg/kg, 3.11 mg/kg, 3.12 mg/kg, 3.13 mg/kg, 3.14 mg/kg, 3.15 mg/kg, 3.16 mg/kg, 3.17 mg/kg, 3.18 mg/kg, 3.19 mg/kg, 3.20 mg/kg, 3.21 mg/kg, 3.22 mg/kg, 3.23 mg/kg, 3.24 mg/kg, 3.25 mg/kg, 3.26 mg/kg, 3.27 mg/kg, 3.28 mg/kg, 3.29 mg/kg, 3.30 mg/kg, 3.31 mg/kg, 3.32 mg/kg, 3.33 mg/kg, 3.34 mg/kg, 3.35 mg/kg, 3.36 mg/kg, 3.37 mg/kg, 3.38 mg/kg, 3.39 mg/kg, 3.40 mg/kg, 3.41 mg/kg, 3.42 mg/kg, 3.43 mg/kg, 3.44 mg/kg, 3.45 mg/kg, 3.46 mg/kg, 3.47 mg/kg, 3.48 mg/kg, 3.49 mg/kg, 3.50 mg/kg, 3.51 mg/kg, 3.52 mg/kg, 3.53 mg/kg, 3.54 mg/kg, 3.55 mg/kg, 3.56 mg/kg, 3.57 mg/kg, 3.58 mg/kg, 3.59 mg/kg, 3.60 mg/kg, 3.61 mg/kg, 3.62 mg/kg, 3.63 mg/kg, 3.64 mg/kg, 3.65 mg/kg, 3.66 mg/kg, 3.67 mg/kg, 3.68 mg/kg, 3.69 mg/kg, 3.70 mg/kg, 3.71 mg/kg, 3.72 mg/kg, 3.73 mg/kg, 3.74 mg/kg, 3.75 mg/kg, 3.76 mg/kg, 3.77 mg/kg, 3.78 mg/kg, 3.79 mg/kg, 3.80 mg/kg, 3.81 mg/kg, 3.82 mg/kg, 3.83 mg/kg, 3.84 mg/kg, 3.85 mg/kg, 3.86 mg/kg, 3.87 mg/kg, 3.88 mg/kg, 3.89 mg/kg, 3.90 mg/kg, 3.91 mg/kg, 3.92 mg/kg, 3.93 mg/kg, 3.94 mg/kg, 3.95 mg/kg, 3.96 mg/kg, 3.97 mg/kg, 3.98 mg/kg, 3.99 mg/kg, 4.00 mg/kg, 4.01 mg/kg, 4.02 mg/kg, 4.03 mg/kg, 4.04 mg/kg, 4.05 mg/kg, 4.06 mg/kg, 4.07 mg/kg, 4.08 mg/kg, 4.09 mg/kg, 4.10 mg/kg, 4.11 mg/kg, 4.12 mg/kg, 4.13 mg/kg, 4.14 mg/kg, 4.15 mg/kg, 4.16 mg/kg, 4.17 mg/kg, 4.18 mg/kg, 4.19 mg/kg, 4.20 mg/kg, 4.21 mg/kg, 4.22 mg/kg, 4.23 mg/kg, 4.24 mg/kg, 4.25 mg/kg, 4.26 mg/kg, 4.27 mg/kg, 4.28 mg/kg, 4.29 mg/kg, 4.30 mg/kg, 4.31 mg/kg, 4.32 mg/kg, 4.33 mg/kg, 4.34 mg/kg, 4.35 mg/kg, 4.36 mg/kg, 4.37 mg/kg, 4.38 mg/kg, 4.39 mg/kg, 4.40 mg/kg, 4.41 mg/kg, 4.42 mg/kg, 4.43 mg/kg, 4.44 mg/kg, 4.45 mg/kg, 4.46 mg/kg, 4.47 mg/kg, 4.48 mg/kg, 4.49 mg/kg, 4.50 mg/kg, 4.51 mg/kg, 4.52 mg/kg, 4.53 mg/kg, 4.54 mg/kg, 4.55 mg/kg, 4.56 mg/kg, 4.57 mg/kg, 4.58 mg/kg, 4.59 mg/kg, 4.60 mg/kg, 4.61 mg/kg, 4.62 mg/kg, 4.63 mg/kg, 4.64 mg/kg, 4.65 mg/kg, 4.66 mg/kg, 4.67 mg/kg, 4.68 mg/kg, 4.69 mg/kg, 4.70 mg/kg, 4.71 mg/kg, 4.72 mg/kg, 4.73 mg/kg, 4.74 mg/kg, 4.75 mg/kg, 4.76 mg/kg, 4.77 mg/kg, 4.78 mg/kg, 4.79 mg/kg, 4.80 mg/kg, 4.81 mg/kg, 4.82 mg/kg, 4.83 mg/kg, 4.84 mg/kg, 4.85 mg/kg, 4.86 mg/kg, 4.87 mg/kg, 4.88 mg/kg, 4.89 mg/kg, 4.90 mg/kg, 4.91 mg/kg, 4.92 mg/kg, 4.93 mg/kg, 4.94 mg/kg, 4.95 mg/kg, 4.96 mg/kg, 4.97 mg/kg, 4.98 mg/kg, 4.99 mg/kg, 5.00 mg/kg, 5.01 mg/kg, 5.02 mg/kg, 5.03 mg/kg, 5.04 mg/kg, 5.05 mg/kg, 5.06 mg/kg, 5.07 mg/kg, 5.08 mg/kg, 5.09 mg/kg, 5.10 mg/kg, 5.11 mg/kg, 5.12 mg/kg, 5.13 mg/kg, 5.14 mg/kg, 5.15 mg/kg, 5.16 mg/kg, 5.17 mg/kg, 5.18 mg/kg, 5.19 mg/kg, 5.20 mg/kg, 5.21 mg/kg, 5.22 mg/kg, 5.23 mg/kg, 5.24 mg/kg, 5.25 mg/kg, 5.26 mg/kg, 5.27 mg/kg, 5.28 mg/kg, 5.29 mg/kg, 5.30 mg/kg, 5.31 mg/kg, 5.32 mg/kg, 5.33 mg/kg, 5.34 mg/kg, 5.35 mg/kg, 5.36 mg/kg, 5.37 mg/kg, 5.38 mg/kg, 5.39 mg/kg, 5.40 mg/kg, 5.41 mg/kg, 5.42 mg/kg, 5.43 mg/kg, 5.44 mg/kg, 5.45 mg/kg, 5.46 mg/kg, 5.47 mg/kg, 5.48 mg/kg, 5.49 mg/kg, 5.50 mg/kg, 5.51 mg/kg, 5.52 mg/kg, 5.53 mg/kg, 5.54 mg/kg, 5.55 mg/kg, 5.56 mg/kg, 5.57 mg/kg, 5.58 mg/kg, 5.59 mg/kg, 5.60 mg/kg, 5.61 mg/kg, 5.62 mg/kg, 5.63 mg/kg, 5.64 mg/kg, 5.65 mg/kg, 5.66 mg/kg, 5.67 mg/kg, 5.68 mg/kg, 5.69 mg/kg, 5.70 mg/kg, 5.71 mg/kg, 5.72 mg/kg, 5.73 mg/kg, 5.74 mg/kg, 5.75 mg/kg, 5.76 mg/kg, 5.77 mg/kg, 5.78 mg/kg, 5.79 mg/kg, 5.80 mg/kg, 5.81 mg/kg, 5.82 mg/kg, 5.83 mg/kg, 5.84 mg/kg, 5.85 mg/kg, 5.86 mg/kg, 5.87 mg/kg, 5.88 mg/kg, 5.89 mg/kg, 5.90 mg/kg, 5.91 mg/kg, 5.92 mg/kg, 5.93 mg/kg, 5.94 mg/kg, 5.95 mg/kg, 5.96 mg/kg, 5.97 mg/kg, 5.98 mg/kg, 5.99 mg/kg, 6.00 mg/kg, 6.01 mg/kg, 6.02 mg/kg, 6.03 mg/kg, 6.04 mg/kg, 6.05 mg/kg, 6.06 mg/kg, 6.07 mg/kg, 6.08 mg/kg, 6.09 mg/kg, 6.10 mg/kg, 6.11 mg/kg, 6.12 mg/kg, 6.13 mg/kg, 6.14 mg/kg, 6.15 mg/kg, 6.16 mg/kg, 6.17 mg/kg, 6.18 mg/kg, 6.19 mg/kg, 6.20 mg/kg, 6.21 mg/kg, 6.22 mg/kg, 6.23 mg/kg, 6.24 mg/kg, 6.25 mg/kg, 6.26 mg/kg, 6.27 mg/kg, 6.28 mg/kg, 6.29 mg/kg, 6.30 mg/kg, 6.31 mg/kg, 6.32 mg/kg, 6.33 mg/kg, 6.34 mg/kg, 6.35 mg/kg, 6.36 mg/kg, 6.37 mg/kg, 6.38 mg/kg, 6.39 mg/kg, 6.40 mg/kg, 6.41 mg/kg, 6.42 mg/kg, 6.43 mg/kg, 6.44 mg/kg, 6.45 mg/kg, 6.46 mg/kg, 6.47 mg/kg, 6.48 mg/kg, 6.49 mg/kg, or 6.50 mg/kg, wherein the dose is given as the mg of the synthetic nanocarriers comprising the rapamycin.

Any one of the doses provided herein for the composition comprising uricase, such as pegsiticase, can be used in any one of the methods or compositions or kits disclosed herein. Generally, when referring to a dose to be administered to a subject the dose is a label dose. Any one of the doses provided herein for the composition comprising synthetic nanocarriers comprising an immunosuppressant, such as rapamycin, can be used in any one of the methods or compositions or kits provided herein. Generally, when referring to a dose to be administered to a subject the dose is a label dose. Thus, in any one of the methods provided herein the dose(s) are label dose(s).

In any one of the methods provided herein, an additional volume (prime volume) may be used to prime the infusion line for administering any of the compositions provided herein to the subject.

Provided herein are a number of possible dosing schedules. Accordingly, any one of the subjects provided herein may be treated according to any one of the dosing schedules provided herein. As an example, any one of the subject provided herein may be treated with a composition comprising uricase, such as pegylated uricase, and/or composition comprising synthetic nanocarriers comprising an immunosuppressant, such as rapamycin, according to any one of these dosage schedules. The mode of administration for the composition(s) of any one of the treatment methods provided may be by intravenous administration, such as an intravenous infusion that, for example, may take place over about 1 hour. Additionally, any one of the methods of treatment provided herein may also include administration of an additional therapeutic, such as a uric acid lowering therapeutic, such as a uricase, or an anti-gout flare prophylactic treatment. The administration of the additional therapeutic may be according to any one of the applicable treatment regimens provided herein.

In the methods disclosed herein, the treatment with a combination of synthetic nanocarrier composition comprising immunosuppressant, such as rapamycin, with a composition comprising uricase, such as pegylated uricase, can comprise three doses of the synthetic nanocarrier composition concomitantly with the uricase-comprising composition followed by two doses of uricase without the concomitant administration of a composition comprising an immunosuppressant, such as a synthetic nanocarrier composition comprising an immunosuppressant, or without the concomitant administration of an additional therapeutic. Each dose may be administered every two to four weeks. A method is disclosed whereby any one of the subjects disclosed herein is concomitantly administered three doses of a synthetic nanocarrier composition with a uricase-comprising composition monthly for three months. This method may further comprise administering 2, 3, 4, 5, 6, 7, 8, 9 or 10 or more monthly doses of a uricase-comprising composition alone or without the concomitant administration of immunosuppressant, such as a synthetic nanocarrier composition comprising an immunosuppressant, or an additional therapeutic. In any one of the methods disclosed herein, the level of uric acid may be measured in the subject at one or more time points before, during and/or after the treatment period.

*Additional Therapeutics*

Additional therapeutics for elevated uric acid levels, gout, gout flare, or conditions associated with gout, may be administered to any one of the subjects disclosed herein, such as for the reduction of uric acid levels and/or gout treatment and/or gout flare prevention. Any one of the methods provided herein may include the administration of one or more of these additional therapeutics. Optionally, the methods disclosed herein may not comprise the concomitant administration of an additional therapeutic. Examples of additional therapeutics include, but are not limited to, the following. Other examples will be known to those of skill in the art.

Additional therapeutics include anti-inflammatory therapeutics (i.e., any therapeutic that can act to reduce inflammation). Anti-inflammatory therapeutics include, but are not limited to, corticosteroids or derivatives of cortisol (hydrocortisone). Corticosteroids include, but are not limited to, glucocorticoids and mineralocorticoids. Still other examples of corticosteroids include, but are not limited to, those that are natural (e.g., 11-Dehydrocorticosterone (11-oxocorticosterone, 17-deoxycortisone) = 21-hydroxypregn-4-ene-3,11,20-trione; 11-Deoxycorticosterone (deoxycortone, desoxycortone; 21-hydroxyprogesterone) = 21-hydroxypregn-4-ene-3,20-dione; 11-Deoxycortisol (cortodoxone, cortexolone) = $17\alpha$,21-dihydroxypregn-4-ene-3,20-dione; 11-Ketoprogesterone (11-oxoprogesterone; Ketogestin) = pregn-4-ene-3, 11,20-trione; 11β-Hydroxypregnenolone = 3β,11β-dihydroxypregn-5-en-20-one; 11β-Hydroxyprogesterone (21-deoxycorticosterone) = 11β-hydroxypregn-4-ene-3,20-dione; 11β,$17\alpha$,21-Trihydroxypregnenolone=3β,11β,$17\alpha$,21-tetrahydroxypregn-5-en-20-one; $17\alpha$,21-Dihydroxypregnenolone=3β,$17\alpha$,21-trihydroxypregn-5-en-20-one; $17\alpha$-Hydroxypregnenolone = 3β,$17\alpha$-dihydroxypregn-5-en-20-one; $17\alpha$-Hydroxyprogesterone = $17\alpha$-hydroxypregn-4-ene-3,11,20-trione; 18-Hydroxy-11-deoxycorticosterone = 18,21-dihydroxypregn-4-ene-3,20-dione; 18-Hydroxycorticosterone = 11β,18,21-trihydroxypregn-4-ene-3,20-dione; 18-Hydroxyprogesterone = 18-hydroxypregn-4-ene-3,20-dione; 21-Deoxycortisol = 11β,$17\alpha$-dihydroxypregn-4-ene-3,20-dione; 21-Deoxycortisone = $17\alpha$-hydroxypregn-4-ene-3,11,20-trione; 21-Hydroxypregnenolone (prebediolone) = 3β,21-dihydroxypregn-5-en-20-one; Aldosterone = 11β,21-dihydroxypregn-4-ene-3,18,20-trione; Corticosterone (17-deoxycortisol) = 11β,21-dihydroxypregn-4-ene-3,20-dione; Cortisol (hydrocortisone) = 11β,$17\alpha$,21-trihydroxypregn-4-ene-3,20-dione; Cortisone = $17\alpha$,21-dihydroxypregn-4-ene-3,11,20-trione; Pregnenolone = pregn-5-en-3β-ol-20-one; and Progesterone = pregn-4-ene-3,20-dione); those that are synthetic, such as progesterone-type (e.g., Flugestone (flurogestone) = $9\alpha$-fluoro-11β,$17\alpha$-dihydroxypregn-4-ene-3,20-dione; Fluorometholone = $6\alpha$-methyl-$9\alpha$-fluoro-11β,$17\alpha$-dihydroxypregna-1,4-diene-3,20-dione; Medrysone (hydroxymethylprogesterone) = $6\alpha$-methyl-11β-hydroxypregn-4-ene-3,20-dione; and Prebediolone acetate (21-acetoxypregnenolone) = 3β,21-dihydroxypregn-5-en-20-one 21-acetate) and progesterone derivative progestins (e.g., chlormadinone acetate, cyproterone acetate, medrogestone, medroxyprogesterone acetate, megestrol acetate, and segesterone acetate); hydrocortisone-type (e.g., Chloroprednisone = 6$\alpha$-chloro-17$\alpha$,21-dihydroxypregna-1,4-diene-3,11,20-trione; Cloprednol = 6-chloro-11$\beta$,17$\alpha$,21-trihydroxypregna-1,4,6-triene-3,20-dione; Difluprednate = 6$\alpha$,9$\alpha$-difluoro-11$\beta$,17$\alpha$,21-trihydroxypregna-1,4-diene-3,20-dione 17$\alpha$-butyrate 21-acetate; Fludrocortisone = 9$\alpha$-fluoro-11$\beta$,17$\alpha$,21-trihydroxypregn-4-ene-3,20-dione; Fluocinolone = 6$\alpha$,9$\alpha$-difluoro-11$\beta$,16$\alpha$,17$\alpha$,21-tetrahydroxypregna-1,4-diene-3,20-dione; Fluperolone = 9$\alpha$-fluoro-11$\beta$,17$\alpha$,21-trihydroxy-21-methylpregna-1,4-diene-3,20-dione; Fluprednisolone = 6$\alpha$-fluoro-11$\beta$,17$\alpha$,21-trihydroxypregna-1,4-diene-3,20-dione; Loteprednol = 11$\beta$,17$\alpha$,dihydroxy-21-oxa-21-chloromethylpregna-1,4-diene-3,20-dione; Methylprednisolone = 6$\alpha$-methyl-11$\beta$,17$\alpha$,21-trihydroxypregna-1,4-diene-3,20-dione; Prednicarbate = 11$\beta$,17$\alpha$,21-trihydroxypregna-1,4-diene-3,20-dione 17$\alpha$-ethylcarbonate 21-propionate; Prednisolone = 11$\beta$,17$\alpha$,21-trihydroxypregna-1,4-diene-3,20-dione; Prednisone = 17$\alpha$,21-dihydroxypregna-1,4-diene-3,11,20-trione; Tixocortol = 11$\beta$,17$\alpha$-dihydroxy-21-sulfanylpregn-4-ene-3,20-dione; and Triamcinolone = 9$\alpha$-fluoro-11$\beta$,16$\alpha$,17$\alpha$,21-tetrahydroxypregna-1,4-diene-3,20-dione); methasone-type (16-methylated) (e.g., Methasone; Alclometasone = 7$\alpha$-chloro-11$\beta$,17$\alpha$,21-trihydroxy-16$\alpha$-methylpregna-1,4-diene-3,20-dione; Beclometasone = 9$\alpha$-chloro-11$\beta$,17$\alpha$,21-trihydroxy-16$\beta$-methylpregna-1,4-diene-3,20-dione; Betamethasone = 9$\alpha$-fluoro-11$\beta$,17$\alpha$,21-trihydroxy-16$\beta$-methylpregna-1,4-diene-3,20-dione; Clobetasol = 9$\alpha$-fluoro-11$\beta$,17$\alpha$-dihydroxy-16$\beta$-methyl-21-chloropregna-1,4-diene-3,20-dione; Clobetasone = 9$\alpha$-fluoro-16$\beta$-methyl-17$\alpha$-hydroxy-21-chloropregna-1,4-diene-3,11,20-trione; Clocortolone = 6$\alpha$-fluoro-9$\alpha$-chloro-11$\beta$,21-dihydroxy-16$\alpha$-methylpregna-1,4-diene-3,20-dione; Desoximetasone = 9$\alpha$-fluoro-11$\beta$,21-dihydroxy-16$\alpha$-methylpregna-1,4-diene-3,20-dione; Dexamethasone = 9$\alpha$-fluoro-11$\beta$,17$\alpha$,21-trihydroxy-16$\alpha$-methylpregna-1,4-diene-3,20-dione; Diflorasone = 6$\alpha$,9$\alpha$-difluoro-11$\beta$,17$\alpha$,21-trihydroxy-16$\beta$-methylpregna-1,4-diene-3,20-dione; Difluocortolone = 6$\alpha$,9$\alpha$-difluoro-11$\beta$,21-dihydroxy-16$\alpha$-methylpregna-1,4-diene-3,20-dione; Fluclorolone = 6$\alpha$-fluoro-9$\alpha$,11$\beta$-dichloro-16$\alpha$,17$\alpha$,21-trihydroxypregna-1,4-dien-3,20-dione; Flumetasone = 6$\alpha$,9$\alpha$-difluoro-11$\beta$,17$\alpha$,21-trihydroxy-16$\alpha$-methylpregna-1,4-diene-3,20-dione; Fluocortin = 6$\alpha$-fluoro-11$\beta$,21-dihydroxy-16$\alpha$-methyl-pregna-1,4-diene-3,20,21-trione; Fluocortolone = 6$\alpha$-fluoro-11$\beta$,21-dihydroxy-16$\alpha$-methylpregna-1,4-diene-3,20-dione; Fluprednidene = 9$\alpha$-fluoro-11$\beta$,17$\alpha$,21-trihydroxy-16-methylenepregna-1,4-diene-3,20-dione; Fluticasone = 6$\alpha$,9$\alpha$-difluoro-11$\beta$,17$\alpha$-dihydroxy-16$\alpha$-methyl-21-thia-21-fluoromethylpregna-1,4-dien-3,20-dione; Fluticasone furoate = 6$\alpha$,9$\alpha$-difluoro-11$\beta$,17$\alpha$-dihydroxy-16$\alpha$-methyl-21 -thia-21 -fluoromethylpregna-1,4-dien-3,20-dione 17$\alpha$-(2-furoate); Halometasone = 2-chloro-6$\alpha$,9$\alpha$-difluoro-11$\beta$,17$\alpha$,21-trihydroxy-16$\alpha$-methylpregna-1,4-diene-3,20-dione; Meprednisone = 16$\beta$-methyl-17$\alpha$,21-dihydroxypregna-1,4-diene-3,11,20-trione; Mometasone = 9$\alpha$,21-dichloro-11$\beta$,17$\alpha$-dihydroxy-16$\alpha$-methylpregna-1,4-diene-3,20-dione; Mometasone furoate = 9$\alpha$,21-dichloro-11$\beta$,17$\alpha$-dihydroxy-16$\alpha$-methylpregna-1,4-diene-3,20-dione 17$\alpha$-(2-furoate); Paramethasone = 6$\alpha$-fluoro-11$\beta$,17$\alpha$,21-trihydroxy-16$\alpha$-methylpregna-1,4-diene-3,20-dione; Prednylidene = 11$\beta$,17$\alpha$,21-trihydroxy-16-methylenepregna-1,4-diene-3,20-dione; Rimexolone = 11$\beta$-hydroxy-16$\alpha$,17$\alpha$,21-trimethylpregna-1,4-dien-3,20-dione; and Ulobetasol (halobetasol) = 6$\alpha$,9$\alpha$-difluoro-11$\beta$,17$\alpha$-dihydroxy-16$\beta$-methyl-21-chloropregna-1,4-diene-3,20-dione); Acetonides and related (e.g., Amcinonide = 9$\alpha$-fluoro-11$\beta$,16$\alpha$,17$\alpha$,21-tetrahydroxypregna-1,4-diene-3,20-dione cyclic 16$\alpha$,17$\alpha$-acetal with cyclopentanone, 21-acetate; Budesonide = 11$\beta$,16$\alpha$,17$\alpha$,21-tetrahydroxypregna-1,4-diene-3,20-dione cyclic 16$\alpha$,17$\alpha$-acetal with butyraldehyde; Ciclesonide = 11$\beta$,16$\alpha$,17$\alpha$,21-tetrahydroxypregna-1,4-diene-3,20-dione cyclic 16$\alpha$,17$\alpha$-acetal with (R)-cyclohexanecarboxaldehyde, 21-isobutyrate; Deflazacort = 11$\beta$,21-dihydroxy-2'-methyl-5'H-pregna-1,4-dieno[17,16-d]oxazole-3,20-dione 21-acetate; Desonide = 11$\beta$,16$\alpha$,17$\alpha$,21-tetrahydroxypregna-1,4-diene-3,20-dione cyclic 16$\alpha$,17$\alpha$-acetal with acetone; Formocortal (fluoroformylone) = 3-(2-chloroethoxy)-9$\alpha$-fluoro-11$\beta$,16$\alpha$,17$\alpha$,21-tetrahydroxy-20-oxopregna-3,5-diene-6-carboxaldehyde cyclic 16$\alpha$,17$\alpha$-acetal with acetone, 21-acetate; Fluclorolone acetonide (flucloronide) = 6$\alpha$-fluoro-9$\alpha$,11$\beta$-dichloro-16$\alpha$,17$\alpha$,21-trihydroxypregna-1,4-dien-3,20-dione cyclic 16$\alpha$,17$\alpha$-acetal with acetone; Fludroxycortide (flurandrenolone, flurandrenolide) = 6$\alpha$-fluoro-11$\beta$,16$\alpha$,17$\alpha$,21-tetrahydroxypregn-4-ene-3,20-dione cyclic 16$\alpha$,17$\alpha$-acetal with acetone; Flunisolide = 6$\alpha$-fluoro-11$\beta$,16$\alpha$,17$\alpha$,21-tetrahydroxypregna-1,4-diene-3,20-dione cyclic 16$\alpha$,17$\alpha$-acetal with acetone; Fluocinolone acetonide = 6$\alpha$,9$\alpha$-difluoro-11$\beta$,16$\alpha$,17$\alpha$,21-tetrahydroxypregna-1,4-diene-3,20-dione cyclic 16$\alpha$,17$\alpha$-acetal with acetone; Fluocinonide = 6$\alpha$,9$\alpha$-difluoro-11$\beta$,16$\alpha$,17$\alpha$,21-tetrahydroxypregna-1,4-diene-3,20-dione cyclic 16$\alpha$,17$\alpha$-acetal with acetone, 21-acetate; Halcinonide = 9$\alpha$-fluoro-11$\beta$,16$\alpha$,17$\alpha$-trihydroxy-21-chloropregn-4-ene-3,20-dione cyclic 16$\alpha$,17$\alpha$-acetal with acetone; and Triamcinolone acetonide = 9$\alpha$-fluoro-11$\beta$,16$\alpha$,17$\alpha$,21-tetrahydroxypregna-1,4-diene-3,20-dione cyclic 16$\alpha$,17$\alpha$-acetal with acetone); and still others (e.g., Cortivazol = 6,16$\alpha$-dimethyl-11$\beta$,17$\alpha$,21-trihydroxy-2'-phenyl[3,2-c]pyrazolopregna-4,6-dien-20-one 21-acetate; and RU-28362 = 6-methyl-11$\beta$,17$\beta$-dihydroxy-17$\alpha$-(1-propynyl)androsta-1,4,6-trien-3-one).

Corticosteroids, particularly glycocorticoids, have anti-inflammatory and immunosuppressive effects that may be effective in managing symptoms, including pain and inflammation associated with gout, gout flare, and/or conditions associated with gout. Administration of corticosteroids may also aid in reducing hypersensitivity reactions associated with one or more additional therapies, for example uricase replacement therapy. Still other non-limiting examples of corticosteroids, include prednisone, prednisolone, Medrol, and methylprednisolone.

Additional therapeutics include short term therapies for gout flare or pain and inflammation associated with any of the symptoms associated with gout or a condition associated with gout include nonsteroidal anti-inflammatory drugs (NSAIDS), colchicine, oral corticosteroids. Non-limiting examples of NSAIDS include both over-the-counter NSAIDS, such as ibuprofen, aspirin, and naproxen, as well as prescription NSAIDS, such as celecoxib, diclofenac, diflunisal, etodolac, indomethacin, ketoprofen, ketorolac, nabumetrone, oxaprozin, piroxiam salsalate, sulindac, and tolmetin.

Colchicine is an anti-inflammatory agent that is generally considered as an alternative for NSAIDs for managing the symptoms, including pain and inflammation associated with gout, gout flare, and/or conditions associated with gout.

Further examples of additional therapeutics include xanthine oxidase inhibitors, which are molecules that inhibit xanithine oxidase, reducing or preventing the oxidation of xanthine to uric acid, thereby reducing the production of uric acid. Xanthine oxidase inhibitors are generally classified as either purine analogues and other types of xanthine oxidase inhibitors. Examples of xanthine oxidase inhibitors include allopurinol, oxypurinol, tisopurine, febuxostat, topiroxostat, inositols (e.g., phytic acid and myo-inositol), flavonoids (e.g., kaempferol, myricetin, quercetin), caffeic acid, and 3,4-dihydrox-5-nitrobenzaldehyde (DHNB).

Still other examples of additional therapeutics include uricosuric agents. Uricosuric agents aim to increase excretion of uric acid in order to reduce serum levels of uric acid by modulating renal tubule reabsorption. For example, some uricosuric agents modulate activity of renal transporters of uric acid (e.g., URAT1/SLC22A12 inhibitors). Non-limiting examples of uricosuric agents include probenecid, benzbromarone, lesinurad, sulfinpyrazone. Other additional therapeutics may also have uricosuric activity, such as aspirin.

Additional therapeutics also include other uricase-based therapies, which include pegylated uricase. Such therapies, such as when infused into humans, have been shown to reduce blood uric acid levels and improve gout symptoms. Rasburicase (Elitek®), an unpegylated recombinant uricase cloned from Aspergillus flavus, is approved for management of uric acid levels in patients with tumor lysis syndrome (Elitek®). KRYSTEXXA® (pegloticase) is a recombinant uricase (primarily porcine with a carboxyl-terminus sequence from baboon) bound by multiple 10 kDa PEG molecules approved for the treatment of chronic refractory gout. As mentioned elsewhere, however, the clinical experience with KRYSTEXXA® has shown that a significant number of patients will develop anti-drug antibodies which limit the long term efficacy of the drug. Thus, prior administration of KRYSTEXXA® may be a contraindication for the use of the methods provided herein.

The treatments provided herein may allow patients to switch to oral gout therapy, such as with xanthine oxidase inhibitors, unless and until such patients experience a subsequent manifestation of uric acid deposits at which time a new course of treatment as provided herein according to any one of the methods provided is then undertaken. Any one of the methods provided herein, thus, can include the subsequent administration of an oral gout therapeutic as an additional therapeutic after the treatment regimen according to any one of the methods provided is performed. It is believed that oral therapy may not completely prevent the build up over time of uric acid crystals in patients with a history of chronic tophaceous gout. As a result, it is anticipated that treatment as provided herein is likely to be required intermittently in such patients. Thus, in such subjects, the subject is also further administered one or more compositions according to any one of the methods provided herein.

The treatments disclosed herein may allow patients to subsequently be treated with a uric acid lowering therapeutic, such as a uricase. In some embodiments, without an immunosuppressant. In some embodiments, without synthetic nanocarriers comprising an immunosuppressant.

Treatment according to any one of the methods disclosed herein may also include a pre-treatment with an anti-gout flare therapeutic, such as with colchicine or NSAIDS. Accordingly, any one of the methods disclosed herein may further comprise such an anti-gout flare therapeutic whereby the anti-gout flare therapeutic is concomitantly administered with the composition comprising uricase and the composition comprising synthetic nanocarriers comprising an immunosuppressant.

Monitoring of a subject, such as the measurement of serum uric acid levels and/or ADAs, may be an additional step further comprised in any one of the methods disclosed herein. Should such subject develop an undesired immune response, the subject may be further administered one or more compositions according to any one of the methods disclosed herein. In any one of the methods disclosed herein, the subject may be monitored with dual energy computed tomography (DECT), that can be used to visualize uric acid deposits in joints and tissues. Imaging, such as with DECT, can be used to assess the efficacy of treatment with any one of the methods or compositions provided herein. As a result, any one of the methods disclosed herein can further include a step of imaging, such as with DECT. In any one of the methods disclosed herein, the subject may be one in which the gout, such as chronic tophaceous gout, or condition associated with gout has been diagnosed with such imaging, such as with DECT.

*Subjects*

Subjects provided herein can be in need of treatment according to any one of the methods or compositions or kits disclosed herein. Such subjects include those with elevated serum uric acid levels or uric acid deposits. Such subjects include those with hyperuricemia. It is within the skill of a clinician to be able to determine subjects in need of a treatment as provided herein.

In some embodiments, any one of the subjects for treatment as disclosed in any one of the methods disclosed has gout or a condition associated with gout or another condition as provided herein. In some embodiments, any one of the subjects for treatment as disclosed in any one of the methods provided the subject has had or is expected to have gout flare.

In some embodiments, the subject has or is at risk of having erosive bone disease associated with gout, cirrhosis or steathohepatitis associated with gout, or visceral gout.

In some embodiments, the subject has or is at risk of having an elevated uric acid level, e.g., an elevated plasma or serum uric acid level. When blood levels of uric acid may exceed the physiologic limit of solubility, the uric acid may crystallize in the tissues, including the joints, and may cause gout and gout-associated conditions.

In some embodiments, serum uric acid levels $\geq 5$ mg/dL, $\geq 6$ mg/dL, or $\geq 7$ mg/dL are indicative that a subject may be a candidate for treatment with any one of the methods or compositions or kits described herein. In some embodiments, such a subject has a serum level of uric acid $\geq 6$ mg/dL, for example, between 6.1 mg/dL - 15 mg/dL, between 6.1 mg/dL - 10 mg/dL, 7 mg/dL - 15 mg/dL, 7 mg/dL - 10 mg/dL, 8 mg/dL - 15 mg/dL, 8 mg/dL - 10 mg/dL, 9 mg/dL -15 mg/dL, 9 mg/dL - 10 mg/dL, 10 mg/dL-15 mg/dL, or 11 mg/dL- 14 mg/dL. In some embodiments, the subject has serum level of uric acid of about 6.1 mg/dL, 6.2 mg/dL, 6.3 mg/dL, 6.4 mg/dL, 6.5 mg/dL, 6.7 mg/dL, 6.8 mg/dL, 6.9 mg/dL, 7.0 mg/dL, 7.1 mg/dL, 7.2 mg/dL, 7.3 mg/dL, 7.4 mg/dL, 7.5 mg/dL,7.6 mg/dL 7.7 mg/dL, 7.8 mg/dL, 7.9 mg/dL, 8.0 mg/dL, 8.1 mg/dL, 8.2 mg/dL, 8.3 mg/dL, 8.4 mg/dL, 8.5 mg/dL, 8.6 mg/dL, 8.7 mg/dL, 8.8 mg/dL, 8.9 mg/dL, 9.0 mg/dL, 9.1 mg/dL, 9.2 mg/dL, 9.3 mg/dL, 9.4 mg/dL, 9.5 mg/dL, 9.6 mg/dL, 9.7 mg/dL, 9.8 mg/dL, 9.9 mg/dL, 10.0 mg/dL, 10.1 mg/dL, 10.2 mg/dL, 10.3 mg/dL, 10.4 mg/dL, 10.5 mg/dL, 10.6 mg/dL, 10.7 mg/dL, 10.8 mg/dL, 10.9 mg/dL, 11.0 mg/dL, 11.1 mg/dL, 11.2 mg/dL, 11.3 mg/dL, 11.4 mg/dL, 11.5 mg/dL, 11.6 mg/dL, 11.7 mg/dL, 11.8 mg/dL, 11.9 mg/dL, 12.0 mg/dL, 12.1 mg/dL, 12.2 mg/dL, 12.3 mg/dL, 12.4 mg/dL, 12.5 mg/dL, 12.6 mg/dL, 12.7 mg/dL, 12.8 mg/dL, 12.9 mg/dL, 13.0 mg/dL, 13.1 mg/dL, 13.2 mg/dL, 13.3 mg/dL, 13.4 mg/dL, 13.5 mg/dL, 13.6 mg/dL, 13.7 mg/dL, 13.8 mg/dL, 13.9 mg/dL, 14.0 mg/dL, 14.1 mg/dL, 14.2 mg/dL, 14.3 mg/dL, 14.4 mg/dL, 14.5 mg/dL, 14.6 mg/dL, 14.7 mg/dL, 14.8 mg/dL, 14.9 mg/dL, 15.0 mg/dL or higher. In some embodiments, the subject has a plasma or serum uric acid level of 5.0 mg/dL, 5.1 mg/dL, 5.2 mg/dL, 5.3 mg/dL, 5.4 mg/dL, 5.5 mg/dL, 5.6 mg/dL, 5.7 mg/dL, 5.8 mg/dL, 5.9 mg/dL, 6.0 mg/dL, 6.1 mg/dL, 6.2 mg/dL, 6.3 mg/dL, 6.4 mg/dL, 6.5 mg/dL, 6.6 mg/dL, 6.7 mg/dL, 6.8 mg/dL, 6.9 mg/dL, or 7.0 mg/dL. In some embodiments, the subject has a plasma or serum uric acid level of greater than or equal to 5.0 mg/dL, 5.1 mg/dL, 5.2 mg/dL, 5.3 mg/dL, 5.4 mg/dL, 5.5 mg/dL, 5.6 mg/dL, 5.7 mg/dL, 5.8 mg/dL, 5.9 mg/dL, 6.0 mg/dL, 6.1 mg/dL, 6.2 mg/dL, 6.3 mg/dL, 6.4 mg/dL, 6.5 mg/dL, 6.6 mg/dL, 6.7 mg/dL, 6.8 mg/dL, 6.9 mg/dL, or 7.0 mg/dL.

In some embodiments, the subject has, or is at risk of having, hyperuricemia. In some embodiments, the subject has, or is at risk of having, gout, acute gout, acute intermittent gout, gouty arthritis, acute gouty arthritis, acute gouty arthropathy, acute polyarticular gout, recurrent gouty arthritis, chronic gout (with our without tophi), tophaceous gout, chronic tophaceous gout, chronic advanced gout (with our without tophi), chronic polyarticular gout (with our without tophi), chronic gouty arthropathy (with our without tophi), idiopathic gout, idiopathic chronic gout (with or without tophi), primary gout, chronic primary gout (with or without tophi), refractory gout, such as chronic refractory gout, axial gouty arthropathy, a gout attack, a gout flare, podagra (i.e., monarticular arthritis of the great toe), chiragra (i.e., monarticular arthritis of the hand), gonagra (i.e., monarticular arthritis of the knee), gouty bursitis, gouty spondylitis, gouty synovitis, gouty tenosynovitis, gout that affects tendons and ligaments, lead-induced gout (i.e., saturnine gout), drug induced gout, gout due to renal impairment, gout due to kidney disease, chronic gout due to renal impairment (with or without tophi), chronic gout due to kidney disease (with or without tophi), erosive bone disease associated with gout, stroke associated with gout, vascular plaque associated with gout, cirrhosis or steatohepatitis associated with gout, liver-associated gout, incident and recurrent gout, diabetes associated with damage to pancreas in gout, general inflammatory diseases exacerbated by gout, other secondary gout, or unspecified gout.

In some embodiments, the subject has, or is at risk of having, a condition associated with the renal system, for example, calculus of urinary tract due to gout, uric acid urolithiasis, uric acid nephrolithiasis, uric acid kidney stones, gouty nephropathy, acute gouty nephropathy, chronic gouty nephropathy, urate nephropathy, uric acid nephropathy, and gouty interstitial nephropathy.

In some embodiments, the subject has, or is at risk of having, a condition associated with the nervous system, for example, peripheral autonomic neuropathy due to gout, gouty neuropathy, gouty peripheral neuropathy, gouty entrapment neuropathy, or gouty neuritis.

In some embodiments, the subject has, or is at risk of having, a condition associated with the cardiovascular system, for example, metabolic syndrome, hypertension, obesity, diabetes, myocardial infarction, stroke, dyslipidemia, hypertriglyceridemia, insulin resistance/hyperglycemia, coronary artery disease/coronary heart disease, coronary artery disease or blockage associated with gout or hyperuricemia, heart failure, peripheral arterial disease, stroke/cerebrovascular disease, peripheral vascular disease, and cardiomyopathy due to gout.

In some embodiments, the subject has, or is at risk of having, a condition associated with the ocular system including, for example, gouty iritis, inflammatory disease in the eye caused by gout, dry eye syndrome, red eye, uveitis, intraocular hypertension, glaucoma, and cataracts.

In some embodiments, the subject has, or is at risk of having, a condition associated with the skin including, for example, gout of the external ear, gouty dermatitis, gouty eczema, gouty panniculitis, and miliarial gout.

*Compositions and Kits*

Compositions provided herein may comprise inorganic or organic buffers (e.g., sodium or potassium salts of phosphate, carbonate, acetate, or citrate) and pH adjustment agents (e.g., hydrochloric acid, sodium or potassium hydroxide, salts of citrate or acetate, amino acids and their salts) antioxidants (e.g., ascorbic acid, alpha-tocopherol), surfactants (e.g., polysorbate 20, polysorbate 80, polyoxyethylene9-10 nonyl phenol, sodium desoxycholate), solution and/or cryo/lyo stabilizers (e.g., sucrose, lactose, mannitol, trehalose), osmotic adjustment agents (e.g., salts or sugars), antibacterial agents (e.g., benzoic acid, phenol, gentamicin), antifoaming agents (e.g., polydimethylsilozone), preservatives (e.g., thimerosal, 2-phenoxyethanol, EDTA), polymeric stabilizers and viscosity-adjustment agents (e.g., polyvinylpyrrolidone, poloxamer 488, carboxymethylcellulose) and co-solvents (e.g., glycerol, polyethylene glycol, ethanol).

Compositions according to the invention may comprise pharmaceutically acceptable excipients. The compositions may be made using conventional pharmaceutical manufacturing and compounding techniques to arrive at useful dosage forms. Techniques suitable for use in practicing the present invention may be found in Handbook of Industrial Mixing: Science and Practice, Edited by Edward L. Paul, Victor A. Atiemo-Obeng, and Suzanne M. Kresta, 2004 John Wiley & Sons, Inc.; and Pharmaceutics: The Science of Dosage Form Design, 2nd Ed. Edited by M. E. Auten, 2001, Churchill Livingstone. In an embodiment, compositions are suspended in a sterile saline solution for injection together with a preservative.

It is to be understood that the compositions of the invention can be made in any suitable manner, and the invention is in no way limited to compositions that can be produced using the methods described herein. Selection of an appropriate method of manufacture may require attention to the properties of the particular elements being associated.

In some embodiments, compositions are manufactured under sterile conditions or are initially or terminally sterilized. This can ensure that resulting compositions are sterile and non-infectious, thus improving safety when compared to non-sterile compositions. This provides a valuable safety measure, especially when subjects receiving the compositions have immune defects, are suffering from infection, and/or are susceptible to infection. In some embodiments, the compositions may be lyophilized and stored in suspension or as lyophilized powder depending on the formulation strategy for extended periods without losing activity.

Administration according to the present invention may be by a variety of routes, including but not limited to an intravenous route. The compositions referred to herein may be manufactured and prepared for administration using conventional methods.

The compositions of the invention can be administered in effective amounts, such as the effective amounts described elsewhere herein. Doses of compositions as provided herein may contain varying amounts of elements according to the invention. The amount of elements present in the compositions for dosing can be varied according to their nature, the therapeutic benefit to be accomplished, and other such parameters. The compositions for dosing may be administered according to any one of the frequencies provided herein.

Another aspect of the disclosure relates to kits. The kit may comprise any one or more of the compositions provided herein. The kit may comprise any one or more of the compositions comprising uricase as provided herein. Preferably, the uricase-comprising composition(s) is/are in an amount to provide any one or more doses as provided herein. The uricase-comprising composition(s) can be in one container or in more than one container in the kit. The kit may further comprise any one or more of the synthetic nanocarrier compositions provided herein. Preferably, the synthetic nanocarrier composition(s) is/are in an amount to provide one or more of the synthetic nanocarrier doses provided herein. The synthetic nanocarrier composition(s) can be in one container or in more than one container in the kit. The container may be a vial or an ampoule. The composition(s) may be in lyophilized form each in a separate container or in the same container, such that they may be reconstituted at a subsequent time. The lyophilized composition may further comprise a sugar, such as mannitol. The composition(s) may be in the form of a frozen suspension each in a separate container or in the same container, such that they may be reconstituted at a subsequent time. The frozen suspension may further comprise PBS. The kit may further comprise PBS and/or 0.9% sodium chloride, USP. The kit may further comprise instructions for reconstitution, mixing, administration, etc. The instructions may include a description of any one of the methods described herein. Instructions can be in any suitable form, e.g., as a printed insert or a label. The kit may further comprise one or more syringes or other device(s) that can deliver the composition(s) in vivo to a subject.

## EXAMPLES

### Example 1: SEL 212 clinical trial results, non-human

### Preclinical development

SEL 212 was used to treat uricase deficient mice and wild type mice, rats and nonhuman primates to evaluate efficacy, dose regimens and safety.

**Proof of concept study in uricase deficient mice**

A pharmacology study in mice that were genetically deficient in endogenous uricase was conducted. The study evaluated the efficacy of a dose regimen consisting of three immunizations with SEL 212 followed by doses of pegsiticase alone in preventing the formation of ADAs to pegsiticase. The treatment period consisted of the first 14 days of the study. In the study, mice were separated into three treatment groups. During the treatment period:

- the first group, referred to as the Untreated Group, received no treatment;
- the second group, referred to as the Pegsiticase Group, was treated with pegsiticase alone; and
- the third group, referred to as the SVP Rapamycin + Pegsiticase Group, was treated with SVP Rapamycin co administered with pegsiticase.

The Pegsiticase Group and SVP Rapamycin + Pegsiticase Group were treated on days zero, seven and 14 of the treatment period. Each group was then treated with pegsiticase alone on days 35 and 42 of the study, or the challenge period. Uricase specific ADA levels were recorded to determine the formation of ADAs to pegsiticase. Uric acid levels were measured to determine effectiveness of SVP Rapamycin co administered with pegsiticase in lowering uric acid levels below 6 mg/dl, which is the treatment target for gout patients.

Antibody formation. The Pegsiticase Group developed uricase specific ADAs when exposed to pegsiticase during the treatment period. The Untreated Group also developed uricase specific ADAs as soon as they were challenged with pegsiticase. Despite exposure to pegsiticase during both the treatment and challenge periods, the SVP Rapamycin + Pegsiticase Group did not develop uricase specific ADAs during either period.

Uric acid levels. After initial exposure to pegsiticase, the Untreated Group maintained high uric acid levels of approximately 10 mg/dl. The Pegsiticase Group recorded uric acid levels below 6 mg/dl after the first dose in the treatment period. However, during subsequent doses in the treatment period and challenge period, uric acid levels returned to levels well in excess of 6 mg/dl. In contrast, the SVP Rapamycin + Pegsiticase Group maintained uric acid levels that were close to zero throughout the study.

**Proof of concept study in nonhuman primates**

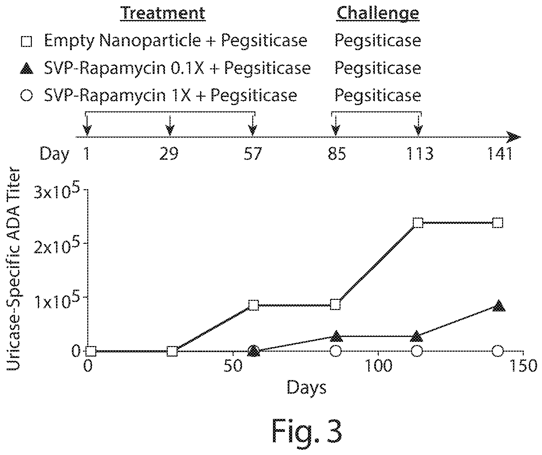

A preclinical study to evaluate the ability of SVP Rapamycin to mitigate the formation of uricase specific ADAs in nonhuman primates was also conducted. As depicted in **Fig. 3,** during the study:

- pegsiticase was administered alone, referred to as the Empty Nanoparticle Group, or
- was co-administered with one of two dose levels of SVP Rapamycin, referred to as the SVP Rapamycin 0.1X and SVP Rapamycin 1X Groups, respectively. The SVP Rapamycin 0.1X Group received a dose level of SVP Rapamycin of 0.3 mg/kg and the SVP Rapamycin 1X Group received a dose level of SVP Rapamycin of 3 mg/kg.

The Empty Nanoparticle Group received three monthly doses of pegsiticase and each of the SVP Rapamycin 0.1X Group and SVP Rapamycin 1X Group received three monthly doses of pegsiticase co-administered with SVP Rapamycin. All groups then received two monthly doses of pegsiticase alone. The SVP Rapamycin 0.1X Group received one tenth of the dose administered in the SVP Rapamycin 1X Group.

Antibody formation. It was observed that the Empty Nanoparticle Group produced high levels of uricase specific ADAs by the end of the study. The SVP Rapamycin 0.1X Group and SVP Rapamycin 1X Group were able to reduce the levels of uricase specific ADAs significantly compared to the Empty Nanoparticle Group and, in the case of the SVP Rapamycin 1X Group, inhibited the formation of antibodies. The observations in this study confirmed in non-human primates the mitigation of uricase specific ADAs that was observed in mice.

Uric acid levels. As expected, the effect that pegsiticase alone or pegsiticase co-administered with SVP Rapamycin had on uric acid levels in nonhuman primates could not be determined due to the activity of naturally occurring uricase in these animals.

Based on these preclinical studies, as well as toxicology studies conducted to conform to regulatory guidelines, referred to as current good laboratory practice, or GLP, it is believed that SEL 212 demonstrated sufficient efficacy and safety in the preclinical animal models to justify movement into clinical development.

**Example 2: SEL 212 clinical trial results, human**

**Phase 1a clinical trial**

The Phase 1a clinical trial for SEL 212 was an ascending dose trial of pegsiticase alone in 22 subjects with elevated serum uric acid levels greater than 6 mg/dl who were separated into five cohorts. Each cohort received a single intravenous infusion of pegsiticase at the following dose levels of 0.1 mg/kg for Cohort #1, 0.2 mg/kg for Cohort #2, 0.4 mg/kg for Cohort #3, 0.8 mg/kg for Cohort #4 and 1.2 mg/kg for Cohort #5. Dosing began with the lowest dose and only after an entire cohort was safely dosed was the next cohort started. The subjects were monitored during a 30 day period post infusion with visits occurring on day 7, 14 21 and the end of trial visit on day 30. Blood and serum of each patient was evaluated for serum uric acid, ADAs (specifically anti-peg, anti-uricase and anti-pegsiticase) and safety parameters. It was observed that pegsiticase demonstrated no serious adverse events and was well tolerated at the five dose levels tested. Additionally, it was observed that pegsiticase rapidly reduced (within hours) and sustained average serum uric acid levels below 6 mg/dl for each cohort for 14 to 30 days, depending on the dose level. Consistent with preclinical studies in animals, pegsiticase induced uricase specific ADAs in all subjects with varying levels in this Phase 1a trial.

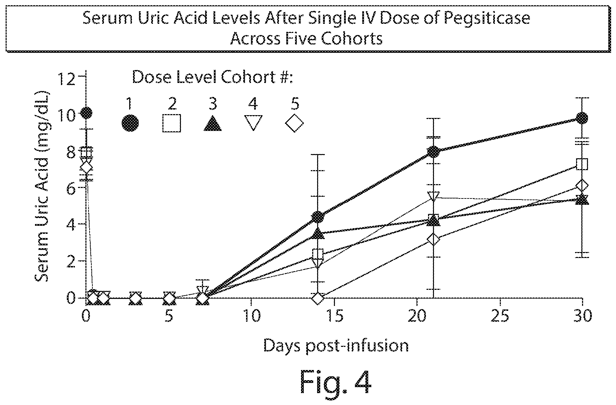

Fig. 4 depicts average serum uric acid levels of the Phase 1a clinical trial's five cohorts tested at different measurement intervals (Day 7, 14, 21 and 30) during the course of the 30 day period following the single intravenous infusion of pegsiticase at the outset of the trial.

The serum uric acid levels were measured at baseline and days seven, 14, 21 and 30 and uricase specific ADA levels at baseline and days seven, 14 and 30 following a single intravenous injection of pegsiticase. Uricase specific ADA levels at day 21 in the Phase 1a clinical trial were not measured. Based on the results from the Phase 1a clinical trial, it was observed that pegsiticase at a tolerated dose is capable of achieving and maintaining a reduction of serum uric acid below the target of 6 mg/dl for a 30 day period in the absence of inhibitory uricase specific ADAs.

**Phase 1b clinical trial**

The Phase 1b clinical trial enrolled 63 patients with serum uric acid levels greater than 6 mg/dl separated into 11 cohorts. A single intravenous infusion of SVP Rapamycin alone at the following ascending dose levels was administered to four cohorts in ascending order. Each cohort consisted of seven patients and were designated as follows: Cohort #1 (0.03 mg/kg), Cohort #3 (0.1 mg/kg), Cohort #5 (0.3 mg/kg) and Cohort #7 (0.5 mg/kg) collectively the SVP Rapamycin Cohorts. After a cohort of the SVP-Rapamycin alone had successfully and safely been dosed the corresponding dose level of SVP Rapamycin was combined with a fixed dose of pegsiticase (0.4mg/kg). The combination was co-administered sequentially as a single intravenous infusion, with the SVP Rapamycin infusion preceding the pegsiticase infusion. The cohort designation is as follows for the six cohorts (5 patients per cohort), which were Cohort #2 (SVP Rapamycin 0.03 mg/kg + 0.4 mg/kg pegsiticase), Cohort #4 (SVP Rapamycin 0.1mg/kg + 0.4 mg/kg pegsiticase), Cohort #6 (SVP Rapamycin 0.3 mg/kg + 0.4 mg/kg pegsiticase), Cohort #10 (0.4 mg/kg pegsiticase + 0.03 mg/kg SVP Rapamycin separated by 48 hours), Cohort #12 (SVP Rapamycin 0.15 mg/kg + 0.4 mg/kg pegsiticase) and Cohort #14 (SVP Rapamycin 0.1 mg/kg + 0.4 mg/kg pegsiticase) collectively the SEL 212 Cohorts. In Cohort #9 a fixed amount of pegsiticase alone at a dose level of 0.4 mg/kg was administered to five patients, which is referred to as the Pegsiticase Cohort. Methods of such treatment are also provided. The subjects were monitored during a 30 day period post infusion with visits occurring on day 7, 14 ,21 and the end of trial visit on day 30. Blood and serum of each patient was evaluated for serum uric acid, ADAs (specifically anti-PEG, anti-uricase and anti-pegsiticase) and safety parameters. The primary objective of the Phase 1b clinical trial was to evaluate the safety and tolerability of SVP Rapamycin alone and in combination with a fixed dose of pegsiticase. A secondary clinical objective was to evaluate the ability of SVP Rapamycin co-administered with pegsiticase to reduce serum uric acid levels and mitigate the formation of uricase specific ADAs when compared to administration of pegsiticase alone.

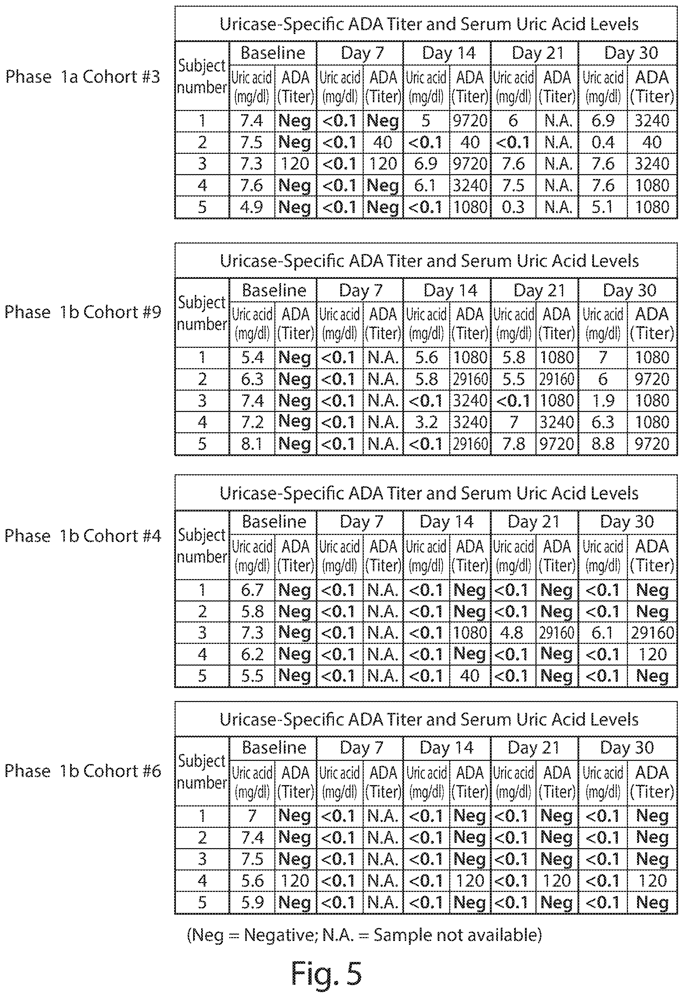

Fig. 5 indicates the serum uric acid levels of Cohort #3 from the Phase 1a clinical trial, in which subjects received a fixed amount of pegsiticase alone (at the same 0.4 mg/kg pegsiticase. Also in the first graph is the data from Cohort# 9 (pegsiticase 0.4 mg/kg) of the Phase 1b clinical trial. This graph represents the reproducibility of the data across two separate studies. In both cohorts there is initial control of the serum uric acid (levels maintained below 6mg/dL) but past day 14, individuals loose the enzyme activity. Also in **Fig. 5,** the data from the SVP rapamycin alone cohorts is displayed. All values remain essentially the same throughout the 30 days of testing indicating that SVP Rapamycin alone has no effect on serum uric acid levels. For Cohort #2 from the Phase 1b clinical trial, which received the lowest dose of SVP Rapamycin co-administered with pegsiticase, it was observed that four out of five subjects tested maintained serum uric acid levels below 6 mg/dl through day 21 of the trial. It was also observed that four out of five subjects in Cohort #4 from the Phase 1b clinical trial, which received the second lowest dose of SVP Rapamycin co-administered with pegsiticase, maintained levels of serum uric acid of less than 0.1 mg/dl through day 30. For Cohort #6 (SEL 212 Cohort), it was observed that four (out of the projected five) subjects maintained levels of serum uric acid of less than 0.1 mg/dl through day 21 and two (out of the projected five) subjects maintained levels of serum uric acid of less than 0.1 mg/dl through day 30. By comparison, for Cohort #9 (Pegsiticase Cohort), four of the five subjects returned to baseline serum uric acid levels by day 30.

Fig. 5 shows the serum uric acid levels and uricase specific ADA levels for each subject in Cohort #3 of the Phase 1a clinical trial and Cohort #9 (Pegsiticase Cohort) of the Phase 1b clinical trial for comparison to the serum uric acid levels and uricase specific ADA levels for each subject in Cohort # 4 (SEL 212 Cohort) in the Phase 1b clinical trial. Cohort #3 from the Phase 1a clinical trial is depicted along with Cohort #9 from the Phase 1b clinical trial for purposes of comparison against Cohort #4 from the Phase 1b clinical trial because the subjects in these cohorts received the same fixed dose of pegsiticase. In addition, Cohort #4 from the Phase 1b clinical trial is depicted in **Fig. 5** because the subjects in Cohort #4 from the Phase 1b clinical trial received a higher dose of SVP Rapamycin than did the subjects in Cohort #2 in the Phase 1b clinical trial, the other SEL 212 Cohort for which 30 day observation period data from the Phase 1b clinical trial was available.

As depicted in **Fig. 5,** in Cohort #3 from the Phase 1a clinical trial and Cohort #9 from the Phase 1b clinical trial, uricase specific ADA formation at day 14 resulting in a return to baseline levels of serum uric acid was observed. In comparison, for Cohort #4 from the Phase 1b clinical trial, it was observed that minimal uricase specific ADA formation in four of the five subjects tested with corresponding maintenance of control of serum uric acid levels through day 30. In the Phase 1a clinical trial, uricase specific ADA levels at day 21 was not measured. However, in the course of conducting the Phase 1a clinical trial, it was learned that it would be useful to measure uricase specific ADA levels at day 21 to more fully understand any variations in such levels between day 14 and day 30. As a result, for the Phase 1b clinical trial, uricase specific ADA levels at day 21 were monitored.

Additional serum uric acid and uricase specific ADA data after day 30 was collected for three of the subjects in Cohort #4 (SEL 212 Cohort) that had no or very low serum uric acid and uricase specific ADA levels at day 30. Data on day 37 was collected for all three of these subjects and again on day 42 or day 44 for two of the three subjects. Each of these three subjects had no or very low uricase specific ADA levels on day 37, day 42 or day 44, as applicable. Serum uric acid levels remained below baseline on day 37 in all three subjects. With respect to the two subjects for which day 42 or day 44 data was available, serum uric acid levels approached or exceeded baseline by the last time point measured. Based on the observations from the Phase 1b clinical trial data that SEL 212 was capable of controlling uric acid levels for at least 30 days in the majority of subjects in Cohort # 4.

On a combined basis, a total of 85 subjects have been dosed with either SEL 212 (SVP Rapamycin and pegsiticase), SVP Rapamycin alone or pegsiticase alone in connection with the Phase 1a and Phase 1b clinical trials. It has been generally observed that SEL 212 and its components, SVP Rapamycin and pegsiticase, have been well tolerated. There have been a total of four serious adverse events, or SAEs, in both Phase 1 clinical trials. All SAEs fully resolved.

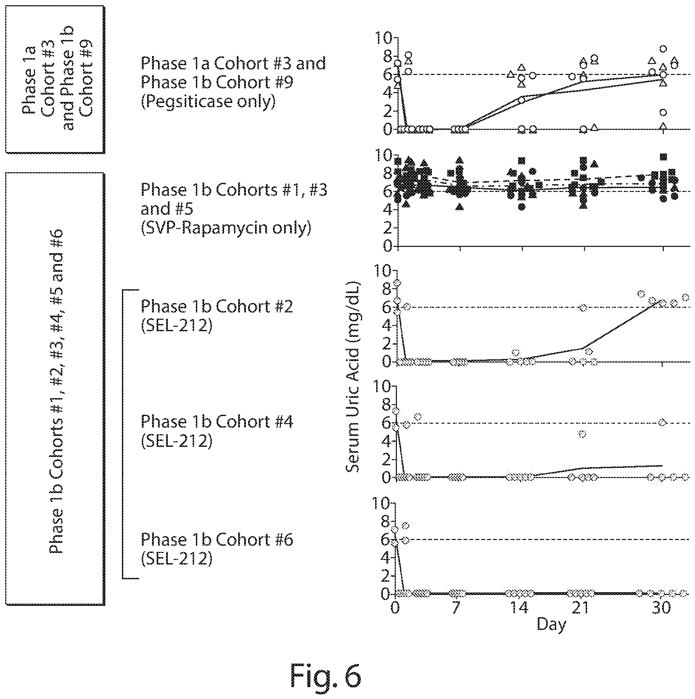

**Fig. 6** shows the serum uric acid levels and uricase-specific ADA levels for each subject in Cohort #3 of the Phase 1a clinical trial and Cohort #9 (Pegsiticase Cohort) of the Phase 1b clinical trial for comparison to the serum uric acid levels and uricase-specific ADA levels for each subject in Cohort # 4 (SEL-212 Cohort) and Cohort #6 (SEL-212 Cohort) in the Phase 1b clinical trial. Cohort #3 from the Phase 1a clinical trial is also depicted along with Cohort #9 from the Phase 1b clinical trial for purposes of comparison against Cohort #4 and Cohort #6 from the Phase 1b clinical trial because the subjects in these cohorts received the same fixed dose of pegsiticase. In addition, Cohort #4 from the Phase 1b clinical trial is depicted because the subjects in Cohort #4 from the Phase 1b clinical trial received a higher dose of SVP-Rapamycin than did the subjects in Cohort #2 in the Phase 1b clinical trial. Also included is Cohort #6 from the Phase 1b clinical trial because these subjects received the highest dose of SVP-Rapamycin tested to date-higher than both Cohorts #2 and #4.

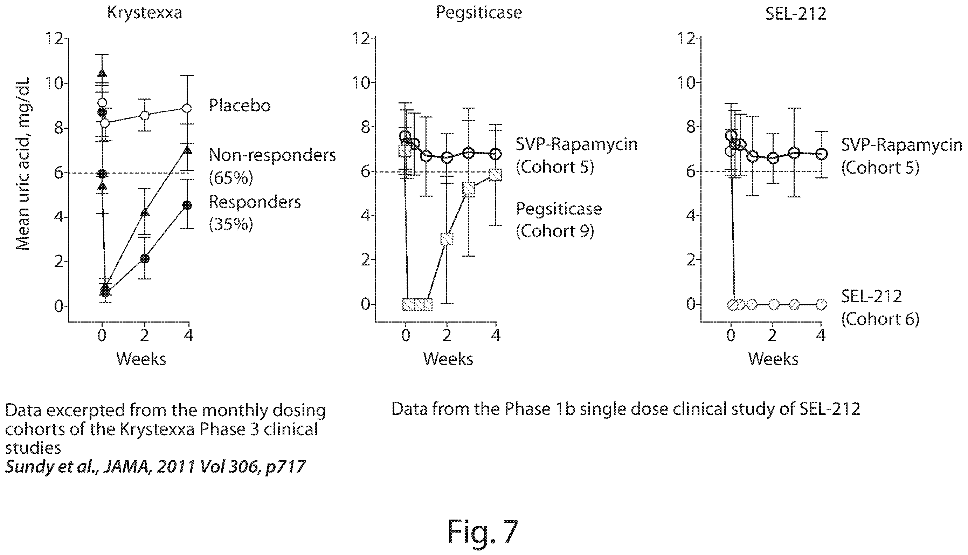

**Fig. 7** presents a non-head-to-head comparison of the efficacy of SEL-212 in Cohort #6 of the Phase 1b clinical trial with Cohort #5 of the Phase 1b clinical trial and data from two replicate, randomized, double-blind, placebo-controlled clinical trials of KRYSTEXXA® as reported in the Journal of the American Medical Association in 2011. These two KRYSTEXXA® clinical trials included 85 patients who received biweekly doses of KRYSTEXXA®, 84 patients who received monthly doses of KRYSTEXXA® and 43 patients who received a placebo.

KRYSTEXXA® has been approved for the treatment of refractory gout on a biweekly dose regimen whereas the monthly dose regimen of KRYSTEXXA® has not been approved for marketing. The graph on the left below depicts the data for the four-week period after the first dose of Krystexxa® from the cohorts of subjects in the KRYSTEXXA® clinical trials who received monthly doses.

The placebo control subjects, indicated in open circles in **Fig. 7,** had uric acid levels above 6 mg/dl for the entire four weeks. The KRYSTEXXA®-treated subjects that went on to become responders, as defined by maintenance of uric acid levels below 6 mg/dl for 80% of the time at months three and six, are indicated in black circles. The KRYSTEXXA®-treated subjects that went on to become non-responders, as defined by the inability to maintain uric acid levels below 6 mg/dl for 80% of the time at months three and six, are indicated in black triangles. Only 35% of KRYSTEXXA®-treated subjects in the monthly dosing cohorts were classified as responders. It is notable that, even at four weeks, the mean uric acid levels were above 6 mg/dl in the non-responders, representing 65% of subjects, and were above 4 mg/dl in the responders. 89% of all KRYSTEXXA®-treated subjects developed ADAs. In comparison, the graph on the right in **Fig. 7** depicts data from Cohort #5 of the Phase 1b clinical trial, which received a single dose of SVP-Rapamycin alone, and Cohort #6 of the Phase 1b clinical trial, which received a single dose of SEL-212. All five subjects in Cohort #6 of the Phase 1b clinical trial, treated with SEL-212, maintained levels of serum uric acid of less than 0.1 mg/dl through day 30. Subjects in Cohort #5 of the Phase 1b clinical trial, treated with SVP-Rapamycin alone, experienced no significant reduction in uric acid levels, as such levels remained relatively constant over the 30-day period. Also shown is a comparison of data from Cohort #5 of the Phase 1b clinical trial, which received a single dose of SVP-Rapamycin alone, with Cohort #9 of the Phase 1b clinical trial, which received pegstiticase alone.

While it is believed that the above comparison is useful in evaluating the results of Cohort #6 of the Phase 1b clinical trial, the Phase 1b clinical trial and the KRYSTEXXA® clinical trials were separate trials conducted by different investigators at different sites. In addition, there were substantial differences, including, for example, that the KRYSTEXXA® clinical trials were double-blind trials involving a substantial number of patients with refractory gout while the Phase 1b clinical trial evaluated SEL-212 in an unblended manner in a small number of subjects with elevated uric acid levels. Moreover, only the efficacy of SEL-212 with the four-week period following the first injection of KRYSTEXXA® could be compared as SEL-212 had not yet been evaluated in a multi-dose clinical trial.

Additional serum uric acid and uricase-specific ADA data was collected after day 30 for three of the subjects in Cohort #4 (SEL-212 Cohort) that had no or very low serum uric acid and uricase-specific ADA levels at day 30. Data was collected on day 37 for all three of these subjects and again on day 42 or day 44 for two of the three subjects. Each of these three subjects had no or very low uricase-specific ADA levels on day 37, day 42 or day 44, as applicable. Serum uric acid levels remained below baseline on day 37 in all three subjects. With respect to the two subjects for which day 42 or day 44 data was available, serum uric acid levels approached or exceeded baseline by the last time point measured.

## Example 2 - Phase 2 Clinical Trial

Presented herein is a phase 2 clinical trial of SEL-212. The study consists of multiple doses of SEL-212 concomitantly administered with doses of SEL-037. SEL-212 is a combination of SEL-037 and SEL-110. SEL-037 comprises pegsiticase (Recombinant Pegylated Candida Urate Oxidase). SEL-110 is a nanoparticle comprising PLA (poly(D,L-lactide)) and PLA-PEG (poly(D,L- lactide)-block-poly (ethylene-glycol)) encapsulating rapamycin.

SEL-037 can be provided with phosphate buffer and mannitol as excipients. Prior to administration, 6 mg, measured as uricase protein, lyophilized SEL-037 can be reconstituted with 1.1 ml of sterile water for injection, USP (United States Pharmacopeia) which forms a 6 mg/mL concentrated solution. A sufficient volume of reconstituted SEL-037 at 0.2 mg/kg or 0.4 mg/kg, measured as uricase protein, is diluted in 100 mL of 0.9% sodium chloride for injection, USP and dosed as a single intravenous infusion with an infusion pump over 60 minutes.

SEL-110 is provided as a 2 mg/mL, based on rapamycin content, suspension in PBS. The appropriate amount of SEL-110 on a mg/kg basis is drawn into a syringe or syringes and administered as an IV infusion with a syringe infusion pump. If a subject is part of Cohorts 3, 4, 5, 6, 7 and 8 then SEL-110 is administered prior to SEL-037. SEL-110 is delivered by syringe infusion pump at a single steady rate sufficient to deliver the dose volume over a period of 55 minutes concurrently with a 60 minute infusion of 125 mL of normal saline and then the SEL-037 infusion (0.2 mg/kg for Cohorts 3, 5 and 7; 0.4 mg/kg for Cohorts 4, 6 and 8) are started at the 60 minute mark.

48 subjects were divided into 8 dosing cohorts. Each cohort consists of 6 patients. Cohort 3 receives SEL-212 (with 0.05 mg/kg of SEL-110 + 0.2 mg/kg pegsiticase) , Cohort 4 receives SEL-212 (with 0.05 mg/kg of SEL-110 + 0.4 mg/kg pegsiticase), Cohort 5 receives SEL-212 (with 0.08 mg/kg of SEL-110 + 0.2 mg/kg pegsiticase), Cohort 6 receives SEL-212 (with 0.08 mg/kg of SEL-110 + 0.4 mg/kg pegsiticase), Cohort 7 receives SEL-212 (with 0.1 mg/kg of SEL-110 + 0.2 mg/kg pegsiticase) and Cohort 8 receives SEL-212 (with 0.1 mg/kg of SEL-110 + 0.4 mg/kg pegsiticase).

## Distribution of Subjects

All enrolled subjects were randomized initially to 4 cohorts such that upon reaching 12 subjects total for all 4 cohorts, each cohort contains 3 subjects. After the completion of at least one treatment cycle the subject experience is evaluated before enrollment is opened to all cohorts. The future enrollment is randomized between all open cohorts.

## Premedication for Study Drug Treatments

All subjects receive 180 mg fexofenadine orally the night before receiving study drug (12 h $\pm$ 2h) and again 2 $\pm$ 1 hours before receiving study drug (i.e., SEL-110 for Cohorts 3, 4, 5, 6. 7 and 8). In addition, they also receive methylprednisolone 40 mg (or equivalent drug, for example prednisone 50 mg IV or dexamethasone 8 mg IV) intravenously 1 $\pm$ 0.5 hour before receiving study drug (i.e. prior SEL-110 for Cohorts 3, 4, 5, 6, 7 and 8). This occurs for every treatment dosing of study drug (Part A, Treatment Periods 1-3 and for Part B, Treatment Periods 4 and 5). Cohorts 3-6 have received first and second doses.

**Premedication for Gout Flare**

All subjects that meet all inclusion and exclusion criteria are given premedication for gout flare prevention. The regimen begins 1 week prior to the first dosing of study drug and continue for as long as the subject is enrolled in the clinical study. Subjects are given colchicine 1.2 mg as a single loading dose. Then they will continue with colchicine 0.6 mg QD for the remainder of their participation in the trial. If there is a contraindication to colchicine, the subject receives ibuprofen 600 mg TID or equivalent dose of a NSAID. If there is a contraindication to colchicine and to NSAIDs the subject receives no premedication for gout flare. The gout flare prevention medication continues as long as the subject is enrolled in the clinical study. Subjects who began receiving a NSAID as gout flare prevention medication due to a contraindication to colchicine continue to receive the NSAID as long as the subject is enrolled in the study.

**Duration of treatment for Cohort 3, Cohort 4, Cohort 5, Cohort 6, Cohort 7, and Cohort 8**

**Treatment period 1 - Part A**

Subjects were screened within 45 days of dosing. Once they met inclusion/exclusion criteria and all assessments were considered acceptable they were instructed on when to start their premedication (date and medication, Day -7) for the prevention of gout flares. The day of initial dosing of study drug was designated Day 0. Eligible subjects who have been assigned to Cohorts 3, 4. 5, 6, 7 and 8 received a single IV in fusion of SEL-110 (dose based on a mg/kg basis). SEL-110 was delivered by syringe infusion pump at a single steady rate sufficient to deliver the dose volume over a period of 55 minutes. Concurrently to the administration of SEL-110, the subject received a 125 mL of normal saline over 60 minutes. This was followed ($\pm$ 3 minutes) by an infusion delivered by infusion pump of SEL-037 (0.2 mg/kg for Cohorts 3, 5, and 7; 0.4 mg/kg for Cohorts 4, 6 and 8) diluted into 100 mL of normal saline delivered over 60 minutes. Subjects remained in the clinic for 9 hours after the start of the infusion of SEL-110 for safety evaluations and PK blood draws. Subjects returned for PK and PD blood draws on Treatment Period 1, Days 1,7, 14, 21 and safety and Antibody blood draws on Treatment Period 1, Days 7, 14, 21.

**Treatment period 2 - Part A**

On the morning of Treatment Period 2, Day 0, subjects reported to the clinic for the dosing of study drug. Eligible subjects who had been assigned to Cohorts 3, 4, 5, 6, 7 and 8 received a single IV infusion of SEL-110 (dose based on a mg/kg basis). SEL-110 was delivered by syringe infusion pump at a single steady rate sufficient to deliver the dose volume over a period of 55 minutes. Concurrently to the administration of SEL-110, the subject received a 125 mL of normal saline over 60 minutes. This was followed ($\pm$ 3 minutes) by an infusion delivered by infusion pump of SEL-037 (0.2 mg/kg for Cohorts 3, 5 and 7; 0.4 mg/kg for Cohorts 4, 6 and 8) diluted into 100 mL of normal saline delivered over 60 minutes. Subjects remained in the clinic for 9 hours after the start of the infusion of SEL-110 for safety evaluations and PK blood draws. Subjects returned for PK and PD on Treatment Period 2, Days 1, 7, 14 and 21 and safety and Antibody blood draws on Treatment Period 2, Days 7, 14 and 21.

**Treatment period 3 - Part A**

On the morning of Treatment Period 3, Day 0 subjects will report to the clinic for the dosing of study drug. Eligible subjects who have been assigned to Cohorts 3, 4, 5, 6, 7 and 8 will receive a single IV infusion of SEL-110 (dose based on a mg/kg basis). SEL-110 will be delivered by syringe infusion pump at a single steady rate sufficient to deliver the dose volume over a period of 55 minutes. Concurrently to the administration of SEL-110, the subject will receive a 125 mL of normal saline over 60 minutes. This will be followed ($\pm$ 3 minutes) by an infusion delivered by infusion pump of SEL-037 (0.2 mg/kg for Cohorts 3, 5 and 7; 0.4 mg/kg for Cohorts 4, 6 and 8) diluted into 100 mL of normal saline delivered over 60 minutes. Subjects will remain in the clinic for 9 hours after the start of the infusion of SEL-110 for safety evaluations and PK blood draws. Subjects will return for PK and PD blood draws on Treatment Period 3, Days 1 , 7, 14 and 21 and safety and Antibody blood draws on Treatment Period 3, Days 7, 14 and 21.

**Treatment period 4 - Part B**

On the morning of Treatment Period 4, Day 0 subjects will report to the clinic for the dosing of study drug. Subjects will receive a single IV infusion of SEL-037 (0.2 mg/kg for Cohorts 3, 5 and 7; 0.4 mg/kg for Cohorts 4, 6 and 8) diluted into 100 mL of normal saline over 60 minutes by infusion pump. Subjects will remain in the clinic for 9 hours after the start of the infusion of SEL-037 for safety evaluations and PK blood draws. Subjects will return for PK and PD blood draws on Treatment Period 4, Days 1 , 7, 14 and 21 and safety and Antibody blood draws on Treatment Period 4, Days 7, 14 and 21.

**Treatment period 5 - Part B**

On the morning of Treatment Period 5, Day 0 subjects will report to the clinic for the dosing of study drug. Subjects will receive a single IV infusion of SEL-037 (0.2 mg/kg for Cohorts 3, 5 and 7; 0.4 mg/kg for Cohorts 4, 6 and 8) diluted into 100 ml of normal saline over 60 minutes by infusion pump. Subjects will remain in the clinic for 9 hours after the start of the infusion of SEL-037 for safety evaluations and PK blood draws. Subjects will return for PK and PD blood draws on Treatment Period 5, Days 1, 7, 14 and 21 and safety and Antibody blood draws on Treatment Period 5, Days 7, 14 and 21.

**Results**

When pegsiticase was administered alone in the Phase 1 described in **Example 2,** 57% (4 out of 7 patients) of those with a history of gout had signs of gout flare in the first month after receiving the study drug **(Table 1).** In contrast, however, when PLA/PLA-PEG synthetic nanocarriers comprising rapamycin were concomitantly administered with pegsiticase in a Phase 2 trial described in **Example 3,** only one gout flare was reported in the subjects who had a history of gout (16 out of 63 enrolled patients) **(Table 2).** This subject was in the cohort that received only the rapamycin-comprising nanocarrier (without uricase). Because this subject did not receive the uricase therapy, this subject's serum uric acid level did not drop significantly. The flare was, therefore, unrelated to a change in serum uric acid. One additional subject, who did not have a prior diagnosis of gout, reported a post-treatment flare. This patient's serum uric acid level dropped from 8.8 mg/dL to 0.1 mg/dL within 90 minutes following drug administration. So, although this subject had only been diagnosed with asymptomatic hyperuricemia before the study, a flare did seem to coincide with a drop in serum uric acid.

**Table 1. Flares in subjects with history of gout**

| Subject | Flare in 1st month | Dose of SEL-037 |
|---|---|---|
| 1 | no | 0.1 mg/kg |
| 2 | yes | 0.1 mg/kg |
| 3 | yes | 0.1 mg/kg |
| 4 | yes (positive for tenderness and swelling of right 5th PIP) | 0.1 mg/kg |
| 5 | no | 0.2 mg/kg |
| 6 | yes (mild gout, right 3rd toe) | 0.2 mg/kg |
| 7 | no | 0.8 mg/kg |

**Table 2. Flares in SEL-212 subjects**

| SEL-212 subjects with gout | Flare in 1st month | Cohort/dose |
|---|---|---|
| 1 | no | Cohort 1/SEL-110 0.03 mg/kg |
| 2 | no | Cohort 3/SEL-110 0.1 mg/kg |
| 3 | no | Cohort 4/SEL-212 0.1 mg/kg |
| 4 | no | Cohort 5/SEL-110 0.3 mg/kg |
| 5 | no | Cohort 4/SEL-212 0.1 mg/kg |
| 6 | no | Cohort 3/SEL-110 0.1 mg/kg |
| 7 | no | Cohort 14/SEL-212 0.1 mg/kg |
| 8 | no | Cohort 6/SEL-212 0.3 mg/kg |
| 9 | no | Cohort 5/ SEL-110 0.3 mg/kg |
| 10 | yes | Cohort 7/ SEL-110 0.5 mg/kg |

(continued)

| SEL-212 subjects with gout | Flare in 1st month | Cohort/dose |
|---|---|---|
| 11 | no | Cohort 14/ SEL-212 0.1 mg/kg |
| 12 | no | Cohort 14/ SEL-212 0.1 mg/kg |
| 13 | no | Cohort 14/ SEL-212 0.1 mg/kg |
| 14 | no | Cohort 12/ SEL-212 0.15 mg/kg |
| 15 | no | Cohort 12/ SEL-212 0.15 mg/kg |
| 16 | no | Cohort 12/ SEL-212 0.15 mg/kg |
| 17 | yes | Cohort 2/ SEL-212 0.03 mg/kg |

A phase 2 study has been undertaken **(Example 3).** This study involved the administration of multiple IV infusions of PLA/PLA-PEG synthetic nanocarriers comprising rapamycin together with pegsiticase in order to assess its safety and tolerability. Thirty-eight subjects were randomized and dosed, with 8 subjects reported as suffering from a gout flare **(Table 3).**

**Table 3. Subjects who suffered from gout flare following treatment**

| SEL-212 subject | Cohort | Dose | Gout flare prophylaxis with colchicine/NSAID |
|---|---|---|---|
| 1 | 1 | SEL-037 0.2 mg/kg | no |
| 2 | 1 | SEL-037 0.2 mg/kg | no |
| 3 | 1 | SEL-037 0.2 mg/kg | no |
| 4 | 3 | SEL-110 0.05 mg/kg + SEL-037 0.2 mg/kg | yes |
| 5 | 3 | SEL-110 0.05 mg/kg + SEL-037 0.2 mg/kg | no |
| 6 | 4 | SEL-110 0.05 mg/kg + SEL-037 0.4 mg/kg | no |
| 7 | 4 | SEL-110 0.05 mg/kg + SEL-037 0.4 mg/kg | no |
| 8 | 5 | SEL-110 0.08 mg/kg + SEL-037 0.2 mg/kg | yes |

Flare rates in the above subjects were compared to the flare rates in the pegloticase trials. Those subjects who received gout flare prophylaxis (with colchicine or NSAIDS) only were chosen to match the pegloticase subject conditions. Flare frequency (number of flares per patient month) was selected as a measure by which to compare flare rates. This measure was chosen based on the fact that the trial data covers 2 months, or 2 treatment cycles; while the pegloticase trials varied in length from 35 days (Sundy et al., Pharmacokinetics and pharmacodynamics of intravenous PEGylated recombinant mammalian urate oxidase in patients with refractory gout. Arthritis and Rheumatism. Vol. 56, No. 3, March 2007, pp 1021-1028) to 6 months (John S. Sundy, MD, PhD; Herbert S. B. Baraf, MD; Robert A. Yood, MD; et al. Efficacy and Tolerability of Pegloticase for the Treatment of Chronic Gout in Patients Refractory to Conventional TreatmentTwo Randomized Controlled Trials. JAMA. 2011;306(7):711-720). Patient monthly rates were chosen to be able to compare between trials.
Cohorts 3 and 4 were grouped together for this analysis, as they were given the same dose of synthetic nanocarriers comprising rapamycin (0.05 mg/kg), and likewise cohorts 5 and 6 have been grouped together (with a synthetic nanocarrier comprising rapamycin dose of 0.08 mg/kg). In cohorts 3 and 4, 19 subjects have been dosed for a total of 24 treatment cycles. Not all subjects received all treatments, as certain subjects were discontinued following protocol changes. In cohorts 5 and 6, thus far, 13 subjects have been dosed with a total of 24 treatment cycles. This means that, for subjects who received gout flare prophylaxis, 2 flares in total have occurred over 48 treatment cycles. This can be equated to 0.04 flares per treatment cycle; in other words, a flare frequency of 0.04 flares per patient month.
In contrast, the Phase 3 pegloticase trials (John S. Sundy, MD, PhD; Herbert S. B. Baraf, MD; Robert A. Yood, MD; et al. Efficacy and Tolerability of Pegloticase for the Treatment of Chronic Gout in Patients Refractory to Conventional TreatmentTwo Randomized Controlled Trials. JAMA. 2011;306(7):711-720) reported the following: 2.3 flares per patient over the first 3 months for 85 patients who received biweekly pegloticase, and 2.7 flares per patient over the first 3 months for 84 patients who received monthly pegloticase. These numbers equate to a flare frequency of 0.77 and 0.9 flares per patient month, respectively.

Further comparisons can be made with the two primary branded oral uric acid lowering medication, febuxostat and lesinurad. In a phase 3, randomized, double-blind, multi-center trial, the safety and efficacy of febuxostat was studied over 52 weeks (Michael A. Becker, M.D., H. Ralph Schumacher, Jr., M.D., Robert L. Wortmann, M.D., Patricia A. MacDonald, B.S.N., N.P., Denise Eustace, B.A., William A. Palo, M.S., Janet Streit, M.S., and Nancy Joseph-Ridge, M.D. Febuxostat Compared with Allopurinol in Patients with Hyperuricemia and Gout. N Engl J Med 2005; 353:2450-2461December 8, 2005). The comparison period for this analysis included only the first 8 weeks of that study, when gout flare prophylaxis was administered. At a dose of 80 mg/day, 55 out of 255 subjects required treatment for at least one gout flare. This would be the equivalent to a flare frequency of at least 0.22 flares per patient month, and possibly more. At a dose of 120 mg/day, 90 out of 250 subjects required treatment for at least one gout flare, equating to at least a flare frequency of 0.36 flares per patient month, and possibly more.

In a phase 2, randomized, double-blind study to assess the efficacy and tolerability of lesinurad, subjects were given colchicine for gout flare prophylaxis and treated with lesinurad at different doses over 1 month (Perez -Ruiz F, Sundy JS, Miner JN for the RDEA594-203 Study Group, et al. Lesinurad in combination with allopurinol: results of a phase 2, randomised, double-blind study in patients with gout with an inadequate response to allopurinol, Annals of the Rheumatic Diseases 2016;75:1074-1080). During this treatment period, gout flares requiring treatment were reported in 10 out of 46 patients in a month in those dosed at 200 mg daily, 13 out of 42 patients in a month in those dosed at 400 mg daily, and 15 out of 48 patients in a month in those dosed at 600 mg daily. This equates to a flare frequency of 0.22, 0.31, and 0.31 flares per patient month, respectively.

The tabulated data outlining the comparison in flare frequency between the different medications alongside their efficacy in reducing serum uric acid (sUA) is compiled in **Table 4**.

**Table 4. Flares per patient month compared with other uric acid lowering treatments**

| Medication and dosage | Flares per patient month | Time of efficacy (area under curve of mean sUA levels over time)* |
|---|---|---|
| SEL-212 monthly | 0.04 | 25.66 |
| Pegloticase biweekly | 0.77 | Responders- 26.6<br>Nonresponders- 84.35 |
| Pegloticase monthly | 0.90 | Responders-53.55<br>Nonresponders-99.75 |
| Febuxostat 80 mg/day | 0.22 | |
| Febuxostat 120 mg/day | 0.36 | |
| Lesinurad 200 mg/day | 0.22 | 158.5 |
| Lesinurad 400 mg/day | 0.31 | 165.8 |
| Lesinurad 600 mg/day | 0.31 | 167.5 |
| * Indicator of efficacy. | | |

The flare frequency is clearly reduced for the subjects who received the rapamycin-containing nanocarrier concomitantly administered with pegsiticase as compared to all of the other medications. This unexpected outcome is significantly better than with other therapies. This also has the benefit for patient adherence to uric acid lowering therapies, such as uricase, as adherence is greatly reduced when rebound flares occur following initiation of therapy (Treatment of chronic gouty arthritis: it is not just about urate-lowering therapy. Schlesinger N - Semin. Arthritis Rheum. - October 1, 2012; 42 (2); 155-65).

**Claims**

1. A composition comprising synthetic nanocarriers comprising poly(D,L lactide) (PLA) and poly(D,L lactide) poly(ethylene glycol) (PLA-PEG) and comprising rapamycin, and a composition comprising pegsiticase for use in a method of treating a subject with gout or a condition associated with gout, hyperuricemia, or an elevated serum uric acid level and/or undesired uric acid deposits, wherein the method comprises administering the compositions concomitantly to a subject and wherein the composition comprising pegsiticase is administered at a dose of 0.1 mg/kg, 0.2 mg/kg, 0.4 mg/kg, 0.8 mg/kg, or 1.2 mg/kg pegsiticase with each administration, and wherein the composition comprising synthetic nanocarriers and the composition comprising pegsiticase are administered monthly for at least three months.

2. A composition comprising synthetic nanocarriers comprising poly(D,L lactide) (PLA) and poly(D,L lactide) poly(ethylene glycol) (PLA-PEG) and comprising rapamycin, and a composition comprising pegsiticase for use in a method of preventing gout flare, wherein the method comprises administering the compositions concomitantly to a subject, wherein the composition comprising pegsiticase is administered at a dose of 0.1 mg/kg, 0.2 mg/kg, 0.4 mg/kg, 0.8 mg/kg, or 1.2 mg/kg pegsiticase with each administration, wherein the subject is not administered an additional therapeutic to prevent gout flare concomitantly with the concomitant administration, and wherein the composition comprising synthetic nanocarriers and the composition comprising pegsiticase are administered monthly for at least three months.

3. The compositions for the use of claim 1 or claim 2, wherein the subject is identified as having had or as being expected to have gout flare from treatment with a gout therapy without concomitant administration of an additional therapeutic to prevent gout flare, and/or wherein the subject is not administered an additional therapeutic to prevent gout flare concomitantly with each concomitant administration.

4. The compositions for the use of any one of the preceding claims, wherein the concomitant administration occurs at least four times, at least five times, at least six times, at least seven times, at least eight times, at least nine times, or at least ten times in the subject.

5. The compositions for the use of any one of the preceding claims, wherein the composition comprising synthetic nanocarriers comprising rapamycin and the composition comprising pegsiticase are administered monthly for at least four months, five months, six months, seven months, eight months, nine months, ten months or more.

6. The compositions for the use of any one of the preceding claims, wherein:

i) the composition comprising synthetic nanocarriers comprising rapamycin is administered at a dose of 0.05 - 0.5 mg/kg rapamycin with each administration;
ii) the composition comprising synthetic nanocarriers comprising rapamycin is administered at a dose of 0.05 mg/kg, 0.08 mg/kg, 0.1 mg/kg, 0.125 mg/kg, 0.15 mg/kg, 0.2 mg/kg, 0.25 mg/kg, 0.3 mg/kg, 0.35 mg/kg, 0.4 mg/kg, 0.45 mg/kg, or 0.5 mg/kg rapamycin with each administration;
iii) the composition comprising synthetic nanocarriers comprising rapamycin is administered at a dose of 0.55 - 6.5 mg/kg with each administration, wherein the dose is given as the mg of the synthetic nanocarriers comprising rapamycin; or
iv) the composition comprising synthetic nanocarriers comprising rapamycin is administered at a dose of 0.55 mg/kg, 0.65 mg/kg, 0.7 mg/kg, 0.8 mg/kg, 0.9 mg/kg, 1.0 mg/kg, 1.1 mg/kg, 1.25 mg/kg, 1.5 mg/kg, 2.0 mg/kg, 2.5 mg/kg, 3.0 mg/kg, 3.5 mg/kg, 4.0 mg/kg, 4.5 mg/kg, 5.0 mg/kg, 5.5 mg/kg, 6.0 mg/kg, or 6.5 mg/kg with each administration, wherein the dose is given as the mg of the synthetic nanocarriers comprising rapamycin.

7. The compositions for the use of claim 6, wherein the composition comprising synthetic nanocarriers comprising rapamycin is administered at a dose of 0.1 mg/kg or 0.15 mg/kg rapamycin with each administration.

8. The compositions for the use of any one of the preceding claims, wherein the composition comprising pegsiticase is administered at a dose of 0.2 mg/kg pegsiticase with each administration.

9. The compositions for the use of any one of the preceding claims, wherein:

i) a composition comprising pegsiticase is administered to the subject at least once after the one or more concomitant administrations without an immunosuppressant and/or a composition comprising pegsiticase is administered to the subject at least once after the one or more concomitant administrations without synthetic nanocarriers comprising an immunosuppressant;
ii) a composition comprising pegsiticase is administered to the subject at least once after the one or more concomitant administrations without an immunosuppressant and/or a composition comprising pegsiticase is administered to the subject at least once after the one or more concomitant administrations without synthetic nanocarriers comprising an immunosuppressant, wherein the composition comprising pegsiticase is administered at least twice, at least thrice, four, five or more times after the one or more concomitant administrations; or

iii) a composition comprising pegsiticase is administered to the subject at least once after the one or more concomitant administrations without an immunosuppressant and/or a composition comprising pegsiticase is administered to the subject at least once after the one or more concomitant administrations without synthetic nanocarriers comprising an immunosuppressant, wherein the composition comprising pegsiticase is administered monthly for two months after the one or more concomitant administration.

10. The compositions for the use of any one of the preceding claims, wherein the composition comprising synthetic nanocarriers comprising rapamycin is administered prior to the composition comprising pegsiticase with each concomitant administration and/or the composition comprising synthetic nanocarriers comprising rapamycin and the composition comprising pegsiticase are administered within an hour of each other with each concomitant administration.

11. The compositions for the use of any one of the preceding claims, wherein the subject has acute gout; chronic gout with or without tophi; idiopathic gout; refractory gout; secondary gout; unspecified gout; gout associated with a cardiovascular condition, renal condition, pulmonary condition, neurological condition, ocular condition, dermatological condition or hepatic condition; or has had a gout attack or gout flare.

12. The compositions for the use of claim 11, wherein the subject has chronic refractory gout.

13. The compositions for the use of any one of the preceding claims, wherein the rapamycin is encapsulated in the synthetic nanocarriers.

14. The compositions for the use of any one of the preceding claims, wherein:

i) the mean of a particle size distribution obtained using dynamic light scattering of the synthetic nanocarriers is a diameter greater than 120nm, preferably greater than 150nm, preferably greater than 200nm, preferably greater than 250nm; or
ii) the mean of a particle size distribution obtained using dynamic light scattering of the synthetic nanocarriers is a diameter greater than 120nm, preferably greater than 150nm, and wherein the diameter is less than 300nm, preferably less than 250nm, preferably less than 200nm.

15. The compositions for the use of any one of the preceding claims, wherein the load of the rapamycin of the synthetic nanocarriers is 7-12% or 8-12% by weight, preferably wherein the load of the rapamycin of the synthetic nanocarriers is 7-10% or 8-10% by weight or the load of the rapamycin of the synthetic nanocarriers is 7%, 8%, 9%, 10%, 11%, or 12% by weight.

16. The compositions for the use of any one of the preceding claims, wherein each administration of each composition is an intravenous administration, preferably wherein the intravenous administration is an intravenous infusion.

17. The compositions for the use of any one of the preceding claims, wherein:

i) the method further comprises administering an additional therapeutic to the subject;
ii) the method further comprises administering an additional therapeutic to the subject, wherein the additional therapeutic is an oral gout therapeutic;
iii) the method further comprises administering an additional therapeutic to the subject, wherein the additional therapeutic is administered at least once after the one or more concomitant administrations without an immunosuppressant or wherein the additional therapeutic is administered at least once, preferably at least twice, at least thrice, four times, five times or more, after the one or more concomitant administrations without synthetic nanocarriers comprising an immunosuppressant;
iv) the method further comprises administering an additional therapeutic to the subject, wherein the additional therapeutic is an oral gout therapeutic, wherein the additional therapeutic is administered at least once after the one or more concomitant administrations without an immunosuppressant or wherein the additional therapeutic is administered at least once, preferably at least twice, at least thrice, four times, five times or more, after the one or more concomitant administrations without synthetic nanocarriers comprising an immunosuppressant;

v) the method further comprises administering an additional therapeutic to the subject, wherein the additional therapeutic is an anti-gout flare treatment; or

vi) the method further comprises administering an additional therapeutic to the subject, wherein the additional therapeutic is an anti-gout flare treatment, and wherein the anti-gout flare treatment is a prophylactic treatment administered concomitantly but prior to the administration of each pegsiticase composition and/or wherein the anti-gout flare treatment is colchicine or an NSAID.

18. The compositions for the use of any one of the preceding claims, wherein the subject has an elevated serum uric acid level that is ≥ 6 mg/dL.

19. The compositions for the use of any one of the preceding claims, wherein the composition comprising synthetic nanocarriers comprises polymeric synthetic nanocarriers comprising PLA, PLA-PEG, and encapsulated rapamycin; and wherein the composition comprising polymeric synthetic nanocarriers comprising PLA, PLA-PEG, and encapsulated rapamycin is administered at a dose of 0.1 mg/kg or 0.15 mg/kg rapamycin with each administration, and the dose of the composition comprising pegsiticase, is 0.2 mg/kg with each administration.

**Patentansprüche**

1. Zusammensetzung, umfassend synthetische Nanocarrier, umfassend Poly(D,L-Lactid) (PLA) und Poly(D,L-Lactid)-Poly(ethylenglycol) (PLA-PEG) und umfassend Rapamycin, und eine Zusammensetzung, umfassend Pegsiticase zur Verwendung in einem Verfahren zum Behandeln eines Subjekts mit Gicht oder einer mit Gicht assoziierten Erkrankung, Hyperurikämie oder einem erhöhten Serumharnsäurespiegel und/oder unerwünschten Harnsäureablagerungen, wobei das Verfahren ein begleitendes Verabreichen der Zusammensetzungen an ein Subjekt umfasst, und wobei die Zusammensetzung, umfassend Pegsiticase, in einer Dosis von 0,1 mg/kg, 0,2 mg/kg, 0,4 mg/kg, 0,8 mg/kg oder 1,2 mg/kg Pegsiticase bei jeder Verabreichung verabreicht wird, und wobei die Zusammensetzung, umfassend synthetische Nanocarrier, und die Zusammensetzung, umfassend Pegsiticase, für mindestens drei Monate monatlich verabreicht werden.

2. Zusammensetzung, umfassend synthetische Nanocarrier, umfassend Poly(D,L-Lactid) (PLA) und Poly(D,L-Lactid)-Poly(ethylenglycol) (PLA-PEG) und umfassend Rapamycin, und eine Zusammensetzung, umfassend Pegsiticase zur Verwendung in einem Verfahren zum Vorbeugen eines Gichtanfalls, wobei das Verfahren das begleitende Verabreichen der Zusammensetzungen an ein Subjekt umfasst, wobei die Zusammensetzung, umfassend Pegsiticase, in einer Dosis von 0,1 mg/kg, 0,2 mg/kg, 0,4 mg/kg, 0,8 mg/kg oder 1,2 mg/kg Pegsiticase bei jeder Verabreichung verabreicht wird, und bei jeder Verabreichung verabreicht wird, wobei dem Subjekt kein zusätzliches Therapeutikum, um dem Gichtanfall vorzubeugen, mit der begleitenden Verabreichung begleitend verabreicht wird, und wobei die Zusammensetzung, umfassend synthetische Nanocarrier und die Zusammensetzung, umfassend Pegsiticase, für mindestens drei Monate monatlich verabreicht werden.

3. Zusammensetzungen für die Verwendung nach Anspruch 1 oder 2, wobei das Subjekt als ein Subjekt identifiziert wird, dass einen Gichtanfall durch eine Behandlung mit einer Gichttherapie ohne begleitende Verabreichung eines zusätzlichen Therapeutikums, um dem Gichtanfall vorzubeugen, hatte oder dies erwartet wird, und/oder wobei dem Subjekt nicht begleitend mit jeder begleitenden Verabreichung ein zusätzliches Therapeutikum verabreicht wird, um dem Gichtanfall vorzubeugen.

4. Zusammensetzungen für die Verwendung nach einem der vorstehenden Ansprüche, wobei die begleitende Verabreichung mindestens viermal, mindestens fünfmal, mindestens sechsmal, mindestens siebenmal, mindestens achtmal, mindestens neunmal oder mindestens zehnmal in dem Subjekt erfolgt.

5. Zusammensetzungen für die Verwendung nach einem der vorstehenden Ansprüche, wobei die Zusammensetzung, umfassend synthetische Nanocarrier, umfassend Rapamycin, und die Zusammensetzung, umfassend Pegsiticase, für mindestens vier Monate, fünf Monate, sechs Monate, sieben Monate, acht Monate, neun Monate, zehn Monate oder länger monatlich verabreicht werden.

6. Zusammensetzungen für die Verwendung nach einem der vorstehenden Ansprüche, wobei:

i) die Zusammensetzung, umfassend synthetische Nanocarrier, umfassend Rapamycin, in einer Dosis von 0,05 - 0,5 mg/kg Rapamycin bei jeder Verabreichung verabreicht wird;

ii) die Zusammensetzung, umfassend synthetische Nanocarrier, umfassend Rapamycin, in einer Dosis von 0,05 mg/kg, 0,08 mg/kg, 0,1 mg/kg, 0,125 mg/kg, 0,15 mg/kg, 0,2 mg/kg, 0,25 mg/kg, 0,3 mg/kg, 0,35 mg/kg, 0,4 mg/kg, 0,45 mg/kg oder 0,5 mg/kg Rapamycin bei jeder Verabreichung verabreicht wird;

iii) die Zusammensetzung, umfassend synthetische Nanocarrier, umfassend Rapamycin, in einer Dosis von 0,55 - 6,5 mg/kg bei jeder Verabreichung verabreicht wird, wobei die Dosis als das mg der synthetischen Nanocarrier, umfassend Rapamycin, angegeben wird; oder

iv) die Zusammensetzung, umfassend synthetische Nanocarrier, umfassend Rapamycin, in einer Dosis von 0,55 mg/kg, 0,65 mg/kg, 0,7 mg/kg, 0,8 mg/kg, 0,9 mg/kg, 1,0 mg/kg, 1,1 mg/kg, 1,25 mg/kg, 1,5 mg/kg, 2,0 mg/kg, 2,5 mg/kg, 3,0 mg/kg, 3,5 mg/kg, 4,0 mg/kg, 4,5 mg/kg, 5,0 mg/kg, 5,5 mg/kg, 6,0 mg/kg oder 6,5 mg/kg bei jeder Verabreichung verabreicht wird, wobei die Dosis als das mg der synthetischen Nanocarrier, umfassend Rapamycin, angegeben wird.

7. Zusammensetzungen für die Verwendung nach Anspruch 6, wobei die Zusammensetzung, umfassend synthetische Nanocarrier, umfassend Rapamycin, in einer Dosis von 0,1 mg/kg oder 0,15 mg/kg Rapamycin bei jeder Verabreichung verabreicht wird.

8. Zusammensetzungen für die Verwendung nach einem der vorstehenden Ansprüche, wobei die Zusammensetzung, umfassend Pegsiticase, in einer Dosis von 0,2 mg/kg Pegsiticase bei jeder Verabreichung verabreicht wird.

9. Zusammensetzungen für die Verwendung nach einem der vorstehenden Ansprüche, wobei:

i) eine Zusammensetzung, umfassend Pegsiticase, an das Subjekt mindestens einmal nach der einen oder den mehreren begleitenden Verabreichungen ohne ein Immunsuppressivum verabreicht wird und/oder eine Zusammensetzung, umfassend Pegsiticase, an das Subjekt mindestens einmal nach der einen oder den mehreren begleitenden Verabreichungen ohne synthetische Nanocarrier, umfassend ein Immunsuppressivum, verabreicht wird;

ii) eine Zusammensetzung, umfassend Pegsiticase, an das Subjekt mindestens einmal nach der einen oder den mehreren begleitenden Verabreichungen ohne ein Immunsuppressivum verabreicht wird und/oder eine Zusammensetzung, umfassend Pegsiticase, an das Subjekt mindestens einmal nach der einen oder den mehreren begleitenden Verabreichungen ohne synthetische Nanocarrier, umfassend ein Immunsuppressivum, verabreicht wird, wobei die Zusammensetzung, umfassend Pegsiticase, mindestens zweimal, dreimal, viermal, fünfmal oder mehr Male nach der einen oder den mehreren Verabreichungen verabreicht wird; oder

iii) eine Zusammensetzung, umfassend Pegsiticase, an das Subjekt mindestens einmal nach der einen oder den mehreren begleitenden Verabreichungen ohne ein Immunsuppressivum verabreicht wird und/oder eine Zusammensetzung, umfassend Pegsiticase, an das Subjekt mindestens einmal nach der einen oder den mehreren begleitenden Verabreichungen ohne synthetische Nanocarrier, umfassend ein Immunsuppressivum, verabreicht wird, wobei die Zusammensetzung, umfassend Pegsiticase, für zwei Monate nach der einen oder den mehreren Verabreichungen monatlich verabreicht wird.

10. Zusammensetzungen für die Verwendung nach einem der vorstehenden Ansprüche, wobei die Zusammensetzung, umfassend synthetische Nanocarrier, umfassend Rapamycin, vor der Zusammensetzung, umfassend Pegsiticase, bei jeder begleitenden Verabreichung verabreicht wird und/oder die Zusammensetzung, umfassend synthetische Nanocarrier, umfassend Rapamycin, und die Zusammensetzung, umfassend Pegsiticase, bei jeder begleitenden Verabreichung innerhalb einer Stunde voneinander verabreicht werden.

11. Zusammensetzungen für die Verwendung nach einem der vorstehenden Ansprüche, wobei das Subjekt aufweist: akute Gicht; chronische Gicht mit oder ohne Tophi; idiopathische Gicht; refraktäre Gicht; sekundäre Gicht; nicht spezifizierte Gicht; Gicht, die mit einer kardiovaskulären Erkrankung, einer Nierenerkrankung, einer Lungenerkrankung, einer neurologischen Erkrankung, einer Augenerkrankung, einer dermatologischen Erkrankung oder einer Lebererkrankung assoziiert wird; oder eine Gichtattacke oder einen Gichtanfall gehabt hat.

12. Zusammensetzungen für Verwendung nach Anspruch 11, wobei das Subjekt chronische refraktäre Gicht aufweist.

13. Zusammensetzung nach einem der vorstehenden Ansprüche, wobei das Rapamycin in den synthetischen Nanocarriern eingekapselt ist.

14. Zusammensetzungen für die Verwendung nach einem der vorstehenden Ansprüche, wobei:

i) der Mittelwert einer Partikelgrößenverteilung, die unter Verwendung dynamischer Lichtstreuung der synthetischen Nanocarrier erhalten wird, ein Diameter ist, der über 120 nm, vorzugsweise über 150 nm, vorzugsweise über 200 nm, vorzugsweise über 250 nm, ist; oder

ii) der Mittelwert einer Partikelgrößenverteilung, die unter Verwendung dynamischer Lichtstreuung der synthetischen Nanocarrier erhalten wird, ein Diameter ist, der über 120 nm, vorzugsweise über 150 nm, ist, und wobei der Diameter kleiner als 300 nm, vorzugsweise kleiner als 250 nm, vorzugsweise kleiner als 200 nm, ist.

**15.** Zusammensetzungen für die Verwendung nach einem der vorstehenden Ansprüche, wobei die Ladung des Rapamycins der synthetischen Nanocarrier 7-12 Gew.-% oder 8-12 Gew.-% ist, vorzugsweise wobei die Ladung des Rapamycins der synthetischen Nanocarrier 7-10 Gew.-% oder 8-10 Gew.-% ist oder die Ladung des Rapamycins der synthetischen Nanocarrier 7, 8, 9, 10, 11 oder 12 Gew.-% ist.

**16.** Zusammensetzungen für die Verwendung nach einem der vorstehenden Ansprüche, wobei jede Verabreichung jeder Zusammensetzung eine intravenöse Verabreichung ist, vorzugsweise wobei die intravenöse Verabreichung eine intravenöse Infusion ist.

**17.** Zusammensetzungen für die Verwendung nach einem der vorstehenden Ansprüche, wobei:

i) das Verfahren ferner das Verabreichen eines zusätzlichen Therapeutikums an das Subjekt umfasst;

ii) das Verfahren ferner das Verabreichen eines zusätzlichen Therapeutikums an das Subjekt umfasst, wobei das zusätzliche Therapeutikum ein orales Gichttherapeutikum ist;

iii) das Verfahren ferner das Verabreichen eines zusätzlichen Therapeutikums an das Subjekt umfasst, wobei das zusätzliche Therapeutikum mindestens einmal nach der einen oder den mehreren begleitenden Verabreichungen ohne ein Immunsuppressivum verabreicht wird, oder wobei das zusätzliche Therapeutikum mindestens einmal, vorzugsweise mindestens zweimal, mindestens dreimal, viermal, fünfmal oder öfter nach der einen oder den mehreren begleitenden Verabreichungen ohne synthetische Nanocarrier, umfassend ein Immunsuppressivum, verabreicht wird;

iv) das Verfahren ferner das Verabreichen eines zusätzlichen Therapeutikums an das Subjekt umfasst, wobei das zusätzliche Therapeutikum ein orales Gichttherapeutikum ist, wobei das zusätzliche Therapeutikum mindestens einmal nach der einen oder den mehreren begleitenden Verabreichungen ohne ein Immunsuppressivum verabreicht wird, oder wobei das zusätzliche Therapeutikum mindestens einmal, vorzugsweise mindestens zweimal, mindestens dreimal, viermal, fünfmal oder öfter nach der einen oder den mehreren begleitenden Verabreichungen ohne synthetische Nanocarrier, umfassend ein Immunsuppressivum, verabreicht wird;

v) das Verfahren ferner das Verabreichen eines zusätzlichen Therapeutikums an das Subjekt umfasst, wobei das zusätzliche Therapeutikum eine Antigichtanfallbehandlung ist; oder

vi) das Verfahren ferner das Verabreichen eines zusätzlichen Therapeutikums an das Subjekt umfasst, wobei das zusätzliche Therapeutikum eine Antigichtanfallbehandlung ist, und wobei die Antigichtanfallbehandlung eine prophylaktische Behandlung ist, die begleitend, aber vor der Verabreichung jeder Pegsiticase-Zusammensetzung verabreicht wird, und/oder wobei die Antigichtanfallbehandlung Colchicin oder ein NSAID ist.

**18.** Zusammensetzungen für die Verwendung nach einem der vorstehenden Ansprüche, wobei das Subjekt einen erhöhten Serumharnsäurespiegel aufweist, der ≥ 6 mg/dL ist.

**19.** Zusammensetzungen für die Verwendung nach einem der vorstehenden Ansprüche, wobei die Zusammensetzung, umfassend synthetische Nanocarrier, polymere synthetische Nanocarrier, umfassend PLA, PLA-PEG und eingekapseltes Rapamycin umfasst; und wobei die Zusammensetzung, umfassend polymere synthetische Nanocarrier, umfassend PLA, PLA-PEG und eingekapseltes Rapamycin, in einer Dosis von 0,1 mg/kg oder 0,15 mg/kg Rapamycin bei jeder Verabreichung verabreicht wird, und die Dosis der Zusammensetzung, umfassend Pegsiticase, 0,2 mg/kg bei jeder Verabreichung ist.

**Revendications**

**1.** Composition comprenant des nanovecteurs synthétiques comprenant du poly(D,L lactide) (PLA) et du poly(D,L lactide) poly(éthylène glycol) (PLA-PEG) et comprenant de la rapamycine, et une composition comprenant de la pegsiticase destinée à être utilisée dans une procédé de traitement d'un sujet souffrant de goutte ou d'un problème médical associé à la goutte, l'hyperuricémie, ou un niveau élevé d'acide urique sérique et/ou des dépôts indésirables d'acide urique, dans laquelle le procédé comprend l'administration des compositions de manière concomitante à un sujet et dans laquelle la composition comprenant de la pegsiticase est administrée à une dose de 0,1 mg/kg, 0,2 mg/kg, 0,4 mg/kg, 0,8 mg/kg ou 1,2 mg/kg de pegsiticase à chaque administration, et dans laquelle la composition comprenant des nanovecteurs synthétiques et la composition comprenant de la pegsiticase sont administrées mensuellement pendant au moins trois mois.

2. Composition comprenant des nanovecteurs synthétiques comprenant du poly(D,L lactide) (PLA) et du poly(D,L lactide) poly(éthylène glycol) (PLA-PEG) et comprenant de la rapamycine, et une composition comprenant de la pegsiticase destinée à être utilisée dans un procédé de prévention des poussées de goutte, dans laquelle le procédé comprend l'administration des compositions de manière concomitante à un sujet, dans laquelle la composition comprenant de la pegsiticase est administrée à une dose de 0,1 mg/kg, 0,2 mg/kg, 0,4 mg/kg, 0,8 mg/kg ou 1,2 mg/kg de pegsiticase à chaque administration, dans laquelle le sujet ne se fait pas administrer de traitement supplémentaire pour prévenir les poussées de goutte en même temps que l'administration concomitante, et dans laquelle la composition comprenant des nanovecteurs synthétiques et la composition comprenant de la pegsiticase sont administrées mensuellement pendant au moins trois mois.

3. Compositions destinées à être utilisées selon la revendication 1 ou la revendication 2, dans lesquelles le sujet est identifié comme ayant eu ou comme étant susceptible d'avoir une poussée de goutte à la suite d'un traitement contre la goutte sans administration concomitante d'un traitement supplémentaire pour prévenir les poussées de goutte, et/ou dans lesquelles le sujet ne se fait pas administrer de traitement supplémentaire pour prévenir les poussées de goutte de manière concomitante à chaque administration concomitante.

4. Compositions destinées à être utilisées selon l'une quelconque des revendications précédentes, dans lesquelles l'administration concomitante a lieu au moins quatre fois, au moins cinq fois, au moins six fois, au moins sept fois, au moins huit fois, au moins neuf fois ou au moins dix fois chez le sujet.

5. Compositions destinées à être utilisées selon l'une quelconque des revendications précédentes, dans lesquelles la composition comprenant des nanovecteurs synthétiques comprenant de la rapamycine et la composition comprenant de la pegsiticase sont administrées mensuellement pendant au moins quatre mois, cinq mois, six mois, sept mois, huit mois, neuf mois, dix mois ou plus.

6. Compositions destinées à être utilisées selon l'une quelconque des revendications précédentes, dans lesquelles :

   i) la composition comprenant des nanovecteurs synthétiques comprenant de la rapamycine est administrée à une dose de 0,05 à 0,5 mg/kg de rapamycine à chaque administration ;
   ii) la composition comprenant des nanovecteurs synthétiques comprenant de la rapamycine est administrée à une dose de 0,05 mg/kg, 0,08 mg/kg, 0,1 mg/kg, 0,125 mg/kg, 0,15 mg/kg, 0,2 mg/kg, 0,25 mg/kg, 0,3 mg/kg, 0,35 mg/kg, 0,4 mg/kg, 0,45 mg/kg ou 0,5 mg/kg de rapamycine à chaque administration ;
   iii) la composition comprenant des nanovecteurs synthétiques comprenant de la rapamycine est administrée à une dose de 0,55 à 6,5 mg/kg à chaque administration, dans lesquelles la dose est exprimée en mg de nanovecteurs synthétiques comprenant de la rapamycine ; ou
   iv) la composition comprenant des nanovecteurs synthétiques comprenant de la rapamycine est administrée à une dose de 0,55 mg/kg, 0,65 mg/kg, 0,7 mg/kg, 0,8 mg/kg, 0,9 mg/kg, 1,0 mg/kg, 1,1 mg/kg, 1,25 mg/kg, 1,5 mg/kg, 2,0 mg/kg, 2,5 mg/kg, 3,0 mg/kg, 3,5 mg/kg, 4,0 mg/kg, 4,5 mg/kg, 5,0 mg/kg, 5,5 mg/kg, 6,0 mg/kg ou 6,5 mg/kg à chaque administration, dans lesquelles la dose est exprimée en mg de nanovecteurs synthétiques comprenant de la rapamycine.

7. Compositions destinées à être utilisées selon la revendication 6, dans lesquelles la composition comprenant des nanovecteurs synthétiques comprenant de la rapamycine est administrée à une dose de 0,1 mg/kg ou 0,15 mg/kg de rapamycine à chaque administration.

8. Compositions destinées à être utilisées selon l'une quelconque des revendications précédentes, dans lesquelles la composition comprenant de la pegsiticase est administrée à une dose de 0,2 mg/kg de pegsiticase à chaque administration.

9. Compositions destinées à être utilisées selon l'une quelconque des revendications précédentes, dans lesquelles :

   i) une composition comprenant de la pegsiticase est administrée au sujet au moins une fois après la ou les administrations concomitantes sans immunosuppresseur et/ou une composition comprenant de la pegsiticase est administrée au sujet au moins une fois après la ou les administrations concomitantes sans nanovecteurs synthétiques comprenant un immunosuppresseur ;

ii) une composition comprenant de la pegsiticase est administrée au sujet au moins une fois après la ou les administrations concomitantes sans immunosuppresseur et/ou une composition comprenant de la pegsiticase est administrée au sujet au moins une fois après la ou les administrations concomitantes sans nanovecteurs synthétiques comprenant un immunosuppresseur, dans lesquelles la composition comprenant de la pegsiticase est administrée au moins deux fois, au moins trois fois, quatre fois, cinq fois ou plus après la ou les administrations concomitantes ; ou

iii) une composition comprenant de la pegsiticase est administrée au sujet au moins une fois après la ou les administrations concomitantes sans immunosuppresseur et/ou une composition comprenant de la pegsiticase est administrée au sujet au moins une fois après la ou les administrations concomitantes sans nanovecteurs synthétiques comprenant un immunosuppresseur, dans lesquelles la composition comprenant de la pegsiticase est administrée mensuellement pendant deux mois après la ou les administrations concomitantes.

10. Compositions destinées à être utilisées selon l'une quelconque des revendications précédentes, dans lesquelles la composition comprenant des nanovecteurs synthétiques comprenant de la rapamycine est administrée avant la composition comprenant de la pegsiticase lors de chaque administration concomitante et/ou la composition comprenant des nanovecteurs synthétiques comprenant de la rapamycine et la composition comprenant de la pegsiticase sont administrées à une heure d'intervalle avec chaque administration concomitante.

11. Compositions destinées à être utilisées selon l'une quelconque des revendications précédentes, dans lesquelles le sujet souffre de goutte aiguë ; de goutte chronique avec ou sans tophi ; de goutte idiopathique ; de goutte réfractaire ; de goutte secondaire ; de goutte non spécifiée ; de goutte associée à une affection cardiovasculaire, affection rénale, affection pulmonaire, affection neurologique, affection oculaire, affection dermatologique ou affection hépatique ; ou a eu une crise de goutte ou une poussée de goutte.

12. Compositions destinées à être utilisées selon la revendication 11, dans lesquelles le sujet souffre de goutte chronique réfractaire.

13. Compositions destinées à être utilisées selon l'une quelconque des revendications précédentes, dans lesquelles la rapamycine est encapsulée dans les nanovecteurs synthétiques.

14. Compositions destinées à être utilisées selon l'une quelconque des revendications précédentes, dans lesquelles :

i) la moyenne d'une distribution de taille des particules obtenue à l'aide d'une diffusion dynamique de lumière des nanovecteurs synthétiques est un diamètre supérieur à 120 nm, de préférence supérieur à 150 nm, de préférence supérieur à 200 nm, de préférence supérieur à 250 nm ; ou

ii) la moyenne de la distribution de taille des particules obtenue par diffusion dynamique de lumière des nanovecteurs synthétiques est un diamètre supérieur à 120 nm, de préférence supérieur à 150 nm, et dans lesquelles le diamètre est inférieur à 300 nm, de préférence inférieur à 250 nm, de préférence inférieur à 200 nm.

15. Compositions destinées à être utilisées selon l'une quelconque des revendications précédentes, dans lesquelles la charge de la rapamycine des nanovecteurs synthétiques est de 7 à 12 % ou 8 à 12 % en poids, de préférence dans lesquelles la charge de rapamycine des nanovecteurs synthétiques est de 7 à 10 % ou 8 à 10 % en poids ou la charge de la rapamycine des nanovecteurs synthétiques est de 7 %, 8 %, 9 %, 10 %, 11 %, ou 12 % en poids.

16. Compositions destinées à être utilisées selon l'une quelconque des revendications précédentes, dans lesquelles chaque administration de chaque composition est une administration intraveineuse, de préférence dans lesquelles l'administration intraveineuse est une infusion intraveineuse.

17. Compositions destinées à être utilisées selon l'une quelconque des revendications précédentes, dans lesquelles :

i) le procédé comprend en outre l'administration d'un traitement supplémentaire au sujet ;

ii) le procédé comprend en outre l'administration d'un traitement supplémentaire au sujet, dans lesquelles le traitement supplémentaire est un traitement oral contre la goutte ;

iii) le procédé comprend en outre l'administration d'un traitement supplémentaire au sujet, dans lesquelles le traitement supplémentaire est administré au moins une fois après la ou les administrations concomitantes sans immunosuppresseur ou dans lesquelles le traitement supplémentaire est administré au moins une fois, de préférence au moins deux fois, au moins trois fois, quatre fois, cinq fois ou plus, après la ou les administrations concomitantes sans nanovecteurs synthétiques comprenant un immunosuppresseur ;

iv) le procédé comprend en outre l'administration d'un traitement supplémentaire au sujet, dans lesquelles le traitement supplémentaire est un traitement oral contre la goutte, dans lesquelles le traitement supplémentaire est administré au moins une fois après la ou les administrations concomitantes sans immunosuppresseur ou dans lesquelles le traitement supplémentaire est administré au moins une fois, de préférence au moins deux fois, au moins trois fois, quatre fois, cinq fois ou plus, après la ou les administrations concomitantes sans nanovecteurs synthétiques comprenant un immunosuppresseur ;

v) le procédé comprend en outre l'administration d'un traitement supplémentaire au sujet, dans lesquelles le traitement supplémentaire est un traitement contre les poussées de goutte ; ou

vi) le procédé comprend en outre l'administration d'un traitement supplémentaire au sujet, dans lesquelles le traitement supplémentaire est un traitement anti-goutte, et dans lesquelles le traitement anti-goutte est un traitement prophylactique administré de manière concomitante mais avant l'administration de chaque composition de pegsiticase et/ou dans lesquelles le traitement anti-goutte est la colchicine ou un AINS.

18. Compositions destinées à être utilisées selon l'une quelconque des revendications précédentes, dans lesquelles le sujet a un niveau élevé d'acide urique sérique qui est $\geq$ 6 mg/dL.

19. Compositions destinées à être utilisées selon l'une quelconque des revendications précédentes, dans lesquelles la composition comprenant des nanovecteurs synthétiques comprend des nanovecteurs synthétiques polymériques comprenant du PLA, du PLA-PEG et de la rapamycine encapsulée ; et dans lesquelles la composition comprenant des nanovecteurs synthétiques polymères comprenant du PLA, du PLA-PEG et de la rapamycine encapsulée est administrée à une dose de 0,1 mg/kg ou 0,15 mg/kg de rapamycine à chaque administration, et la dose de la composition comprenant de la pegsiticase est de 0,2 mg/kg à chaque administration.

Fig. 1

SVP-Rapamycin | Pegsiticase

Rapamycin

PLA + PLA-PEG
+ SPAN 40

PEG

Uricase

Fig. 2

Fig. 3

Fig. 4

**Phase 1a Cohort #3**

| | Uricase-Specific ADA Titer and Serum Uric Acid Levels | | | | | | | | | |
|---|---|---|---|---|---|---|---|---|---|---|
| Subject number | Baseline | | Day 7 | | Day 14 | | Day 21 | | Day 30 | |
| | Uric acid (mg/dl) | ADA (Titer) | Uric acid (mg/dl) | ADA (Titer) | Uric acid (mg/dl) | ADA (Titer) | Uric acid (mg/dl) | ADA (Titer) | Uric acid (mg/dl) | ADA (Titer) |
| 1 | 7.4 | Neg | <0.1 | Neg | 5 | 9720 | 6 | N.A. | 6.9 | 3240 |
| 2 | 7.5 | Neg | <0.1 | 40 | <0.1 | 40 | <0.1 | N.A. | 0.4 | 40 |
| 3 | 7.3 | 120 | <0.1 | 120 | 6.9 | 9720 | 7.6 | N.A. | 7.6 | 3240 |
| 4 | 7.6 | Neg | <0.1 | Neg | 6.1 | 3240 | 7.5 | N.A. | 7.6 | 1080 |
| 5 | 4.9 | Neg | <0.1 | Neg | <0.1 | 1080 | 0.3 | N.A. | 5.1 | 1080 |

**Phase 1b Cohort #9**

| | Uricase-Specific ADA Titer and Serum Uric Acid Levels | | | | | | | | | |
|---|---|---|---|---|---|---|---|---|---|---|
| Subject number | Baseline | | Day 7 | | Day 14 | | Day 21 | | Day 30 | |
| | Uric acid (mg/dl) | ADA (Titer) | Uric acid (mg/dl) | ADA (Titer) | Uric acid (mg/dl) | ADA (Titer) | Uric acid (mg/dl) | ADA (Titer) | Uric acid (mg/dl) | ADA (Titer) |
| 1 | 5.4 | Neg | <0.1 | N.A. | 5.6 | 1080 | 5.8 | 1080 | 7 | 1080 |
| 2 | 6.3 | Neg | <0.1 | N.A. | 5.8 | 29160 | 5.5 | 29160 | 6 | 9720 |
| 3 | 7.4 | Neg | <0.1 | N.A. | <0.1 | 3240 | <0.1 | 1080 | 1.9 | 1080 |
| 4 | 7.2 | Neg | <0.1 | N.A. | 3.2 | 3240 | 7 | 3240 | 6.3 | 1080 |
| 5 | 8.1 | Neg | <0.1 | N.A. | <0.1 | 29160 | 7.8 | 9720 | 8.8 | 9720 |

**Phase 1b Cohort #4**

| | Uricase-Specific ADA Titer and Serum Uric Acid Levels | | | | | | | | | |
|---|---|---|---|---|---|---|---|---|---|---|
| Subject number | Baseline | | Day 7 | | Day 14 | | Day 21 | | Day 30 | |
| | Uric acid (mg/dl) | ADA (Titer) | Uric acid (mg/dl) | ADA (Titer) | Uric acid (mg/dl) | ADA (Titer) | Uric acid (mg/dl) | ADA (Titer) | Uric acid (mg/dl) | ADA (Titer) |
| 1 | 6.7 | Neg | <0.1 | N.A. | <0.1 | Neg | <0.1 | Neg | <0.1 | Neg |
| 2 | 5.8 | Neg | <0.1 | N.A. | <0.1 | Neg | <0.1 | Neg | <0.1 | Neg |
| 3 | 7.3 | Neg | <0.1 | N.A. | <0.1 | 1080 | 4.8 | 29160 | 6.1 | 29160 |
| 4 | 6.2 | Neg | <0.1 | N.A. | <0.1 | Neg | <0.1 | Neg | <0.1 | 120 |
| 5 | 5.5 | Neg | <0.1 | N.A. | <0.1 | 40 | <0.1 | Neg | <0.1 | Neg |

**Phase 1b Cohort #6**

| | Uricase-Specific ADA Titer and Serum Uric Acid Levels | | | | | | | | | |
|---|---|---|---|---|---|---|---|---|---|---|
| Subject number | Baseline | | Day 7 | | Day 14 | | Day 21 | | Day 30 | |
| | Uric acid (mg/dl) | ADA (Titer) | Uric acid (mg/dl) | ADA (Titer) | Uric acid (mg/dl) | ADA (Titer) | Uric acid (mg/dl) | ADA (Titer) | Uric acid (mg/dl) | ADA (Titer) |
| 1 | 7 | Neg | <0.1 | N.A. | <0.1 | Neg | <0.1 | Neg | <0.1 | Neg |
| 2 | 7.4 | Neg | <0.1 | N.A. | <0.1 | Neg | <0.1 | Neg | <0.1 | Neg |
| 3 | 7.5 | Neg | <0.1 | N.A. | <0.1 | Neg | <0.1 | Neg | <0.1 | Neg |
| 4 | 5.6 | 120 | <0.1 | N.A. | <0.1 | 120 | <0.1 | 120 | <0.1 | 120 |
| 5 | 5.9 | Neg | <0.1 | N.A. | <0.1 | Neg | <0.1 | Neg | <0.1 | Neg |

(Neg = Negative; N.A. = Sample not available)

## Fig. 5

Fig. 6

Krystexxa

Placebo

Non-responders (65%)

Responders (35%)

Data excerpted from the monthly dosing cohorts of the Krystexxa Phase 3 clinical studies
*Sundy et al., JAMA, 2011 Vol 306, p717*

Pegsiticase

SVP-Rapamycin (Cohort 5)

Pegsiticase (Cohort 9)

Data from the Phase 1b single dose clinical study of SEL-212

SEL-212

SVP-Rapamycin (Cohort 5)

SEL-212 (Cohort 6)

Fig. 7

EP 3 426 285 B1

**Serum Uric Acid Levels by Cohort**

SVP-Rapamycin only
(0.03 ●, 0.1 ■, and 0.3 ▲ mg/kg)

Pegsiticase only
(0.4mg/kg)

SEL-212
(0.03mg/kg SVP-Rapamycin
+ 0.4mg/kg pegsiticase)

SEL-212
(0.1mg/kg SVP-Rapamycin
+ 0.4mg/kg pegsiticase)

SEL-212
(0.3mg/kg SVP-Rapamycin
+ 0.4mg/kg pegsiticase)

**Day 30 sUA levels and ADA levels**

| Pegsiticase alone (0.4 mg/kg) | | | SEL-212 0.1 mg/kg SVP-Rapamycin + 0.4 mg/kg Pegsiticase | | | SEL-212 0.3 mg/kg SVP-Rapamycin + 0.4 mg/kg Pegsiticase | | |
|---|---|---|---|---|---|---|---|---|
| Subject number | Day 30 | | Subject number | Day 30 | | Subject number | Day 30 | |
| | Uric acid (mg/dL) | ADA (Titer) | | Uric acid (mg/dL) | ADA (Titer) | | Uric acid (mg/dL) | ADA (Titer) |
| 108-0010 | 7 | 1080 | 107-0018 | <0.1 | Neg | 107-0027 | <0.1 | Neg |
| 103-0015 | 6 | 9270 | 107-0021 | <0.1 | Neg | 107-0028 | <0.1 | Neg |
| 104-0032 | 1.9 | 1080 | 104-0027 | 6.1 | 29160 | 104-0050 | <0.1 | Neg |
| 109-0012 | 6.3 | 1080 | 108-0008 | <0.1 | 120 | 104-0060 | <0.1 | 120 |
| 104-0036 | 8.8 | 9720 | 102-0005 | <0.1 | Neg | 103-0019 | <0.1 | Neg |

(Neg = Negative)

# Fig. 8

| Cohort | SEL-110 | SEL-037 |
|--------|----------|-----------|
| 1 | NA | 0.2 mg/kg |
| 2 | NA | 0.4 mg/kg |
| 3 | 0.05mg/kg | 0.2 mg/kg |
| 4 | 0.05mg/kg | 0.4 mg/kg |
| 5 | 0.08mg/kg | 0.2 mg/kg |
| 6 | 0.08mg/kg | 0.4 mg/kg |

Fig. 9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20160128986 A
- US 20160128987 A
- WO 1998002441 A
- US 8455510 B
- US 5543158 A, Gref
- US 20060002852 A, Saltzman
- US 20090028910 A, DeSimone
- WO 2009051837 A, von Andrian
- US 20080145441 A, Penades
- US 5700674 A
- US 6913915 B
- US 6123727 A
- US 5804178 A
- US 5770417 A
- US 5736372 A
- US 5716404 A
- US 6095148 A
- US 5837752 A

- US 5902599 A
- US 5696175 A
- US 5514378 A
- US 5512600 A
- US 5399665 A
- US 5019379 A
- US 5010167 A
- US 4806621 A
- US 4638045 A
- US 4946929 A
- US 6506577 B
- US 6632922 B
- US 6686446 B
- US 6818732 B
- US 5578325 A
- US 6007845 A
- US 6632671 B, Unger

**Non-patent literature cited in the description**

- Urate-Lowering Therapy. **MIKULS T.R.** Kelley's Textbook of Rheumatology. Elsevier Saunders, 2009
- **SUNDY JS ; BECKER MA ; BARAF HS et al.** Reduction of plasma urate levels following treatment with multiple doses of pegloticase (polyethylene glycol-conjugated uricase) in patients with treatment-failure gout: results of a phase II randomized study. *Arthritis Rheum.,* 2008, vol. 58, 2882-2891
- **JOHN S. SUNDY, MD, PHD ; HERBERT S. B. BARAF, MD ; ROBERT A. YOOD, MD et al.** Efficacy and Tolerability of Pegloticase for the Treatment of Chronic Gout in Patients Refractory to Conventional Treatment Two Randomized Controlled Trials. *JAMA.,* 2011, vol. 306 (7), 711-720
- **MICHAEL A. BECKER, M.D. ; H. RALPH SCHUMACHER, JR., M.D. ; ROBERT L. WORTMANN, M.D. ; PATRICIA A. MACDONALD, B.S.N., N.P. ; DENISE EUSTACE, B.A. ; WILLIAM A. PALO, M.S. ; JANET STREIT, M.S. ; NANCY JOSEPH-RIDGE, M.D.** Febuxostat Compared with Allopurinol in Patients with Hyperuricemia and Gout. *N Engl J Med,* 08 December 2005, vol. 353, 2450-2461

- **PEREZ-RUIZ F ; SUNDY JS ; MINER JN et al.** Lesinurad in combination with allopurinol: results of a phase 2, randomised, double-blind study in patients with gout with an inadequate response to allopurinol. *Annals of the Rheumatic Diseases,* 2016, vol. 75, 1074-1080
- **SCHLESINGER N.** Treatment of chronic gouty arthritis: it is not just about urate-lowering therapy. *Semin. Arthritis Rheum.,* 01 October 2012, vol. 42 (2), 155-65
- **P. PAOLICELLI et al.** Surface-modified PLGA-based Nanoparticles that can Efficiently Associate and Deliver Virus-like Particles. *Nanomedicine.,* 2010, vol. 5 (6), 843-853
- **LOOK et al.** Nanogel-based delivery of mycophenolic acid ameliorates systemic lupus erythematosus in mice. *J. Clinical Investigation,* 2013, vol. 123 (4), 1741-1749
- PCR Technology. Principles and Applications for DNA Amplification. Stockton Press, 1989
- **SAMBROOK, J et al.** Molecular Cloning. A Laboratory Manual. Cold Spring Harbor Laboratory Press, 1989
- **PARK et al.** *Anticancer Res.,* 1981, vol. 1, 373-376

- **ZAPLIPSKY ; LEE.** Polyethylene Glycol Chemistry: Biotechnical and Biomedical Applications. Plenum Press, 1992
- **PAPISOV.** *ACS Symposium Series,* 2001, vol. 786, 301
- **WANG et al.** *J. Am. Chem. Soc.,* 2001, vol. 123, 9480
- **LIM et al.** *J. Am. Chem. Soc.,* 2001, vol. 123, 2460
- **LANGER.** *Acc. Chem. Res.,* 2000, vol. 33, 94
- **LANGER.** *J. Control. Release,* 1999, vol. 62, 7
- **UHRICH et al.** *Chem. Rev.,* 1999, vol. 99, 3181
- Concise Encyclopedia of Polymer Science and Polymeric Amines and Ammonium Salts. Pergamon Press, 1980
- Principles of Polymerization by Odian. John Wiley & Sons, 2004
- **ALLCOCK et al.** Contemporary Polymer Chemistry. Prentice-Hall, 1981
- **DEMING et al.** *Nature,* 1997, vol. 390, 386
- **PELLEGRINO et al.** *Small,* 2005, vol. 1, 48
- **MURRAY et al.** *Ann. Rev. Mat. Sci.,* 2000, vol. 30, 545
- **TRINDADE et al.** *Chem. Mat.,* 2001, vol. 13, 3843
- **DOUBROW.** Microcapsules and Nanoparticles in Medicine and Pharmacy. CRC Press, 1992
- **MATHIOWITZ et al.** *J. Control. Release,* 1987, vol. 5, 13
- **MATHIOWITZ et al.** *Reactive Polymers,* 1987, vol. 6, 275
- **MATHIOWITZ et al.** *J. Appl. Polymer Sci.,* 1988, vol. 35, 755
- **C. ASTETE et al.** Synthesis and characterization of PLGA nanoparticles. *J. Biomater. Sci. Polymer Edn,* 2006, vol. 17 (3), 247-289

- **K. AVGOUSTAKIS.** Pegylated Poly(Lactide) and Poly(Lactide-Co-Glycolide) Nanoparticles: Preparation, Properties and Possible Applications in Drug Delivery. *Current Drug Delivery,* 2004, vol. 1, 321-333
- **C. REIS et al.** Nanoencapsulation I. Methods for preparation of drug-loaded polymeric nanoparticles. *Nanomedicine,* 2006, vol. 2, 8-21
- **BAYLE et al.** *Chemistry & Biology,* 2006, vol. 13, 99-107
- Handbook of Industrial Mixing: Science and Practice. John Wiley & Sons, Inc, 2004
- Pharmaceutics: The Science of Dosage Form Design. Churchill Livingstone, 2001
- **SUNDY et al.** Pharmacokinetics and pharmacodynamics of intravenous PEGylated recombinant mammalian urate oxidase in patients with refractory gout. *Arthritis and Rheumatism.,* March 2007, vol. 56 (3), 1021-1028
- **JOHN S. SUNDY, MD, PHD ; HERBERT S. B. BARAF, MD ; ROBERT A. YOOD, MD et al.** Efficacy and Tolerability of Pegloticase for the Treatment of Chronic Gout in Patients Refractory to Conventional TreatmentTwo Randomized Controlled Trials. *JAMA.,* 2011, vol. 306 (7), 711-720
- **PEREZ -RUIZ F ; SUNDY JS et al.** Lesinurad in combination with allopurinol: results of a phase 2, randomised, double-blind study in patients with gout with an inadequate response to allopurinol. *Annals of the Rheumatic Diseases,* 2016, vol. 75, 1074-1080
- **SCHLESINGER N.** Treatment of chronic gouty arthritis: it is not just about urate-lowering therapy. *Semin. Arthritis Rheum.,* 01 October 2012, vol. 42 (2), 155-65